(19) Europäisches Patentamt
European Patent Office
Office européen des brevets

(11) Veröffentlichungsnummer: **0 000 365**
**A1**

# (12) EUROPÄISCHE PATENTANMELDUNG

(21) Anmeldenummer: 78100290.2

(22) Anmeldetag: 30.06.78

(51) Int. Cl.²: C07C157/14, A01N9/12

(30) Priorität: 07.07.77 DE 2730620

(43) Veröffentlichungstag der Anmeldung: 24.01.79
Patentblatt 79/2

(84) Benannte Vertragsstaaten: BE CH DE FR GB NL

(71) Anmelder: Bayer Aktiengesellschaft, Zentralbereich Patente, Marken und Lizenzen Bayerwerk, D-5090 Leverkusen 1 (DE)

(72) Erfinder: Enders, Edgar, Dr., René-Bohn-Strasse 16, D-5000 Köln (DE)
Stendel, Wilhelm, Dr., In den Birken 55, D-5600 Wuppertal (DE)
Hammann, Ingeborg, Dr., Belfortstrasse 9, D-5000 Köln (DE)
Behrenz, Wolfgang, Dr., Untergruendemich 14, D-5063 Overath (DE)

## (54) Neue N-Aryl-N'-alkyl-S-alkyl-isothioharnstoffe, Verfahren zu ihrer Herstellung sowie ihre Verwendung als Mittel zur Bekämpfung von tierischen und pflanzlichen Schädlingen.

(57) Die vorliegende Erfindung betrifft neue N-Aryl-N'-alkyl-S-alkyl-isothioharnstoffe, Verfahren zu ihrer Herstellung sowie ihre Verwendung als Mittel zur Bekämpfung von tierischen und pflanzlichen Schädlingen, insbesondere als Ektoparasitizide, Insektizide und Akarizide.

EP 0 000 365 A1

BAYER AKTIENGESELLSCHAFT 5090 Leverkusen, Bayerwerk
Zentralbereich Er/kl
Patente, Marken und Lizenzen

Neue N-Aryl-N'-alkyl-S-alkyl-isothioharnstoffe, Verfahren zu ihrer Herstellung sowie ihre Verwendung als Mittel zur Bekämpfung von tierischen und pflanzlichen Schädlingen

Die vorliegende Erfindung betrifft neue N-Aryl-N'-alkyl-S-alkyl-isothioharnstoffe, Verfahren zu ihrer Herstellung sowie ihre Verwendung als Mittel zur Bekämpfung von tierischen und pflanzlichen Schädlingen, insbesondere als Ektoparasitizide, Insektizide und Akarizide.

Es ist bereits bekannt geworden, daß N-Aryl-N',N'-dialkyl-thioharnstoffe als Ektoparasitizide, insbesondere als Tickizide gegen Zecken der Gattung Boophilus, wirksam sind (siehe dazu Deutsche Offenlegungsschrift 2 337 122).

Im Vergleich mit den bekannten und chemisch verwandten Verbindungen zeigen die erfindungsgemäßen neuen N-Aryl-N'-alkyl-S-alkyl-isothioharnstoffe eine bessere Wirkung gegen Phosphorester-resistente Zecken der Gattung Boophilus sowie zusätzlich eine starke insektizide Wirkung

die den Vergleichsprodukten aus der Deutschen Offenlegungsschrift 2 337 122 fehlt. Die erfindungsgemäßen Verbindungen können außerdem in der Landwirtschaft vor allem gegen pflanzenschädigende Insekten und gegen Acariden eingesetzt werden.

Es wurde gefunden, daß die neuen N-Aryl-N'-alkyl-S-alkyl-isothioharnstoffe der allgemeinen Formel

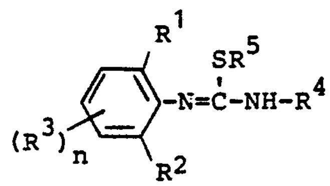

(I)

in welcher

$R^1$ für Alkyl oder Cycloalkyl,
$R^2$ für Alkyl mit mindestens zwei Kohlenstoffatomen oder für Cycloalkyl,
die Reste $R^3$ gleich oder verschieden sein können und
$R^3$ für Alkyl, Cycloalkyl oder Halogen,
n für eine ganze Zahl von 0 bis 2 steht
und $R^4$ für gegebenenfalls substituiertes Alkyl, gegebenenfalls substituiertes Alkenyl oder gegebenenfalls substituiertes Cycloalkyl steht
und $R^5$ für Alkyl, Alkenyl oder Cycloalkyl steht,
und deren Salze, eine starke Wirkung gegenüber tierischen und pflanzlichen Schädlingen, insbesondere eine ektoparasitizide, insektizide und akarizide Wirkung aufweisen.

Besonders bevorzugt sind Verbindungen der Formel

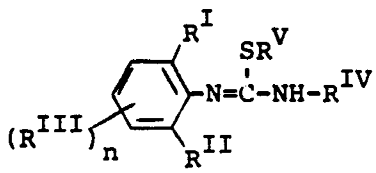

(Ia)

in welcher

$R^I$ für Alkyl ($C_1$-$C_6$) oder Cycloalkyl ($C_4$-$C_7$),
$R^{II}$ für Alkyl ($C_2$-$C_6$) oder Cycloalkyl ($C_4$-$C_7$) steht,
die Reste $R^{III}$ gleich oder verschieden sein können und für Alkyl ($C_1$-$C_7$), Halogen, Cycloalkyl ($C_4$-$C_7$) stehen
und n für 0,1 oder 2 steht,
$R^{IV}$ für gegebenenfalls durch Halogen, Cycloalkyl ($C_4$-$C_7$), Cycloalkenyl ($C_5$-$C_7$) oder Trifluormethyl substituiertes Alkyl ($C_1$-$C_{14}$) oder für gegebenenfalls durch Halogen und/oder $CF_3$ ein- oder mehrfach substituiertes Alkenyl ($C_3$-$C_{12}$) steht oder für gegebenenfalls durch Alkyl ($C_1$-$C_4$), Halogen und/oder $CF_3$ ein- oder mehrfach substituiertes Cycloalkyl ($C_3$-$C_{10}$) steht,
und $R^V$ für Alkyl ($C_1$-$C_6$), Alkenyl ($C_3$-$C_6$) oder Cycloalkyl ($C_3$-$C_6$) steht,

und deren Salze eine starke Wirkung gegenüber tierischen und pflanzlichen Schädlingen, insbesondere eine ektoparazitizide, insektizide und akarizide Wirkung besitzen.

Weiterhin wurde gefunden, daß man die neuen N-Aryl-N'-alkyl-S-alkyl-isothioharnstoffe der Formel (I) erhält, wenn man Thioharnstoffe der Formel

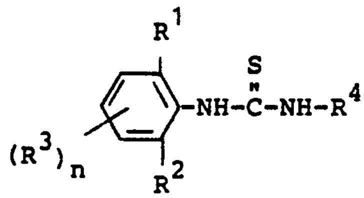 (II)

in welcher $R^1$, $R^2$, $R^3$, $R^4$ und n die oben angegebene Bedeutung besitzen, mit einer Verbindung der allgemeinen Formel

$$R^5-X \qquad \text{(III)}$$ umsetzt,

wobei $R^5$ die oben angegebene Bedeutung besitzt und X einen anionisch abspaltbaren Rest bedeutet.

Die als Ausgangsprodukte einzusetzenden und ebenfalls neuen Thioharnstoffe der Formel (II) können erhalten werden, wenn man

a) Arylisothiocyanaten der Formel

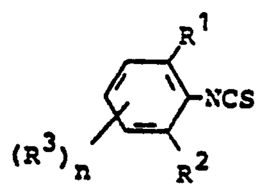 (IV)

mit Alkylaminen der Formel

$$H_2N-R^4 \qquad \text{(V)}$$

oder alternativ

b) Alkylisothiocyanate der Formel

$H_2N-R^4$ (VI)

mit Arylaminen der Formel

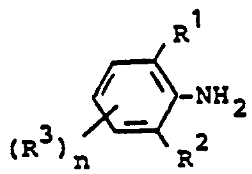

(VII)

umsetzt, wobei

$R^1$, $R^2$, $R^3$, $R^4$ und n in den Formeln (IV) bis (VII) jeweils die oben angegebene Bedeutung besitzen.

Überraschenderweise zeigen die erfindungsgemäßen neuen N-Aryl-N'-alkyl-S-alkyl-isothioharnstoffe eine stärker ausgeprägte ektoparasitizide Wirkung als die N-Aryl-N',N'-dialkyl-thioharnstoffe aus der Deutschen Offenlegungsschrift 2 337 122 sowie zusätzlich eine insektizide Wirkung sowie eine Wirkung gegen Milben, welche den Verbindungen aus der Deutschen Offenlegungsschrift 2 337 122 fehlt.

Die Bereitstellung der erfindungsgemäßen Verbindungen stellt somit eine Bereicherung der Technik dar.

Verwendet man N-(2,6-Diisopropyl-phenyl)-N'-tert.-butyl-thioharnstoff und Methyljodid als Ausgangsverbindungen, so kann der Reaktionsablauf durch folgendes Formelschema wiedergegeben werden:

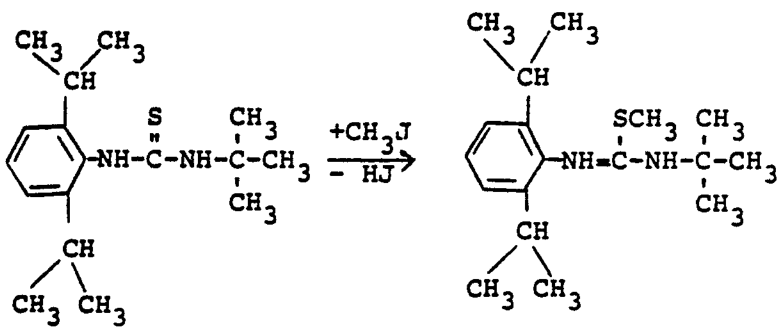


Wie durch Vergleich von Formel (Ia) mit der Formel (I) zu ersehen ist, steht Alkyl $R^1$ vorzugsweise für Alkyl mit 1 bis 6, insbesondere 1 bis 4 Kohlenstoffatomen. Weiterhin steht Cycloalkyl $R^1$ und $R^2$ vorzugsweise für Cycloalkyl mit 4 bis 7 Kohlenstoffatomen. An Alkylreste $R^1$ und Cycloalkylrest $R^1$ und $R^2$ seien beispielhaft genannt: Methyl, Äthyl, Propyl, Isopropyl, Butyl, sec.-Butyl, iso-Butyl, tert.-Butyl, Pentyl-(2), Pentyl-(3), tert.-Pentyl, Cyclopentyl, Hexyl-(2), Hexyl-(3), Cyclohexyl, Cycloheptyl.

Alkyl $R^2$ steht vorzugsweise für Alkyl mit 2 bis 6, insbesondere 2 bis 4 Kohlenstoffatomen, genannt seien beispielhaft: Äthyl, Propyl, Isopropyl, Butyl, sec.-Butyl, iso-Butyl, tert.-Butyl.

Alkyl $R^3$ steht vorzugsweise für geradkettiges oder verzweigtes Alkyl mit 1 bis 7 Kohlenstoffatomen.

Cycloalkyl $R^3$ steht vorzugsweise für Cycloalkyl mit 4 bis 7 Kohlenstoffatomen.

An individuellen Alkylresten $R^3$ sowie Cycloalkylresten $R^3$ seien beispielhaft genannt:

Methyl, Äthyl, Propyl, Isopropyl, Butyl, sec.-Butyl, Isobutyl, tert.-Butyl, n-Pentyl, Pentyl-(2), Pentyl-(3), 2-Methyl-butyl, 3-Methyl-butyl, 2,2-Dimethylpropyl, tert.-Pentyl, n-Hexyl, Hexyl-(2), Hexyl-(3), 2,3-Dimethyl-butyl, 2-Methyl-pentyl, 2,3-Dimethyl-butyl-(2), n-Heptyl, 1,1-Diäthyl-propyl, Cyclobutyl, Cyclopentyl, 1-Methyl-cyclopentyl, Cycloheptyl, Cyclohexyl, 1-Methyl-cyclohexyl. An Halogen $R^3$ seien vorzugsweise genannt: Fluor, Chlor und Brom.

Der Rest $R^4$ kann für gegebenenfalls substituiertes Alkyl stehen und enthält vorzugsweise 1 bis 14, insbesondere 1 bis 10 Kohlenstoffatome. Beispielhaft seien folgende gegebenenfalls substituierte Reste genannt:

Methyl, Äthyl, Propyl, Butyl, Pentyl, Hexyl, Heptyl, Octyl, Decyl, Isopropyl, sec.-Butyl, iso-Butyl, tert.-Butyl, 2-Methyl-butyl, 3-Methyl-butyl, tert.-Pentyl, Pentyl-(2), 3-Methyl-butyl-(2), 2,3-Dimethyl-butyl-(2), 2,3,3-Trimethyl-butyl-(2), 2,2-Dimethyl-propyl-(1), 2-Methyl-butyl-(2), 4,4-Dimethylpentyl-(2), 2,4,4-Trimethyl-pentyl-(2), Hexyl-(2), Hexyl-(3).

Gegebenenfalls substituiertes Alkenyl $R^4$ enthält vorzugsweise 2 bis 12, insbesondere 2 bis 10 Kohlenstoffatome. Beispielhaft seien die folgenden,gegebenenfalls noch weiter substituierten Reste genannt:

Allyl, Crotyl, 1-Methyl-allyl, 1,1-Dimethyl-allyl, 3,3-Dimethylallyl, 3-Hexyl-allyl.

Gegebenenfalls substituiertes Cycloalkyl $R^4$ enthält vorzugsweise 3 bis 10 Kohlenstoffatome. Beispielhaft seien die gegebenenfalls noch weiter substituierten folgenden Reste genannt:

Cyclopropyl, Cyclobutyl, Cyclopentyl, Cyclohexyl, 1-Methyl-cyclopropyl,Cyclopenten-(1)-yl-(3), Cyclopenten-(1)-yl-(4), Cyclohexen-(1)-yl-(3), 1-Methyl-cyclopentyl-(1), 1-Methyl-cyclohexyl-(1), 2-Methyl-cyclohexyl, 3-Methyl-cyclohexyl, 4-Methyl-cyclohexyl, 2,3-, 2,6-, 2,5- oder 3,5-Dimethyl-cyclohexyl, 3,5-Bis-trifluormethyl-cyclohexyl, 2-, 3- oder 4-Trifluormethyl-cyclohexyl, 4-Methyl-3-trifluormethyl-cyclohexyl, 4-Äthyl-cyclohexyl, 2-Äthyl-cyclohexyl, 4-Isopropyl-cyclohexyl, 2-Sec.-butyl-cyclohexyl, Norbornyl, 3,3,5-Trimethyl-cyclohexyl, Bornyl, Isobornyl, Dekahydronaphthyl-(1), Adamantyl, Cycloheptyl, Cyclooctyl.

Der Rest $R^5$ steht vorzugsweise für Alkyl-($C_1$-$C_6$), Alkenyl-($C_3$-$C_6$) oder Cycloalkyl-($C_3$-$C_6$). Beispielsweise seien genannt: Methyl, Äthyl, Propyl, Isopropyl, Butyl, Isobutyl, Hexyl, Allyl, Crotyl, Methallyl, Buten-(3)-yl-(1), Cyclopropyl, Cyclopentyl, Cyclohexyl.

n steht vorzugsweise für 0, 1 oder 2.

Als anionisch abspaltbarer Rest X der allgemeinen Formel (III) steht vorzugsweise Halogen, insbesondere Chlor, Brom oder Jod bzw. eine Alkylsulfonyloxygruppe oder eine Arylsulfonyloxygruppe der Formeln

$$R'-SO_3^{\ominus} \quad \text{bzw.} \quad R''-SO_3^{\ominus}$$

in welcher

R' für Alkyl mit vorzugsweise 1 bis 6 Kohlenstoffatomen und

R" für Aryl mit vorzugsweise 6 oder 10 Kohlenstoffatomen steht.

Weiterhin steht X vorzugsweise für einen Schwefelsäurealkylester-Rest der Formel

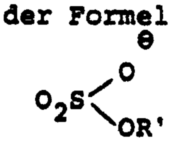 ,

für einen Schwefligsäurealkylester-Rest der Formel

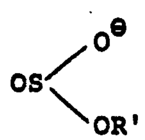 ,

oder für einen Phosphorsäurealkylester-Rest der Formel

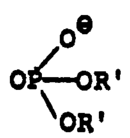 ,

wobei die Reste R' vorzugsweise die oben angegebene Bedeutung besitzen.

Als Beispiele für N-(2,6-Dialkyl-phenyl)-N'-alkyl-thioharnstoffe der allgemeinen Formel (II), die für das erfindungsgemäße Verfahren als Ausgangsverbindungen dienen, seien die folgenden aufgeführt:

N-(2,6-Diäthyl-phenyl)-N'-methyl-thioharnstoff; F: 103-105°C
N-(2,6-Diäthyl-phenyl)-N'-äthyl-thioharnstoff; F: 72-73°C
N-(2,6-Diäthyl-phenyl)-N'-propyl-thioharnstoff; F: 58-60°C
N-(2,6-Diäthyl-phenyl)-N'-butyl-thioharnstoff; F: 41-43°C
N-(2,6-Diäthyl-phenyl)-N'-isobutyl-thioharnstoff; F: 68-70°C
N-(2,6-Diäthyl-phenyl)-N'-neopentyl-thioharnstoff; F: 87-89°C
N-(2,6-Diäthyl-phenyl)-N'-pentyl-thioharnstoff; (F<30°C)
N-(2,6-Diäthyl-phenyl)-N'-hexyl-thioharnstoff; F: 102-105°C
N-(2,6-Diäthyl-phenyl)-N'-heptyl-thioharnstoff; (F<30°C)

N-(2,6-Diäthyl-phenyl)-N'-oktyl-thioharnstoff
N-(2,6-Diäthyl-phenyl)-N'-decyl-thioharnstoff
N-(2,6-Diäthyl-phenyl)-N'-dodecyl-thioharnstoff
N-(4-Methyl-2,6-diäthyl-phenyl)-N'-methyl-thioharnstoff; F: 97-100°C
N-(4-Methyl-2,6-diäthyl-phenyl)-N'-äthyl-thioharnstoff
N-(4-Methyl-2,6-diäthyl-phenyl)-N'-propyl-thioharnstoff
N-(4-Methyl-2,6-diäthyl-phenyl)-N'-butyl-thioharnstoff; F: Öl (F <30°C)

N-(4-Methyl-2,6-diäthyl-phenyl)-N'-isobutyl-thioharnstoff; F: 60-61°C

N-(4-Methyl-2,6-diäthyl-phenyl)-N'-neopentyl-thioharnstoff; F: 106-107°C

N-(4-Methyl-2,6-diäthyl-phenyl)-N'-pentyl-thioharnstoff

N-(4-Methyl-2,6-diäthyl-phenyl)-N'-hexyl-thioharnstoff

N-(4-Methyl-2,6-diäthyl-phenyl)-N'-(2,2-dimethyl-butyl)-thioharnstoff

N-(4-Methyl-2,6-diäthyl-phenyl)-N'-(2,3-dimethyl-butyl)-thioharnstoff

N-(4-Methyl-2,6-diäthyl-phenyl)-N'-(2,2,3-trimethyl-butyl)-thioharnstoff

N-(4-Methyl-2,6-diäthyl-phenyl)-N'-(2,3,3-trimethyl-butyl)-thioharnstoff

N-(4-Methyl-2,6-diäthyl-phenyl)-N'-(2,2,3,3-tetramethyl-butyl)-thioharnstoff

N-(4-Methyl-2,6-diäthyl-phenyl)-N'-(3-Methyl-pentyl)-thioharnstoff

N-(4-Methyl-2,6-diäthyl-phenyl)-N'-(2-isopropyl-butyl)-thioharnstoff

N-(4-Methyl-2,6-diäthyl-phenyl)-N'-(2,4,4-trimethyl-pentyl)-thioharnstoff

N-(4-Methyl-2,6-diäthyl-phenyl)-N'-(2,2,4,4-Tetramethyl-pentyl)-thioharnstoff

N-(4-Methyl-2,6-diäthyl-phenyl)-N'-allyl-thioharnstoff

N-(4-Methyl-2,6-diäthyl-phenyl)-N'-methallyl-thioharnstoff F: 86-88°C

N-(4-Methyl-2,6-diäthyl-phenyl)-N'-crotyl-thioharnstoff

N-(4-Methyl-2,6-diäthyl-phenyl)-N'-(3,3-dimethyl-allyl)-thioharnstoff

N-(4-Methyl-2,6-diäthyl-phenyl)-N'-(2,3-dimethyl-allyl)-thioharnstoff

N-(4-Methyl-2,6-diäthyl-phenyl)-N'-[Buten-(3)-yl-(1)]-thioharnstoff

N-(4-Methyl-2,6-diäthyl-phenyl)-N'-/2,2-dimethyl-buten-(3)-yl-(1)/-thioharnstoff

N-(4-Methyl-2,6-diäthyl-phenyl)-N'-(3-isopropyl-allyl)-thioharnstoff

N-(4-Methyl-2,6-diäthyl-phenyl)-N'-(3-tert.-butyl-allyl)-thioharnstoff

N-(4-Methyl-2,6-diäthyl-phenyl)-N'-(3-hexyl-allyl)-thioharnstoff

N-(4-Methyl-2,6-diäthyl-phenyl)-N'-cyclopropyl-methyl-thioharnstoff

N-(4-Methyl-2,6-diäthyl-phenyl)-N'-(cyclopentyl-methyl)-thioharnstoff

N-(4-Methyl-2,6-diäthyl-phenyl)-N'-/cyclopenten-(1)-yl-(1)-methyl/-thioharnstoff

N-(4-Methyl-2,6-diäthyl-phenyl)-N'-cyclohexyl-thioharnstoff

N-(4-Methyl-2,6-diäthyl-ohenyl)-N'-/cyclohexen-(3)-yl-(1)-methyl/-thioharnstoff

N-(2,6-Diäthyl-phenyl)-N'-(2,3-dimethyl-butyl)-thioharnstoff

N-(2,6-Diäthyl-phenyl)-N'-(2,2,3-trimethyl-butyl)-thioharnstoff

N-(2,6-Diäthyl-phenyl)-N'-(3-methyl-pentyl)-thioharnstoff

N-(2,6-Diäthyl-phenyl)-N'-(2,2,4,4-tetramethyl-pentyl)-thioharnstoff

N-(2,6-Diäthyl-phenyl)-N'-(3,3-dimethyl-allyl)-thioharnstoff

N-(2,6-Diäthyl-phenyl)-N'-(2,3-dimethyl-allyl)-thioharnstoff

N-(2,6-Diäthyl-phenyl)-N'-methallyl-thioharnstoff

N-(2,6-Diäthyl-phenyl)-N'-(cyclopentyl-methyl)-thioharnstoff

N-(2,6-Diäthyl-phenyl)-N'-(cyclohexyl-methyl)-thioharnstoff

N-(3-Methyl-2,6-diäthyl-phenyl)-N'-propyl-thioharnstoff

N-(3-Methyl-2,6-diäthyl-phenyl)-N'-isobutyl-thioharnstoff

N-(3-Methyl-2,6-diäthyl-phenyl)-N'-neopentyl-thioharnstoff F: 102-104°C

N-(3-Methyl-2,6-diäthyl-phenyl)-N'-pentyl-thioharnstoff

N-(3-Methyl-2,6-diäthyl-phenyl)-N'-methallyl-thioharnstoff F: 105-106°C

N-(3-Methyl-2,6-diäthyl-phenyl)-N'-(3-methyl-butyl)-thioharnstoff

N-(3-Methyl-2,6-diäthyl-phenyl)-N'-(2-äthyl-butyl)-thioharnstoff

N-(3,5-Dimethyl-2,6-diäthyl-phenyl)-N'-butyl-thioharnstoff F: 108-110°C

N-(3,5-Dimethyl-2,6-diäthyl-phenyl)-N'-pentyl-thioharnstoff

N-(3,5-Dimethyl-2,6-diäthyl-phenyl)-N'-neopentyl-thioharnstoff

N-(3,5-Dimethyl-2,6-diäthyl-phenyl)-N'-metallyl-thioharnstoff

N-(3,5-Dimethyl-2,6-diäthyl-phenyl)-N'-penten-(4)-yl-(1)-thioharnstoff

N-(3,5-Dimethyl-2,6-diäthyl-phenyl)-N'-cyclopentyl-methyl-thioharnstoff

N-(3,4-Dimethyl-2,6-diäthyl-phenyl)-N'-neopentyl-thioharnstoff

N-(3,4-Dimethyl-2,6-diäthyl-phenyl)-N'-isobutyl-thioharnstoff

N-(3,4-Dimethyl-2,6-diäthyl-phenyl)-N'-pentyl-thioharnstoff

N-(3,4-Dimethyl-2,6-diäthyl-phenyl)-N'-methallyl-thioharnstoff

N-(3-Chlor-2,6-diäthyl-phenyl)-N'-propyl-thioharnstoff

N-(3-Chlor-2,6-diäthyl-phenyl)-N'-butyl-thioharnstoff F: 108:111°C

N-(3-Chlor-2,6-diäthyl-phenyl)-N'-neopentyl-thioharnstoff F: 106-110°C

N-(3-Chlor-2,6-diäthyl-phenyl)-N'-(3-methyl-pentyl)-thioharnstoff

N-(3-Chlor-4-methyl-2,6-diäthyl-phenyl)-N'-isobutyl-thioharnstoff

N-(3-Chlor-4-methyl-2,6-diäthyl-phenyl)-N'-neopentyl-thioharnstoff

N-(3-Chlor-4-methyl-2,6-diäthyl-phenyl)-N'-methallyl-thioharnstoff

N-(2,4,6-Trimethyl-phenyl)-N'-neopentyl-thioharnstoff

N-(2,4,6-Trimethyl-phenyl)-N'-methallyl-thioharnstoff

N-(2,3,4,6-Tetramethyl-phenyl)-N'-butyl-thioharnstoff

N-(2,3,4,6-Tetramethyl-phenyl)-N'-methallyl-thioharnstoff

N-(2,6-Diisopropyl-phenyl)-N'-methyl-thioharnstoff F: 167-168°C

N-(2,6-Diisopropyl-phenyl)-N'-äthyl-thioharnstoff

N-(2,6-Diisopropyl-phenyl)-N'-propyl-thioharnstoff F: 106-107°C

N-(2,6-Diisopropyl-phenyl)-N'-butyl-thioharnstoff F: 99-103°C

N-(2,6-Diisopropyl-phenyl)-N'-isobutyl-thioharnstoff F: 108-112°C

N-(2,6-Diisopropyl-phenyl)-N'-(3-methyl-butyl)-thioharnstoff F: 64-68°C

N-(2,6-Diisopropyl-phenyl)-N'-pentyl-thioharnstoff F: 76-83°C

N-(2,6-Diisopropyl-phenyl)-N'-neopentyl-thioharnstoff F: 150-155°C

N-(2,6-Diisopropyl-phenyl)-N'-methallyl-thioharnstoff F: 93-95°C

N-(2,6-Diisopropyl-phenyl)-N'-hexyl-thioharnstoff

N-(2,6-Diisopropyl-phenyl)-N'-dodecyl-thioharnstoff

N-(2,6-Diisopropyl-phenyl)-N'-(2-äthyl-butyl)-thioharnstoff

N-(2,6-Diisopropyl-phenyl)-N'-(3,3-dimethyl-butyl)-thioharnstoff

N-(2,6-Diisopropyl-phenyl)-N'-(2,3-dimethyl-butyl)-thioharnstoff

N-(2,6-Diisopropyl-phenyl)-N'-(2,2,3-trimethyl-butyl)-thioharnstoff

N-(2,6-Diisopropyl-phenyl)-N'-(2,4,4-trimethyl-pentyl)-thioharnstoff

N-(2,6-Diisopropyl-phenyl)-N'-(2,3,3-trimethyl-allyl)-thioharnstoff

N-(2,6-Diisopropyl-phenyl)-N'-(cyclopentyl-methyl)-thioharnstoff

N-(2-Äthyl-6-isobutyl-phenyl)-N'-methallyl-thioharnstoff F: 105-106°C

N-(2-Äthyl-6-isobutyl-phenyl)-N'-butyl-thioharnstoff

N-(2-Äthyl-6-isobutyl-phenyl)-N'-neopentyl-thioharnstoff

N-(2-Äthyl-6-isobutyl-phenyl)-N'-isobutyl-thioharnstoff

N-(2-Methyl-6-isopropyl-phenyl)-N'-butyl-thioharnstoff

N-(2-Methyl-6-isopropyl-phenyl)-N'-isobutyl-thioharnstoff

N-(2-Methyl-6-isopropyl-phenyl)-N'-neopentyl-thioharnstoff

N-(2-Methyl-6-äthyl-phenyl)-N'-isobutyl-thioharnstoff

N-(2-Methyl-6-äthyl-phenyl)-N'-neopentyl-thioharnstoff

N-(2-Methyl-6-äthyl-phenyl)-N'-(3,3-dimethyl-butyl)-thioharnstoff

N-(2,6-Di-sek-butyl-phenyl)-N'-methyl-thioharnstoff

N-(2,6-Di-sek-butyl-phenyl)-N'-äthyl-thioharnstoff

N-(2,6-Di-sek-butyl-phenyl)-N'-propyl-thioharnstoff F: 85-87°C

N-(2,6-Di-sek-butyl-phenyl)-N'-butyl-thioharnstoff

N-(2,6-Di-sek-butyl-phenyl)-N'-isobutyl-thioharnstoff F: 83-85°C

N-(2,6-Di-sek-butyl-phenyl)-N'-allyl-thioharnstoff F: 83-86°C

N-(2,6-Di-sek-butyl-phenyl)-N'-methallyl-thioharnstoff F: 69-72°C

N-(2,6-Di-sek-butyl-phenyl)-N'-neopentyl-thioharnstoff F: 88-90°C

N-(2,6-Di-sek-butyl-phenyl)-N'-pentyl-thioharnstoff
N-(2,6-Di-sek-butyl-phenyl)-N'-hexyl-thioharnstoff
N-(2,6-Di-sek-butyl-phenyl)-N'-octyl-thioharnstoff
N-(2,6-Di-sek-butyl-phenyl)-N'-decyl-thioharnstoff
N-(2,6-Di-sek-butyl-phenyl)-N'-(2-äthyl-butyl)-thioharnstoff
N-(2,6-Di-sek-butyl-phenyl)-N'-(3,3-dimethyl-butyl)-thioharnstoff
N-(2,6-Di-sek-butyl-phenyl)-N'-(2-methyl-pentyl)-thioharnstoff
N-(2,6-Di-sek-butyl-phenyl)-N'-(2,3,3-trimethyl)-allyl-thioharnstoff

N-(2-Methyl-6-sek.-butyl-phenyl)-N'-propyl-thioharnstoff
N-(2-Äthyl-6-sek.-butyl-phenyl)-N'-methallyl-thioharnstoff
N-(2-Isopropyl-6-sek.-butyl-phenyl)-N'-isobutyl-thioharnstoff
N-(4-Methyl-2,6-di-sek.-butyl-phenyl)-N'-isobutyl-thioharnstoff F: 98-102°C
N-(4.Methyl-2,6-di-sek.-butyl-phenyl)-N'-butyl-thioharnstoff F: 97-100°C

N-(2,6-Di-cyclopentyl-phenyl)-N'-methyl-thioharnstoff
N-(2,6-Di-cyclopentyl-phenyl)-N'-butyl-thioharnstoff
N-(2,6-Di-cyclopentyl-phenyl)-N'-isobutyl-thioharnstoff
N-(2,6-Di-cyclopentyl-phenyl)-N'-neopentyl-thioharnstoff
N-(2,6-Di-cyclopentyl-phenyl)-N'-methallyl-thioharnstoff
N-(2,6-Di-cyclopentyl-phenyl)-N'-(cyclopropyl-methyl)-thioharnstoff
N-(2,6-Di-cyclopentyl-phenyl)-N'-pentyl-thioharnstoff

N-(2-Äthyl-6-cyclopentyl-phenyl)-N'-isobutyl-thioharnstoff

N-(2-Methyl-6-tert.-butyl-phenyl)-N'-isobutyl-thioharnstoff
N-(2-Methyl-6-tert.-butyl-phenyl)-N'-neopentyl-thioharnstoff

N-(2-Methyl-6-tert.-butyl-phenyl)-N'-methallyl-thioharnstoff
N-(2-Methyl-6-tert.-butyl-phenyl)-N'-pentyl-thioharnstoff

N-(2-Methyl-4,6-di-tert.-butyl-phenyl)-N'-methyl-thioharnstoff
N-(2-Methyl-4,6-di-tert.-butyl-phenyl)-N'-isobutyl-thioharnstoff
N-(2-Methyl-4,6-di-tert.-butyl-phenyl)-N'-methallyl-thioharnstoff

N-(4-Methyl-2,6-diisopropyl-phenyl)-N'-methyl-thioharnstoff
N-(4-Methyl-2,6-diisopropyl-phenyl)-N'-propyl-thioharnstoff F: 125-127°C
N-(4-Methyl-2,6-diisopropyl-phenyl)-N'-butyl-thioharnstoff F: 126-129°C
N-(4-Methyl-2,6-diisopropyl-phenyl)-N'-isobutyl-thioharnstoff F: 150-153°C
N-(4-Methyl-2,6-diisopropyl-phenyl)-N'-neopentyl-thioharnstoff
N-(4-Methyl-2,6-diisopropyl-phenyl)-N'-methallyl-thioharnstoff
N-(4-Methyl-2,6-diisopropyl-phenyl)-N'-hexyl-thioharnstoff

N-(4-Äthyl-2,6-diisopropyl-phenyl)-N'-isobutyl-thioharnstoff
N-(4-Äthyl-2,6-diisopropyl-phenyl)-N'-neopentyl-thioharnstoff

N-(2,4,6-triisopropyl-phenyl)-N'-methyl-thioharnstoff
N-(2,4,6-triisopropyl-phenyl)-N'-butyl-thioharnstoff
N-(2,4,6-triisopropyl-phenyl)-N'-allyl-thioharnstoff
N-(2,4,6-triisopropyl-phenyl)-N'-methallyl-thioharnstoff F: 115-118°C
N-(2,4,6-triisopropyl-phenyl)-N'-(3-methyl-pentyl)-thioharnstoff
N-(2,4,6-triisopropyl-phenyl)-N'-(2-äthyl-pentyl)-thioharnstoff

N-(2,6-Dimethyl-phenyl)-N'-butyl-thioharnstoff

N-(2,6-Dimethyl-phenyl)-N'-neopentyl-thioharnstoff
N-(2,6-Dimethyl-phenyl)-N'-methallyl-thioharnstoff
N-(2,6-Dimethyl-phenyl)-N'-isobutyl-thioharnstoff
N-(2,6-Dimethyl-phenyl)-N'-hexyl-thioharnstoff

N-(2-Methyl-6-äthyl-phenyl)-N'-methyl-thioharnstoff F: 65-67°C
N-(2,4-Dimethyl-6-äthyl-phenyl)-N'-methyl-thioharnstoff F: 125-126°C
N-(2,4-6-Triäthyl-phenyl)-N'-propyl-thioharnstoff
N-(2,4,6-Triäthyl-phenyl)-N'-butyl-thioharnstoff, Öl(F<30°C)
N-(2,4,6-Triäthyl-phenyl)-N'-isobutyl-thioharnstoff F:74-76°C
N-(2,4,6-Triäthyl-phenyl)-N'-methallyl-thioharnstoff
N-(2,4,6-Triäthyl-phenyl)-N'-neophentyl-thioharnstoff F:78-80°C
N-(4-Butyl-2,6-diäthyl-phenyl)-N'-isobutyl-thioharnstoff, Öl (F<30°C)
N-(4-Butyl-2,6-diäthyl-phenyl)-N'-neopentyl-thioharnstoff F:52-54°C
N-(4-Butyl-2,6-diäthyl-phenyl)-N'-crotyl-thioharnstoff
N-(4-butyl-2,6-diäthyl-phenyl)-N'-heptyl-thioharnstoff
N-(4-Cyclohexyl-2,6-diäthyl-phenyl)-N'-butyl-thioharnstoff F:63-66°C
N-(4-Cyclohexyl-2,6-diäthyl-phenyl)-N'-isobutyl-thioharnstoff F:118-120°
N-(4-Cyclopentyl-2,6-diäthyl-phenyl)-N'-isobutyl-thioharnstoff
N-(4-Cyclopentyl-2,6-diäthyl-phenyl)-N'-methallyl-thioharnstoff
N-(4-Cyclopentyl-2,6-diäthyl-phenyl)-N'-neopentyl-thioharnstoff
N-(4-Propyl-2,6-diäthyl-phenyl)-N'-n-pentyl-thioharnstoff; Öl (F<30°C)
N-(4-Isobutyl-2,6-diäthyl-phenyl)-N'-methyl-thioharnstoff F:123-126°C

N-(4-Isobutyl-2,6-diäthyl-phenyl)-N'-n-hexyl-thioharnstoff; Öl (F<30°C)

N-(4-tert.-Butyl-2,6-diäthyl-phenyl)-N'-isobutyl-thioharnstoff F:121-122°C

N-(4-tert.-Butyl-2,6-diäthyl-phenyl)-N'-butyl-thioharnstoff F:81-83°C

N-(4-tert.-Butyl-2,6-diäthyl-phenyl)-N'-n-pentyl-thioharnstoff, Öl(F<30°C)

N-(2-Methyl-4,6-di-tert.-butyl-phenyl)-N'-isobutyl-thioharnstoff F:157-160°C

N-(2-Methyl-4,6-di-tert.-butyl-phenyl)-N'-neopentyl-thioharnstoff F:157-159°C

N-(4-Methyl-2,6-di-cyclopentyl-phenyl)-N'-butyl-thioharnstoff F:151-153°C

N-(2,6-Diisopropyl-phenyl)-N'-tert.-butyl-thioharnstoff F: 135 - 137°C

N-(2,6-Diisopropyl-phenyl)-N'-tert.-pentyl-thioharnstoff F: 134 - 135°C

N-(2,6-Diisopropyl-phenyl)-N'-(1,1,2,2-tetramethyl-propyl) thioharnstoff F: 161 - 162°C

N-(2,6-Diäthyl-phenyl)-N'-tert.-butyl-thioharnstoff F: 98 - 100°C

N-(2,6-Diäthyl-phenyl)-N'-tert.-pentyl-thioharnstoff F: 85 - 87°C

N-(2,6-Di-sek.-butyl-phenyl)-N'-tert.-pentyl-thioharnstoff F: 116 - 118°C

N-(2,6-Di-sek.-butyl-phenyl)-N'-tert.-butyl-thioharnstoff F: 115 - 117°C

N-(2,6-Diäthyl-phenyl)-N'-isopropyl-thioharnstoff F: 108 - 110°C

N-(2,6-Diäthyl-phenyl)-N'-sek.-butyl-thioharnstoff
F: 78 - 80°C

N-(2,6-Di-sek.-butyl-phenyl)-N'-isopropyl-thioharnstoff
F: 108 - 111°C

N-(2,6-Di-sek.-butyl-phenyl)-N'-sek.-butyl-thioharnstoff
F: 103 - 105°C

N-(2,4-Dimethyl-6-äthyl-phenyl)-N'-tert.-butyl-thioharnstoff
F: 130 - 132°C

N-(2,4-Dimethyl-6-äthyl-phenyl)-N'-tert.-pentyl-thioharnstoff
F: 105 - 107°C

N-(4-Methyl-2,6-diäthyl-phenyl)-N'-tert.-butyl-thioharnstoff
F: 121 - 123°C

N-(4-Methyl-2,6-diäthyl-phenyl)-N'-tert.-pentyl-thioharnstoff
F: 98 - 101°C

N-(2-Methyl-6-äthyl-phenyl)-N'-tert.-butyl-thioharnstoff
F: 94 - 96°C

N-(2-Methyl-6-äthyl-phenyl)-N'-tert.-pentyl-thioharnstoff
F: 78 - 81°C

N-(2,4,6-Trimethyl-phenyl)-N'-tert.-pentyl-thioharnstoff

N-(2,6-Diäthyl-3-methyl-phenyl)-N'-tert.-butyl-thioharnstoff

N-(2,6-Diäthyl-3,5-dimethyl-phenyl)-N'-tert.-butyl-thioharnstoff

N-(2,6-Diäthyl-3,5-dimethyl-phenyl)-N'-pentyl-(2)-thioharnstoff

N-(2,6-Di-isopropyl-phenyl)-N'-[3-methyl-butyl-(2)]-thioharnstoff

N-(2,6-Di-sek.-butyl-phenyl)-N'-[pentyl-(2)]-thioharnstoff

N-(2,6-Di-sek.-butyl-phenyl)-N'-[hexyl-(2)]-thioharnstoff

N-(2,6-Di-sek.-butyl-phenyl)-N'-[pentyl-(3)]-thioharnstoff

N-(2,6-Di-sek.-butyl-phenyl)-N'-[4-methyl-pentyl-(2)]-thioharnstoff

N-(2,6-Di-sek.-butyl-phenyl)-N'-[2-methyl-pentyl-(3)]-thioharnstoff

N-(2,6-Diisopropyl-phenyl)-N'-[pentyl-(2)]-thioharnstoff

N-(2,6-Diisopropyl-phenyl)-N'-[hexyl-(2)]-thioharnstoff

N-(2,6-Diisopropyl-phenyl)-N'-[3-methyl-pentyl-(2)]-thioharnstoff

N-(2,6-Diisopropyl-phenyl)-N'-[pentyl-(3)]-thioharnstoff

N-(2,6-Diisopropyl-phenyl)-N'-[2,2-dimethyl-pentyl-(3)]-thioharnstoff

N-(2-Äthyl-6-sek.-butyl-phenyl)-N'-tert.-butyl-thioharnstoff
F: 96-99°C

N-(2-Isopropyl-6-sek.-butyl-phenyl)-N'-tert.-butyl-thioharnstoff
F: 121-123°C

N-(2-Isopropyl-6-sek.-butyl-phenyl)-N'-tert.-butyl-thioharnstoff
F: 51 - 53°C

N-(2,6-Diisopropyl-phenyl)-N'-[1-cyclopentyl-äthyl]-thioharnstoff

N-(2,6-Di-sek.-butyl-phenyl)-N'-[2-methyl-pentyl-(2)]-thioharnstoff

N-(2,6-Di-sek.-butyl-phenyl)-N'-[2-äthyl-pentyl-(2)]-thioharnstoff

N-(2,6-Di-sek.-butyl-phenyl)-N'-[2-methyl-hexyl-(2)]-thioharnstoff

N-(2,6-Diäthyl-phenyl)-N'-[2-methyl-octyl-(2)]-thioharnstoff

N-(2,6-Diisopropyl-phenyl)-N'-[2-methyl-pentyl-(2)]-thioharnstoff

N-(2,6-Diisopropyl-phenyl)-N'-[2-äthyl-pentyl-(2)]-thioharnstoff

N-(2,6-Diäthyl-3-chlor-phenyl)-N'-tert.-butyl-thioharnstoff

N-(2-Äthyl-6-sek.-butyl-phenyl)-N'-tert.-pentyl-thioharnstoff
F: 74 - 76°C

N-(2,6-Di-cyclopentyl-phenyl)-N'-tert.-butyl-thioharnstoff

N-(2-Äthyl-4-methyl-6-sek.-butyl-phenyl)-N'-tert.-butyl-thioharnstoff

N-(2,4-Dimethyl-6-tert.-butyl-phenyl)-N'-tert.-pentyl-thioharnstoff

N-[2,6-Di-pentyl-(2)-phenyl]-N'-tert.-butyl-thioharnstoff

N-(2,4-Dimethyl-6-cyclohexyl-phenyl)-N'-tert.-butyl-thioharnstoff

N-(2,6-Diisopropyl-4-chlorphenyl)-N'-tert.-butyl-thioharnstoff

N-(2,5-Dimethyl-6-äthyl-phenyl)-N'-tert.-pentyl-thioharnstoff

N-(2,5-Dimethyl-6-isopropyl-phenyl)-N'-tert.-pentyl-thioharnstoff

N-(2,6-Diäthyl-phenyl)-N'-[2-methyl-pentyl-(2)]-thioharnstoff

N-(2,6-Diäthyl-phenyl)-N'-[2-äthyl-pentyl-(2)]-thioharnstoff

N-(2,6-Diäthyl-phenyl)-N'-[2-methyl-hexyl-(2)]-thioharnstoff

N-(2,6-Diäthyl-phenyl)-N'-[decyl-(5)]-thioharnstoff

N-(2,6-Diäthyl-phenyl)-N'-[1,1,2,2-tetramethyl-propyl]-thioharnstoff

N-(2,6-Diäthyl-phenyl)-N'-[2,4-dimethyl-pentyl-(3)]-thioharnstoff

N-(2,6-Dimethyl-phenyl)-N'-[2,2-dimethyl-pentyl-(3)]-thioharnstoff

N-(2-Methyl-6-äthyl-phenyl)-N'-cyclopropyl-thioharnstoff

" " -N'-cyclopentyl-thioharnstoff

" " -N'-cyclohexyl-thioharnstoff

" " -N'-cycloheptyl-thioharnstoff

N-(2,4-Dimethyl-6-äthyl-phenyl)-N'-)3-methyl-cyclohexyl)-thioharnstoff

N-(2,6-Diäthyl-phenyl)-N'-cyclopropyl-thioharnstoff; F: 111 - 113°C

" " -N'-cyclobutyl-thioharnstoff

" " -N'-cyclopentyl-thioharnstoff

N-(2,6-Diäthyl-phenyl)-N'-(1-methyl-cyclopentyl)-thioharnstoff; F: 86 - 88°C
" " -N'-cyclohexyl-thioharnstoff
" " -N'-(2-methyl-cyclohexyl)-thioharnstoff; Öl
" " -N'-cycloheptyl-thioharnstoff
" " -N'-cyclooctyl-thioharnstoff
N-(4-Methyl-2,6-diäthyl-phenyl)-N'-cyclopropyl-thioharnstoff; F: 113 - 115°C
" " -N'-cyclopentyl-thioharnstoff
" " -N'-cyclohexyl-thioharnstoff
" " -N'-(4-methyl-cyclohexyl)-thioharnstoff
" " -N'-(3,3,5-trimethyl-cyclohexyl)-thioharnstoff
" " -N'-(2,6-diäthyl-cyclohexyl)-thioharnstoff
N-(2,4,6-Triäthyl-phenyl)-N'-cyclopropyl-thioharnstoff
" " -N'-cyclopentyl-thioharnstoff
" " -N'-cyclohexyl-thioharnstoff; F: 97 - 99°C
" " -N'-(3-trifluormethyl-cyclohexyl)-thioharnstoff; F: 52 - 55°C
" " -N'-(4-trifluormethyl-cyclohexyl)-thioharnstoff
" " -N'-(3,5-bis-trifluormethyl-cyclohexyl)-thioharnstoff
" " -N'-cyclopentyl-thioharnstoff
" " -N'-cyclooctyl-thioharnstoff
" " -N'-(2-norbornyl)-thioharnstoff
" " -N'-(1-adamantyl)-thioharnstoff

N-(4-n-Propyl-2,6-diäthyl-phenyl)-N'-cyclopentyl-thioharnstoff
" " -N'-cyclohexyl-thioharnstoff
" " -N'-(3-methyl-cyclohexyl)-thioharnstoff
" " -N'-(4-methyl-cyclohexyl)-thioharnstoff
" " -N'-(3-trifluormethyl-cyclohexyl)-thioharnstoff; Öl
" " -N'-(4-trifluormethyl-cyclohexyl)-thioharnstoff; Öl
" " -N'-(2,4-dimethyl-cyclohexyl)-thioharnstoff
" " -N'-(4-äthyl-cyclohexyl)-thioharnstoff
" " -N'-cyclopentyl-thioharnstoff
N-(4-Isopropyl-2,6-diäthyl-phenyl)-N'-cyclopentyl-thioharnstoff
" " -N'-cyclohexyl-thioharnstoff; F: 120 - 123°C
" " -N'-(3-trifluormethyl-cyclohexyl)-thioharnstoff; F: 127 - 140°C
" " -N'-(2-norbornyl)-thioharnstoff
" " -N'-(dekahydronaphthyl-1)-thioharnstoff
N-(4-n-Butyl-2,6-diäthyl-phenyl)-N'-cyclopentyl-thioharnstoff
" " -N'-cyclohexyl-thioharnstoff; Öl
" " -N'-(3-methyl-cyclohexyl)-thioharnstoff; Öl

N-(4-n-Butyl-2,6-diäthyl-phenyl)-N'-(4-methyl-cyclohexyl)-thioharnstoff

" " -N'-(4-äthyl-cyclohexyl)-thioharnstoff

" " -N'-(3-trifluormethyl-cyclohexyl)-thioharnstoff; Öl

" " -N'-cycloheptyl-thioharnstoff

N-(4-n-Pentyl-2,6-diäthyl-phenyl)-N'-cyclopentyl-thioharnstoff

" " -N'-cyclohexyl-thioharnstoff

" " -N'-cycloheptyl-thioharnstoff

N-(4-Pentyl-(3)-2,6-diäthyl-phenyl-N'-cyclopentyl-thioharnstoff

" " -N'-cyclohexyl-thioharnstoff

" " -N'-(3-trifluormethyl-cyclohexyl)-thioharnstoff,

" " -N'-cycloheptyl-thioharnstoff

N-(4-n-Hexyl-2,6-diäthyl-phenyl)-N'-cyclopentyl-thioharnstoff

" " -N'-cyclohexyl-thioharnstoff

N-(4-Cyclohexyl-2,6-diäthyl-phenyl)-N'-cyclopentyl-thioharnstoff

" " -N'-cyclopropyl-thioharnstoff

" " -N'-(3-trifluormethyl-cyclohexyl)-thioharnstoff; F: 118 - 125°C

N-(4-(1-Methyl-cyclohexyl)-2,6-diäthyl)-N'-cyclopentyl-thioharnstoff

N-(4-Cyclopentyl-2,6-diäthyl-phenyl)-N'-cyclopentyl-thioharnstoff

" " -N'-cyclohexyl-thioharnstoff

" " -N'-(cyclohexen-(2)-yl-thioharnstoff

N-(4-sec.-Butyl-2,6-diäthyl-phenyl)-N'-cyclopentyl-thioharnstoff

" " -N'-cyclohexyl-thioharnstoff; F: 140°C

" " -N'-(2-methyl-cyclohexyl)-thioharnstoff; Öl

" " -N'-(3-methyl-cyclohexyl)-thioharnstoff; Öl

" " -N'-(4-methyl-cyclohexyl)-thioharnstoff; F: 88 - 123°C

" " -N'-(3-trifluormethyl-cyclohexyl-thioharnstoff

" " -N'-cycloheptyl-thioharnstoff

" " -N'-(3,3,5-trimethyl-cyclohexyl-thioharnstoff; F: 58 - 80°C

N-(Isobutyl-2,6-diäthyl-phenyl)-N'-cyclopropyl-thioharnstoff

" " -N'-cyclobutyl-thioharnstoff

" " -N'-cyclopentyl-thioharnstoff; Öl

" " -N'-cyclohexyl-thioharnstoff; F: 41 - 44°C

" " -N'-(1-methyl-cyclopentyl)-thioharnstoff; F: 73 - 74°C

" " -N'-(2-methyl-cyclohexyl)-thioharnstoff; Harz

" " -N'-(3-methyl-cyclohexyl)-thioharnstoff; F: 57 - 85°C

N-(4-Isobutyl-2,6-diäthyl-phenyl)-N'-(4-methyl-cyclohexyl)-thioharnstoff; F: 70 - 165°C

" " -N'-(3-trifluormethyl-cyclohexyl)-thioharnstoff; F: 50 - 55°C

" " -N'-(4-trifluormethyl-cyclohexyl)-thioharnstoff; F: 52 - 60°C

" " -N'-(2,6-Dimethyl-cyclohexyl)-thioharnstoff

" " -N'-(2-norbornyl)-thioharnstoff

" " -N'-(1-adamantyl)-thioharnstoff

" " -N'-cycloheptyl-thioharnstoff

N-(4-Cycloheptyl-2,6-diäthyl-phenyl)-N'-cyclopentyl-thioharnstoff

N-(2,6-Diisopropyl-phenyl)-N'-cyclopropyl-thioharnstoff; F: 166 - 168°C

" " -N'-cyclopentyl-thioharnstoff; F: 140 - 142°C

" " -N'-(1-methyl-cyclopentyl)-thioharnstoff; F: 149 - 150°C

" " -N'-cyclohexyl-thioharnstoff; F: 120 - 122°C

" " -N'-(2-methyl-cyclohexyl)-thioharnstoff; F: 128 - 145°C

" " -N'-(3-methyl-cyclohexyl)-thioharnstoff; F: 62 - 76°C

N-(2,6-Diisopropyl-phenyl)-N'-(4-methyl-cyclohexyl)-thioharnstoff; F: 155 - 167°C

" " -N'-(2-trifluormethyl-cyclohexyl)-thioharnstoff; F: 187°C

" " -N'-(3-trifluormethyl-cyclohexyl)-thioharnstoff; F: 67 - 75°C

" " -N'-(4-trifluormethyl-cyclohexyl)-thioharnstoff; F: 142 - 165°C

N-(2,4,6-Triisopropyl-phenyl)-N'-cyclopropyl-thioharnstoff; F: 158 - 160°C

N-(2,6-Diisopropyl-phenyl)-N'-(4-äthyl-cyclohexyl)-thioharnstoff

" " -N'-(cyclohexen-(2)-yl)-thioharnstoff

" " -N'-(3,3,5-trimethyl-cyclohexyl)-thioharnstoff; F: 84 - 120°C

" " -N'-(3,5-bis-trifluormethylcyclohexyl)-thioharnstoff; F: 180 - 181°C

" " -N'-(2,4-dimethyl-cyclohexyl)-thioharnstoff

" " -N'-(3-trifluormethyl-4-methyl-cyclohexyl)-thioharnstoff; F: 145 - 165°C

" " -N'-(2-norbornyl)-thioharnstoff

" " -N'-(2-bornyl)-thioharnstoff

" " -N'-(1-adamantyl)-thioharnstoff

" " -N'-cycloheptyl-thioharnstoff

N-(2-Äthyl-6-isopropyl-phenyl)-N'-cyclohexyl-thioharnstoff

N-(4-Methyl-2,6-diisopropyl-phenyl)-N'-cyclopentyl-thioharnstoff

N-(2-Isopropyl-6-sec.-butyl-phenyl)-N'-cyclopentyl-thioharnstoff

N-(2,6-Di-sec.-butyl-phenyl)-N'-cyclopropyl-thioharnstoff; F: 151 - 153°C

" " -N'-cyclopentyl-thioharnstoff; F: 140 - 142°C

" " -N'-(1-methyl-cyclopentyl)-thioharnstoff

" " -N'-cyclohexyl-thioharnstoff

" " -N'-(3-methyl-cyclohexyl)-thioharnstoff

" " -N'-(3-trifluormethyl-cyclohexyl)-thioharnstoff; F: 58 - 67°C

" " -N'-(3,5-bis-trifluormethyl-cyclohexyl)-thioharnstoff; Öl

" " -N'-(2-norbornyl)-thioharnstoff

" " -N'-cycloheptyl-thioharnstoff

N-(2-Äthyl-6-sec.-butyl-phenyl)-N'-cyclopropyl-thioharnstoff; F: 122 - 123°C

N-(2,6-Di-cyclopentyl)-phenyl)-N'-cyclopentyl-thioharnstoff; F: 168 - 170°C

" " -N'-cyclohexyl-thioharnstoff

N-(2,6-Di-pentyl-(2)-phenyl)-N'-cyclopentyl-thioharnstoff

" " -N'-cyclohexyl-thioharnstoff

N-(3-Chlor-2,6-diäthyl-phenyl)-N'-cyclopropyl-thioharnstoff; F: 130 - 131°C

N-(3-Methyl-2,6-diäthyl-phenyl)-N'-cyclopropyl-thioharnstoff; F: 145 - 146°C

N-(4-Methyl-(2,6-di-sec.-butyl-phenyl)-N'-cyclopropyl-thioharnstoff; F: 154 - 156°C

N-(2,4,6-Diäthyl-phenyl)-N'-cycloheptyl-thioharnstoff; F: 84 - 86°C

N-(4-n-Propyl-2,6-diäthyl-phenyl)-N'-cycloheptyl-thioharnstoff; Öl

N-(4-Isopropyl-2,6-diäthyl-phenyl)-N'-cycloheptyl-thioharnstoff; F: 89 - 92°C

N-(4-n-Butyl-2,6-diäthyl-phenyl)-N'-cycloheptyl-thioharnstoff; Öl

Die N-(2,6-Dialkyl-phenyl)-N'-alkyl-thioharnstoffe der allgemeinen Formel II werden erfindungsgemäß mit Verbindungen der allgemeinen Formel III umgesetzt. An Verbindungen III seien Alkylierungs- Alkenylierungs- bzw. Cycloalkylierungsmittel beispielsweise genannt: Methylchlorid, Methylbromid, Methyljodid, Dimethylsulfat, Methansulfonsäuremethylester, Toluolsulfonsäuremethylester, Trimethylphosphat, Fluorsulfonsäuremethylester, Äthylchlorid, Äthylbromid, Äthyljodid, Triäthyloxonium-fluorborat, Diäthylsulfat, Propylbromid, Propyljodid, Butylbromid, Butyljodid, Hexyljodid, Allylbromid, Methallylchlorid, Methallylbromid, Crotylbromid, 3,3-Dimethylallylchlorid, Buten-(3)-yl-(1)-bromid, 3-Methyl-butylbromid, Isobutylbromid, Cyclopropylbromid, Cyclopentylbromid, Cyclohexyljodid.

Die Umsetzung der Thioharnstoffe II mit den Verbindungen der Formel III erfolgt im allgemeinen in Lösungsmitteln bei ca. 10°C bis ca. 120°C, vorzugsweise bei ca. 20°C bis ca. 80°C. Geeignete Lösungsmittel sind z.B. Kohlenwasserstoffe oder Halogenkohlenwasserstoffe wie Hexan, Cyclohexan, Methylcyclohexan, Octan, Methylenchlorid, Chloroform, 1,2-Dichloräthylen, weiterhin Aromaten wie Benzol, Toluol, Xylol oder polare Lösungsmittel z.B. Äther und Carbonsäurederivate wie Diäthyläther, Diisopropyläther, Tetrahydrofuran, Dioxan, 1,2-Dimethoxyäthan, Acetonitril, Essigsäureäthylester, Dimethylformamid, N-Methyl-pyrrolidon, Phosphorsäure-trisdimethylamid, Dimethylsulfoxid, Aceton, Butanon, Methanol, Äthanol, Isopropanol.

Die Konzentration an Thioharnstoff II im verwendeten Lösungsmittel wird zweckmäßigerweise möglichst hoch gehalten, man arbeitet z.B. mit einer Konzentration von 10 - 70 Prozent Thioharnstoff II im Lösungsmittel.

Bei leicht flüchtigen Verbindungen der Formel III wie Methylchlorid oder Äthylchlorid, kann auch im geschlossenen Gefäß unter Druck gearbeitet werden.

Man arbeitet mit mindestens äquimolaren Mengen an Verbindung der Formel III, bezogen auf eingesetzten Thioharnstoff II, vorzugsweise jedoch mit einem Überschuß von 5 bis 50 Molprozent, vorzugsweise 5 - 30 Molprozent der Komponente III. Bei schwierig verlaufender Alkylierung, Alkenylierung oder Cycloalkylierung kann auch ein höherer Überschuß verwendet und die Verbindung der Formel III anschließend zurückgewonnen werden. Es ist aber auch möglich, ohne Lösungsmittel in überschüssigem Alkylierungs-, Alkenylierungs- bzw. Cycloalkylierungsmittel als Lösungs- und Verdünnungsmittel zu arbeiten.

Bei den angegebenen Verfahren fallen die erfindungsgemäßen Isothioharnstoffe der allgemeinen Formel I primär in Form ihrer Salze an und zwar in Abhängigkeit von der jeweils verwendeten Verbindung der Formel III beipsielsweise als Hydrochlorid, Hydrobromid, Hydrojodid, Methansulfonat, p-Toluolsulfonat, Methosulfat, Sulfat usw.

Im allgemeinen erfolgt bei der Aufarbeitung eine Neutralisation mit einer wäßrigen Base, beispielsweise Sodalösung, Kaliumcarbonatlösung, verdünnte Natronlauge oder andere Basen, wobei die erfindungsgemäßen Isothioharnstoffe entweder als Lösung in einem wasserunlöslichen Lösungsmittel oder lösungsmittelfrei als viskose Öle unter der wäßrigen Phase anfallen. Die übrige Aufarbeitung zu den Reinsubstanzen erfolgt nach den üblichen in der präparativen organischen Chemie wohlbekannten Methoden.

An erfindungsgemäß herstellbaren Isothioharnstoffen der allgemeinen Formel I seien beispielsweise genannt:

N-(2-Methyl-6-äthyl-phenyl)-N'-tert.-butyl-S-methyl-isothioharnstoff

N-(2-Methyl-6-äthyl-phenyl)-N'-isopropyl-S-allyl-isothioharnstoff

N-(2-Methyl-6-äthyl-phenyl)-N'-cyclohexyl-S-crotyl-isothioharnstoff

N-(2-Methyl-6-äthyl-phenyl)-N'-norbornyl-S-methyl-isothioharnstoff

N-(2,6-Diäthyl-phenyl)-N'-isopropyl-S-methyl-isothioharnstoff

N-(2,6-Diäthyl-phenyl)-N'-butyl-S-methyl-isothioharnstoff

N-(2,6-Diäthyl-phenyl)-N'-sek.-butyl-S-äthyl-isothioharnstoff

N-(2,6-Diäthyl-phenyl)-N'-isobutyl-S-methyl isothioharnstoff

N-(2,6-Diäthyl-phenyl)-N'-tert.-butyl-S-methyl-isothioharnstoff

N-(2,6-Diäthyl-phenyl)-N'-pentyl-(2)-S-methyl-isothioharnstoff

N-(2,6-Diäthyl-phenyl)-N'-pentyl-(3)-S-methyl-isothioharnstoff

N-(2,6-Diäthyl-phenyl)-N'-heptyl-(2)-S-methyl-isothioharnstoff

N-(2,6-Diäthyl-phenyl)-N'-octyl-(2)-S-methyl-isothioharnstoff

N-(2,6-Diäthyl-phenyl)-N'-cyclopropyl-S-allyl-isothioharnstoff

N-(2,6-Diäthyl-phenyl)-N'-cyclopentyl-S-methyl-isothioharnstoff

N-(2,6-Diäthyl-phenyl)-N'-cyclohexyl-S-methyl-isothioharnstoff

N-(2,6-Diäthyl-phenyl)-N'-(3-trifluormethyl-cyclohexyl)-S-methyl-isothioharnstoff

N-(2,6-Diäthyl-phenyl)-N'-(4-äthyl-cyclohexyl)-S-methyl-isothioharnstoff

N-(2,6-Diäthyl-phenyl)-N'-cycloheptyl-S-methyl-isothioharnstoff

N-(2,6-Diäthyl-phenyl)-N'-norbornyl-S-methyl-isothioharnstoff

N-(2,6-Diäthyl-phenyl)-N'-bornyl-S-methyl-isothioharnstoff
N-(2,6-Diäthyl-phenyl)-N'-methyl-S-cyclohexyl-isothioharnstoff,
N-(2-Äthyl-6-isopropyl-phenyl)-N'-tert.-butyl-S-methyl-isothioharnstoff
N-(2-Äthyl-6-isopropyl-phenyl)-N'-pentyl-(2)-S-methyl-isothioharnstoff
N-(2-Äthyl-6-isopropyl-phenyl)-N'-cyclohexyl-S-methyl-isothioharnstoff
N-(2,6-Diisopropyl-phenyl)-N'-äthyl-S-äthyl-isothioharnstoff
N-(2,6-Diisopropyl-phenyl)-N'-isopropyl-S-methyl-isothioharnstoff
N-(2,6-Diisopropyl-phenyl)-N'-butyl-S-methyl-isothioharnstoff
N-(2,6-Diisopropyl-phenyl)-N'-sek.-butyl-S-methyl-isothioharnstoff
N-(2,6-Diisopropyl-phenyl)-N'-isobutyl-S-methyl-isothioharnstoff
N-(2,6-Diisopropyl-phenyl)-N'-tert.-butyl-S-methyl-isothioharnstoff
N-(2,6-Diisopropyl-phenyl)-N'-pentyl-S-methyl-isothioharnstoff
N-(2,6-Diisopropyl-phenyl)-N'-pentyl-(2)-S-methyl-isothioharnstoff
N-(2,6-Diisopropyl-phenyl)-N'-pentyl-(3)-S-methyl-isothioharnstoff
N-(2,6-Diisopropyl-phenyl)-N'-(2-methyl-butyl)-S-methyl-isothioharnstoff
N-(2,6-Diisopropyl-phenyl)-N'-(2,2-dimethyl-propyl)-S-methyl-isothioharnstoff

N-(2,6-Diisopropyl-phenyl)-N'-tert.-pentyl-S-methyl-isothioharnstoff

N-(2,6-Diisopropyl-phenyl)-N'-hexyl-(2)-S-methyl-isothioharnstoff

N-(2,6-Diisopropyl-phenyl)-N'-heptyl-(3)-S-methyl-isothioharnstoff

N-(2,6-Diisopropyl-phenyl)-N'-(2,2,3,3-tetramethyl-butyl)-S-methyl-isothioharnstoff

N-(2,6-Diisopropyl-phenyl)-N'-cyclopropyl-S-crotyl-isothioharnstoff

N-(2,6-Diisopropyl-phenyl)-N'-cyclopentyl-S-methyl-isothioharnstoff

N-(2,6-Diisopropyl-phenyl)-N'-cyclohexyl-S-methyl-isothioharnstoff

N-(2,6-Diisopropyl-phenyl)-N'-(3-trifluormethyl-cyclohexyl)-S-methyl-isothioharnstoff

N-(2,6-Diisopropyl-phenyl)-N'-(3,5-bis-trifluormethyl-cyclohexyl)-S-methyl-isothioharnstoff

N-(2,6-Diisopropyl-phenyl)-N'-cycloheptyl-S-methyl-isothioharnstoff

N-(2,6-Diisopropyl-phenyl)-N'-norbornyl-S-methyl-isothioharnstoff

N-(2,6-Diisopropyl-phenyl)-N'-methyl-S-cyclohexyl-isothioharnstoff

N-(2-Isopropyl-6-sek.-butyl-phenyl)-N'-tert.-butyl-S-methyl-isothioharnstoff

N-(2-Isopropyl-6-sek.-butyl-phenyl)-N'-pentyl-(2)-S-methyl-isothioharnstoff

N-(2,6-Di-sek.-butyl-phenyl)-N'-isopropyl-S-methyl-isothioharnstoff

N-(2,6-Di-sek.-butyl-phenyl)-N'-propyl-S-methyl-isothioharnstoff

N-(2,6-Di-sec.-butyl-phenyl)-N'-sec.-butyl-S-methyl-isothioharnstoff

N-(2,6-Di-sec.-butyl-phenyl)-N'-tert.-butyl-S-methyl-isothioharnstoff

N-(2,6-Di-sec.-butyl-phenyl)-N'-pentyl-(2)-S-methyl-isothioharnstoff

N-(2,6-Di-sec.-butyl-phenyl)-N'-heptyl-(3)-S-methyl-isothioharnstoff

N-(2,6-Di-sec.-butyl-phenyl)-N'-cyclopentyl-S-methyl-isothioharnstoff

N-(2,6-Di-sec.-butyl-phenyl)-N'-cyclohexyl-S-methyl-isothioharnstoff

N-(2,6-Di-sec.-butyl-phenyl)-N'-norbornyl-S-methyl-isothioharnstoff

N-[2,6-Bis-pentyl-(2)-phenyl]-N'-isopropyl-S-methyl-isothioharnstoff

N-[2,6-Bis-pentyl-(2)-phenyl]-N'-sec.-butyl-S-methyl-isothioharnstoff

N-[2,6-Bis-pentyl-(2)-phenyl]-N'-tert.-butyl-S-methyl-isothioharnstoff

N-(2-Äthyl-6-sec.-butyl-phenyl)-N'-tert.-butyl-S-methyl-isothioharnstoff

N-(2,6-Dicyclopentyl-phenyl)-N'-isopropyl-S-methyl-isothioharnstoff

N-(2,6-Dicyclopentyl-phenyl)-N'-tert.-butyl-S-methyl-isothioharnstoff

N-(2-Äthyl-6-cyclohexyl-phenyl)-N'-tert.-butyl-S-methyl-isothioharnstoff

N-(4-Methyl-2,6-diäthyl-phenyl)-N'-isopropyl-S-methyl-isothioharnstoff

N-(4-Methyl-2,6-diäthyl-phenyl)-N'-tert.-butyl-S-methyl-isothioharnstoff

N-(4-Methyl-2,6-diäthyl-phenyl)-N'-methyl-S-pentyl-isothioharnstoff

N-(4-Methyl-2,6-diäthyl-phenyl)-N'-propyl-S-cyclopentyl-isothioharnstoff

N-(4-Methyl-2,6-diäthyl-phenyl)-N'-sec.-butyl-S-methyl-isothioharnstoff

N-(4-Methyl-2,6-diäthyl-phenyl)-N'-pentyl-(2)-S-methyl-isothioharnstoff

N-(4-Methyl-2,6-diäthyl-phenyl)-N'-heptyl-(3)-S-methyl-isothioharnstoff

N-(Methyl-2,6-diäthyl-phenyl)-N'-octyl-(2)-S-methyl-isothioharnstoff

N-(4-Methyl-2,6-diäthyl-phenyl)-N'-cyclopentyl-S-methyl-isothioharnstoff

N-(4-Methyl-2,6-diäthyl-phenyl)-N'-cyclohexyl-S-methyl-isothioharnstoff

N-(4-Methyl-2,6-diäthyl-phenyl)-N'-norbronyl-S-methyl-isothioharnstoff

N-(4-Methyl-2,6-diäthyl-phenyl)-N'-dekahydronaphthyl-(1)-S-methyl-isothioharnstoff

N-(4-Methyl-2,6-diisopropyl-phenyl)-N'-tert.-butyl-S-methyl-isothioharnstoff

N-(4-Methyl-2,6-diisopropyl-phenyl)-N'-pentyl-(2)-S-methyl-isothioharnstoff

N-(4-Methyl-2,6-diisopropyl-phenyl)-N'-isopropyl-S-methyl-isothioharnstoff

N-(4-Methyl-2,6-diisopropyl-phenyl)-N'-cyclohexyl-S-methyl-isothioharnstoff

N-(4-Methyl-2,6-di-sec.-butyl-phenyl)-N'-tert.-butyl-S-methyl-isothioharnstoff

N-(2,4,6-Triäthyl-phenyl)-N'-methyl-S-crotyl-isothioharnstoff

N-(2,4,6-Triäthyl-phenyl)-N'-methyl-S-cyclopentyl-isothioharnstoff

N-(2,4,6-Triäthyl-phenyl-N'-isopropyl-S-methyl-isothioharnstoff

N-(2,4,6-Triisopropyl-phenyl)-N'-isopropyl-S-methyl-isothioharnstoff

N-(2,4,6-Triäthyl-phenyl)-N'-sec.-butyl-S-methyl-isothioharnstoff

N-(2,4,6-Triäthyl-phenyl)-N'-isobutyl-S-methyl-isothioharnstoff

N-(2,4,6-Triäthyl-phenyl)-N'-tert.-butyl-S-methyl-isothioharnstoff

N-(2,4,6-Triäthyl-phenyl)-N'-pentyl-(2)-S-methyl-isothioharnstoff

N-(2,4,6-Triäthyl-phenyl)-N'-heptyl-(3)-S-methyl-isothioharnstoff

N-(2,4,6-Triäthyl-phenyl)-N'-cyclohexyl-S-methyl-isothioharnstoff

N-(2,4,6-Triäthyl-phenyl)-N'-cycloheptyl-S-methyl-isothioharnstoff

N-(2,4,6-Triäthyl-phenyl)-N'-cyclooctyl-S-methyl-isothioharnstoff

N-(2,4,6-Triäthyl-phenyl)-N'-(trifluormethyl-cyclohexyl)-S-methyl-isothioharnstoff

N-(2,4,6-Triäthyl-phenyl)-N'-norbornyl-S-methyl-isothioharnstoff

N-(2,4,6-Triäthyl-phenyl)-N'-bornyl-S-methyl-isothioharnstoff

N-(4-Propyl-2,6-diäthyl-phenyl)-N'-äthyl-S-butyl-isothioharnstoff

N-(4-Propyl-2,6-diäthyl-phenyl)-N'-tert.-butyl-S-methyl-isothioharnstoff

N-(4-Propyl-2,6-diäthyl-phenyl)-N'-tert.-pentyl-S-methyl-isothioharnstoff

N-(4-Propyl-2,6-diäthyl-phenyl)-N'-(1,1-dimethylpropyl)-S-methyl-isothioharnstoff

N-(4-Propyl-2,6-diäthyl-phenyl)-N'-cycloheptyl-S-methyl-isothioharnstoff

N-(4-Isopropyl-2,6-diäthyl-phenyl)-N'-tert.-butyl-S-methyl-isothioharnstoff

N-(4-Isopropyl-2,6-diäthyl-phenyl)-N'-isopropyl-S-methyl-isothioharnstoff

N-(4-Isopropyl-2,6-diäthyl-phenyl)-N'-sec.-butyl-S-methyl-isothioharnstoff

N-(4-Isopropyl-2,6-diäthyl-phenyl)-N'-cyclohexyl-S-methyl-isothioharnstoff

N-(4-Isopropyl-2,6-diäthyl-phenyl)-N'-bornyl-S-methyl-isothioharnstoff

N-(4-Butyl-2,6-diäthyl-phenyl)-N'-isopropyl-S-methyl-isothioharnstoff

N-(4-Butyl-2,6-diäthyl-phenyl)-N'-sec.-butyl-S-methyl-isothioharnstoff

N-(4-Butyl-2,6-diäthyl-phenyl)-N'-tert.-butyl-S-methyl-isothioharnstoff

N-(4-Butyl-2,6-diäthyl-phenyl)-N'-cyclopentyl-S-methyl-isothioharnstoff

N-(4-Butyl-2,6-diäthyl-phenyl)-N'-norbornyl-S-methyl-isothioharnstoff

N-(4-sec.-Butyl-2,6-diäthyl-phenyl)-N'-sec.-butyl-S-methyl-isothioharnstoff

N-(4-sec.-Butyl-2,6-diäthyl-phenyl)-N'-tert.-butyl-S-methyl-isothioharnstoff

N-(4-sec.-Butyl-2,6-diäthyl-phenyl)-N'-cyclohexyl-S-methyl-isothioharnstoff

N-(4-Isobutyl-2,6-diäthyl-phenyl)-N'-tert.-butyl-S-methyl-isothioharnstoff

N-(4-Isobutyl-2,6-diäthyl-phenyl)-N'-cyclohexyl-S-methyl-isothioharnstoff

N-(4-Isobutyl-2,6-diäthyl-phenyl)-N'-norbornyl-S-methyl-isothioharnstoff

N-(4-tert.-Butyl-2,6-diäthyl-phenyl)-N'-isopropyl-S-methyl-isothioharnstoff

N-(4-tert.-Butyl-2,6-diäthyl-phenyl)-N'-sec.-butyl-S-methyl-isothioharnstoff

N-(4-tert.-Butyl-2,6-diäthyl-phenyl)-N'-tert.-butyl-S-methyl-isothioharnstoff

N-(4-tert.-Butyl-2,6-diäthyl-phenyl)-N'-tert.-pentyl-S-methyl-isothioharnstoff

N-(4-tert.-Butyl-2,6-diäthyl-phenyl)-N'-heptyl-(3)-S-methyl-isothioharnstoff

N-(4-tert.-Butyl-2,6-diäthyl-phenyl)-N'-cyclohexyl-S-methyl-isothioharnstoff

N-(4-Pentyl-2,6-diäthyl-phenyl)-N'-tert.-butyl-S-methyl-isothioharnstoff

N-(4-Pentyl-2,5-diäthyl-phenyl)-N'-cyclopentyl-S-methyl-isothioharnstoff

N-(4-Pentyl-2,6-diäthyl-phenyl)-N'-norbornyl-S-methyl-isothioharnstoff

N-[4-Pentyl-(3)-2,6-diäthyl-phenyl]-N'-isopropyl-S-methyl-isothioharnstoff

N-[4-Pentyl-(3)-2,6-diäthyl-phenyl]-N'-sec.-butyl-S-methyl-isothioharnstoff

N-[4-Pentyl-(3)-2,6-diäthyl-phenyl]-N'-tert.-butyl-S-methyl-isothioharnstoff

N-[4-Pentyl-(3)-2,6-diäthyl-phenyl]-N'-pentyl-(3)-S-methyl-isothioharnstoff

N-[4-Pentyl-(3)-2,6-diäthyl-phenyl]-N'-cyclohexyl-S-methyl-isothioharnstoff

N-(4-Cyclopentyl-2,6-diäthyl-phenyl)-N'-tert.-butyl-S-methyl-isothioharnstoff

N-(2,4,6-Triisopropyl-phenyl)-N'-tert.-butyl-S-methyl-isothioharnstoff

N-(2,4,6-Triisopropyl-phenyl)-N'-pentyl-(2)-S-methyl-isothioharnstoff

N-(2,4,6-Triisopropyl-phenyl)-N'-cyclopentyl-S-methyl-isothioharnstoff

N-(2,4,6-Triisopropyl-phenyl)-N'-norbornyl-S-methyl-isothioharnstoff

N-(2-Äthyl-4-methyl-6-sec.-butyl-phenyl)-N'-tert.-butyl-S-methyl-isothioharnstoff

N-(2,6-Dicyclohexyl-phenyl)-N'-isopropyl-S-methyl-isothioharnstoff

N-(4-n-Hexyl-2,6-diäthyl-phenyl)-N'-tert.-butyl-S-methyl-isothioharnstoff

N-(4-n-Hexyl-2,6-diäthyl-phenyl)-N'-sec.-butyl-S-methyl-isothioharnstoff

N-(4-Cyclohexyl-2,6-diäthyl-phenyl)-N'-isopropyl-S-methyl-isothioharnstoff

N-(4-Cyclohexyl-2,6-diäthyl-phenyl)-N'-sec.-butyl-S-methyl-isothioharnstoff

N-(4-Cyclohexyl-2,6-diäthyl-phenyl)-N'-tert.-butyl-S-methyl-isothioharnstoff

N-(4-Cyclohexyl-2,6-diäthyl-phenyl)-N'-cyclopentyl-S-methyl-isothioharnstoff

N-(4-Cyclohexyl-2,6-diäthyl-phenyl)-N'-norbornyl-S-methyl-isothioharnstoff

N-(4-Cycloheptyl-2,6-diäthyl-phenyl)-N'-tert.-butyl-S-methyl-isothioharnstoff

N-/4-(1-Methyl-cyclohexyl)-2,6-diäthyl-phenyl)-N'-tert.-butyl-S-methyl-isothioharnstoff

N-(4-Fluor-2,6-diäthyl-phenyl)-N'-tert.-butyl-S-methyl-isothioharnstoff

N-(4-Fluor-2,6-diäthyl-phenyl)-N'-cyclohexyl-S-methyl-isothioharnstoff

N-(3-Methyl-2,6-diäthyl-phenyl)-N'-tert.-butyl-S-methyl-isothioharnstoff

N-(3-Chlor-2,6-diäthyl-phenyl)-N'-tert.-butyl-S-methyl-isothioharnstoff

N-(3,5-Dimethyl-2,6-diäthyl-phenyl)-N'-tert.-butyl-S-methyl-isothioharnstoff

N-(3-Brom-2,6-diäthyl-phenyl)-N'-tert.-butyl-S-methyl-isothioharnstoff

N-(4-Fluor-2,6-diäthyl-phenyl)-N'-norbornyl-S-methyl-isothioharnstoff

N-(3,4-Dimethyl-2,6-diäthyl-phenyl)-N'-tert.-butyl-S-methyl-isothioharnstoff

N-(2,5-Dimethyl-6-äthyl-phenyl)-N'-tert.-butyl-S-methyl-isothioharnstoff

N-(4-Methyl-2,6-diäthyl-phenyl)-N'-adamantyl-S-methyl-isothioharnstoff

Die Wirkstoffe eignen sich bei guter Pflanzenverträglichkeit und günstiger Warmblütertoxizität vor allem zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp..
Aus der Ordnung der Anoplura z. B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae,

Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp.,

Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.
Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on und spot-on) und des Einpuderns sowie durch parenterale Anwendung in Form beispielsweise der Injektion.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

<u>Beispiel A</u>

Plutella-Test

| | | |
|---|---|---|
| Lösungsmittel: | 3 Gewichtsteile | Dimethylformamid |
| Emulgator : | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung besprüht man Kohlblätter (Brassica oleracea) taufeucht und besetzt sie mit Raupen der Kohlschabe (Plutella maculipennis).

Nach den angegebenen Zeiten wird die Abtötung in % bestimmt. Dabei bedeutet 100 %, daß alle Raupen abgetötet wurden; 0 % bedeutet, daß keine Raupen abgetötet wurden.

Wirkstoffe, Wirkstoffkonzentrationen, Auswertungszeiten und Resultate gehen aus der nachfolgenden Tabelle hervor:

**T a b e l l e**

(pflanzenschädigende Insekten)

Plutella-Test

| Wirkstoffe | Wirkstoffkonzentration in % | Abtötungsgrad in % nach 3 Tagen |
|---|---|---|
| 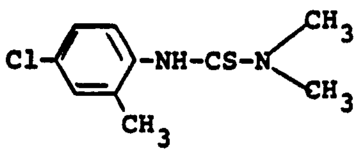<br>(bekannt) | 0,1<br>0,01 | 80<br>0 |
| 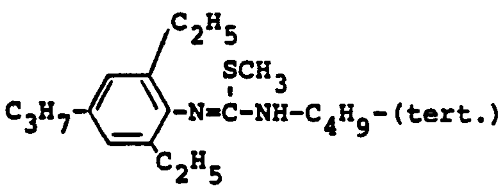 | 0,1<br>0,01 | 100<br>100 |
| 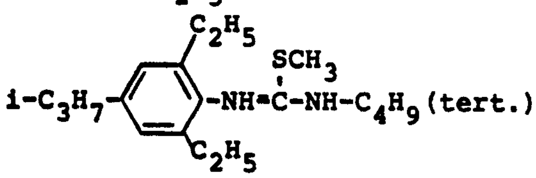 | 0,1<br>0,01 | 100<br>100 |
| 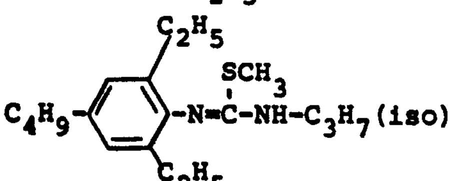 | 0,1<br>0,01 | 100<br>100 |
| 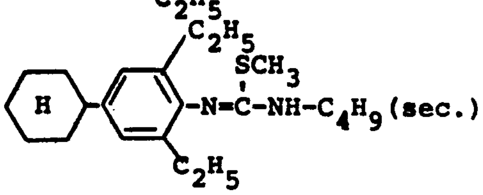 | 0,1<br>0,01 | 100<br>100 |
| 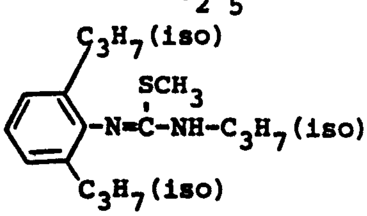 | 0,1<br>0,01 | 100<br>100 |

**T a b e l l e** (Fortsetzung)
(pflanzenschädigende Insekten)
Plutella-Test

| Wirkstoffe | Wirkstoffkonzentration in % | Abtötungsgrad in % nach 3 Tagen |
|---|---|---|
| 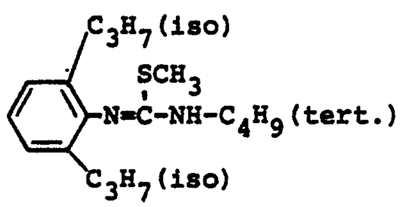 | 0,1<br>0,01 | 100<br>100 |
| 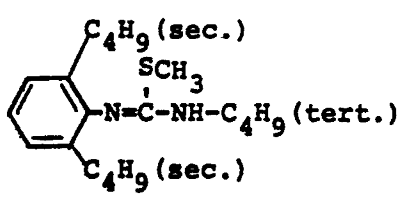 | 0,1<br>0,01 | 100<br>100 |

**Beispiel B**

Tetranychus-Test (resistent)

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Bohnenpflanzen (Phaseolus vulgaris), die stark von allen Entwicklungsstadien der gemeinen Spinnmilbe oder Bohnenspinnmilbe (Tetranychus urticae) befallen sind, tropfnaß besprüht.

Nach den angegebenen Zeiten wird die Abtötung in % bestimmt. Dabei bedeutet 100 %, daß alle Spinnmilben abgetötet wurden; 0 % bedeutet, daß keine Spinnmilben abgetötet wurden.

Wirkstoffe, Wirkstoffkonzentrationen, Auswertungszeiten und Resultate gehen aus der nachfolgenden Tabelle hervor:

<u>T a b e l l e</u>

(pflanzenschädigende Insekten)

Tetranychus-Test

| Wirkstoffe | Wirkstoffkonzentration in % | Abtötungsgrad in % nach 2 Tagen |
|---|---|---|
| 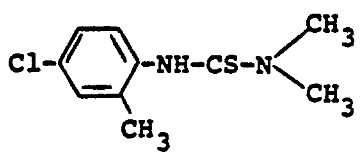 | 0,1 | 60 |
| | 0,01 | 0 |
| (bekannt) | | |
| 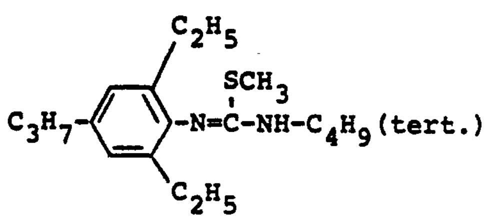 | 0,1 | 100 |
| | 0,01 | 100 |
| 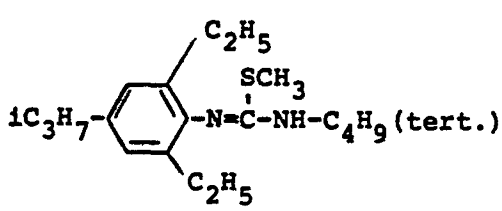 | 0,1 | 100 |
| | 0,01 | 100 |
| 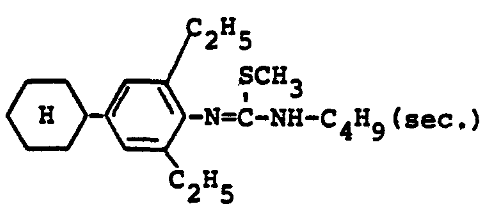 | 0,1 | 100 |
| | 0,01 | 100 |

Beispiel C

Mückenlarven-Test

Testtiere: Aëdes aegypti

Lösungsmittel: 99 Gewichtsteile Aceton
Emulgator: 1 Gewichtsteil Benzylhydroxydiphenylpolyglykoläther

Zur Herstellung einer zweckmäßigen Wirkstoffzubereitung löst man 2 Gewichtsteile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wässrigen Wirkstoffzubereitungen in Gläser und setzt anschließend etwa 25 Mückenlarven in jedes Glas ein.

Nach 24 Stunden wird der Abtötungsgrad in % bestimmt. Dabei bedeutet 100 %, daß alle Larven getötet worden sind, 0 % bedeutet, daß überhaupt keine Larven getötet worden sind.

Wirkstoffe, Wirkstoffkonzentrationen, Testtiere und Ergebnisse gehen aus der nachfolgenden Tabelle hervor:

**T a b e l l e**

Mückenlarven-Test

| Wirkstoffe | Wirkstoffkonzentration der Lösung in ppm | Abtötungsgrad in % |
|---|---|---|
| **Bekannt:** | | |
| 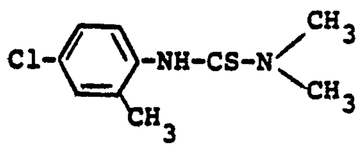 | 10 | 0 |
| **Erfindungsgemäß:** | | |
| 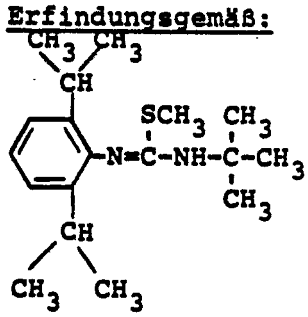 | 1 | 100 |
| 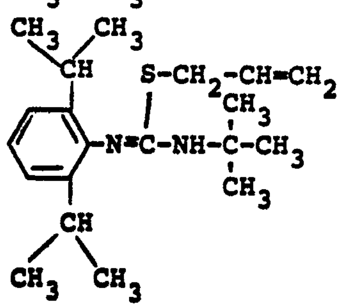 | 10 | 100 |
| | 1 | 90 |
| 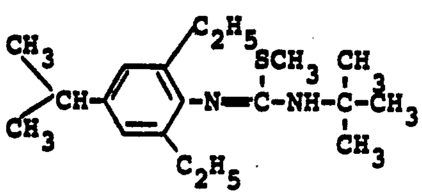 | 10 | 100 |
| | 1 | 90 |

**Tabelle** (Fortsetzung)
Mückenlarven-Test

| Wirkstoffe | Wirkstoffkonzentration der Lösung in ppm | Abtötungsgrad in % |
|---|---|---|
| 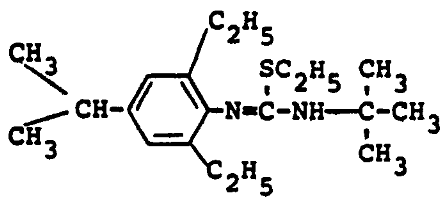 | 1 | 100 |
| 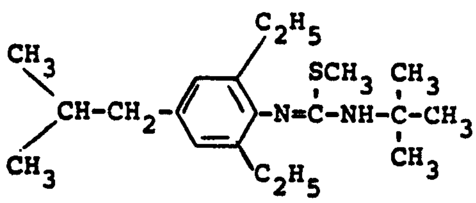 | 10<br>1 | 100<br>90 |

**Beispiel D**

$LD_{100}$-Test

Testtiere: Sitophilus granarius und Blatta orientalis

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstoffflösung ist die Menge Wirkstoff pro $m^2$ Filterpapier verschieden hoch. Anschließend gibt man etwa 10 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Wirkstoffe, Wirkstoffkonzentrationen, Testtiere und Ergebnisse gehen aus der nachfolgenden Tabelle hervor:

T a b e l l e

$LD_{100}$-Test

| Wirkstoffe | Testtiere | Wirkstoffkonzentrationen %ige Lösung | Abtötung in % |
|---|---|---|---|
| Bekannt: | | | |
| 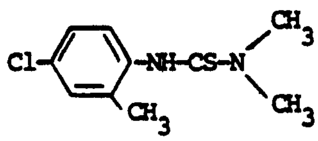 | Sitophilus granarius<br>Blatta orientalis | 0,2<br>0,2 | 0<br>0 |
| Erfindungsgemäß: | | | |
| 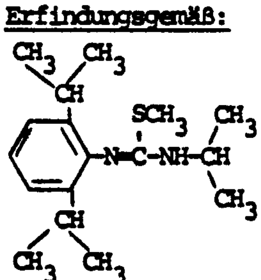 | Sitophilus granarius | 0,2<br>0,02 | 100<br>90 |
| 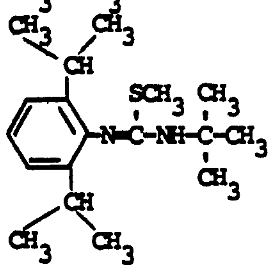 | Sitophilus granarius<br>Blatta orientalis | 0,02<br>0,02 | 100<br>100 |
| 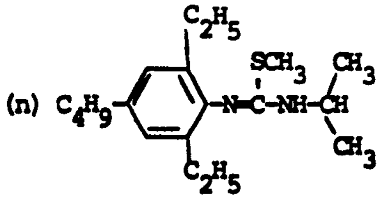 | Sitophilus granarius | 0,2<br>0,02 | 100<br>90 |

Beispiel E

Test mit parasitierenden adulten Rinderzecken (Boophilus microplus res.)

Lösungsmittel: Cremophor

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (B. microplus res.) werden in der zu testenden Wirkstoffzubereitung 1 Min. getaucht. Nach Überführung in Plastikbecher und Aufbewahrung in einem klimatisierten Raum wird der Abtötungsgrad in Prozent bestimmt, wobei 100 % bedeuten, daß alle und 0 %, daß keine Zecken abgetötet worden sind.

| Wirkstoffe | Wirkstoffkonz. ppm | Abtötende Wirkung in % Boophilus microplus res. |
|---|---|---|
| 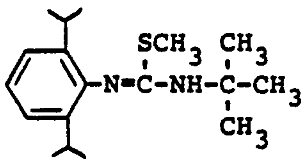 | 300<br>100<br>30 | 100<br>50<br>0 |
| 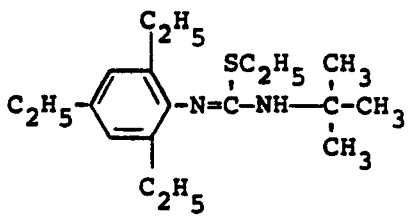 | 1000<br>100 | 100<br>0 |
| 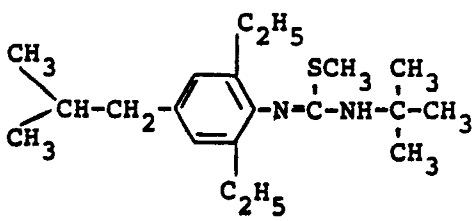 | 1000<br>100 | 100<br>0 |
| 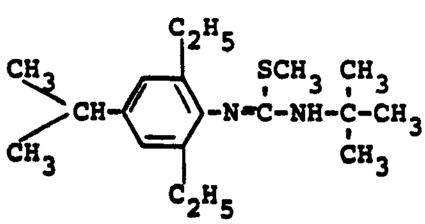 | 1000<br>100 | 100<br>0 |
| 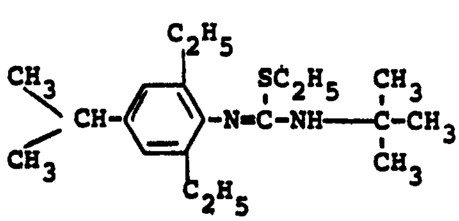 | 1000<br>100 | 100<br>0 |

| Wirkstoffe | Wirkstoffkonz. ppm | Abtötende Wirkung in % Boophilus microplus res. |
|---|---|---|
| 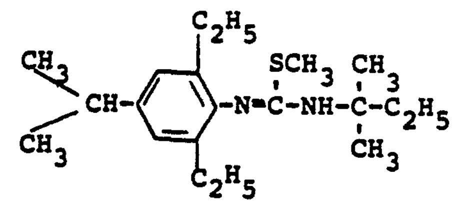 | 1000<br>100 | 100<br>0 |
| 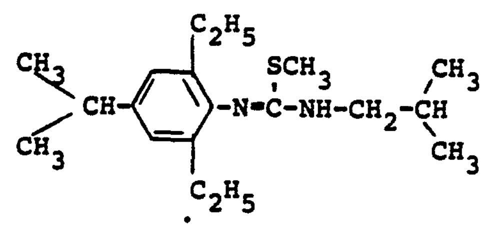 | 1000<br>100 | 100<br>0 |
| 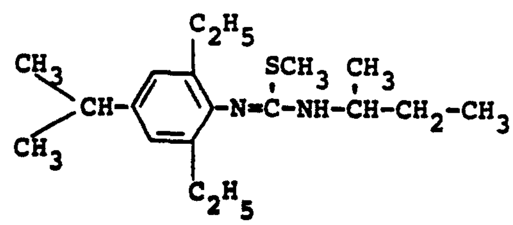 | 1000<br>100 | 100<br>0 |
| 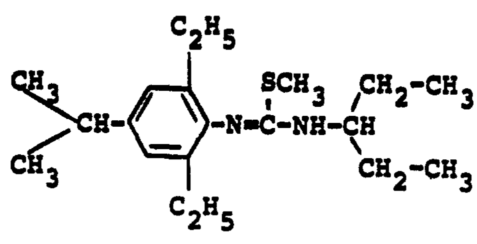 | 1000<br>100 | 100<br>0 |
| 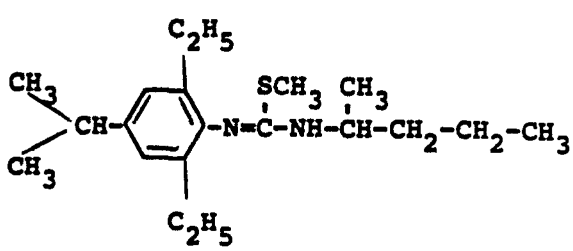 | 1000<br>100 | 100<br>0 |

| Wirkstoffe | Wirkstoffkonz. ppm | Abtötende Wirkung in % Boophilus microplus res. |
|---|---|---|
| 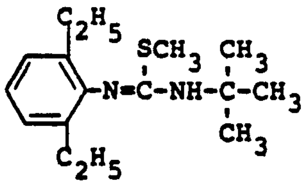 | 1000<br>100 | 100<br>0 |
| 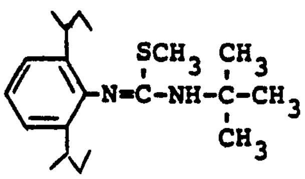 | 1000<br>100 | 100<br>0 |
| 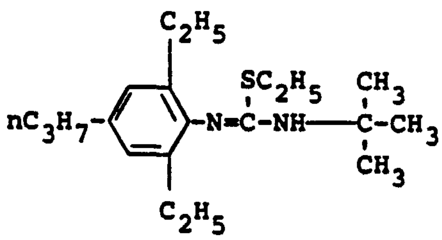 | 1000<br>100. | 100<br>0 |

Beispiel F

Test mit parasitierenden Fliegenlarven

Emulgator: 80 Gewichtsteile Cremophor EL

Zur Herstellung einer zweckmäßigen Wirkstoffzubereitung vermischt man 20 Gewichtsteile der betreffenden aktiven Substanz mit der angegebenen Menge des Emulgators und verdünnt das so erhaltene Gemisch mit Wasser auf die gewünschte Konzentration.

Etwa 20 Fliegenlarven (Lucilia cuprina) werden in ein mit Wattestopfen entsprechender Größe beschicktes Teströhrchen gebracht, welches ca. 3 ml einer 20 %igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad in % bestimmt. Dabei bedeuten 100 %, daß alle und 0 %,daß keine Larven abgetötet worden sind.

| Wirkstoffe | Wirkstoffkonz. ppm | Abtötende Wirkung in % Lucilia cuprina |
|---|---|---|
| 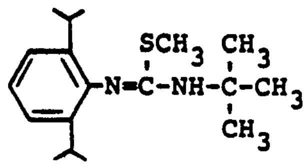 | 1000<br>100 | 100<br>0 |
| 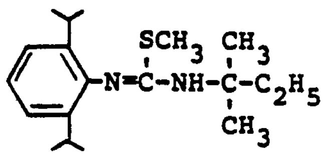 | 1000<br>100 | 100<br>0 |
| 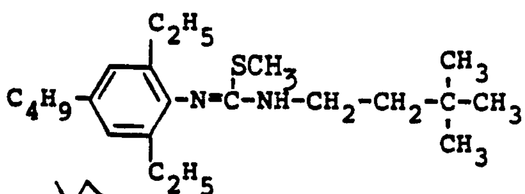 | 1000<br>100 | 100<br>0 |
| 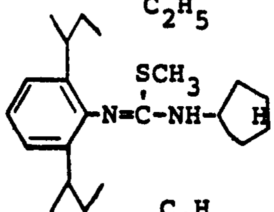 | 1000<br>100 | 100<br>0 |
| 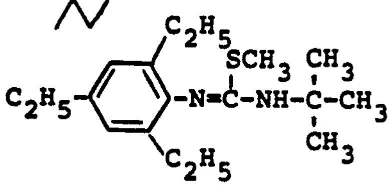 | 1000<br>100 | 100<br>0 |
| 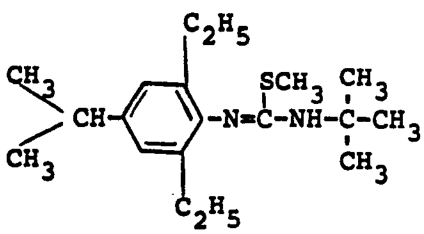 | 1000<br>100 | 100<br>0 |

| Wirkstoff | Wirkstoffkonz. ppm | Abtötende Wirkung in % Lucilia cuprina |
|---|---|---|
| 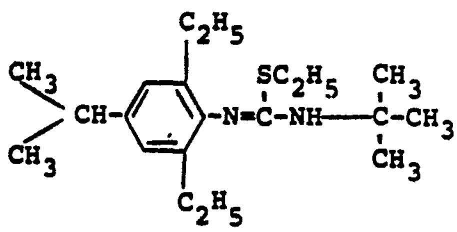 | 1000<br>100 | 100<br>0 |
| 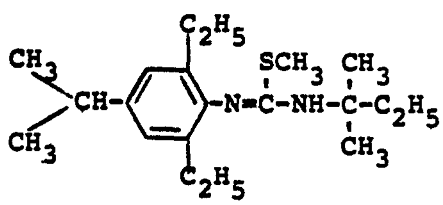 | 1000<br>100 | 100<br>0 |
| 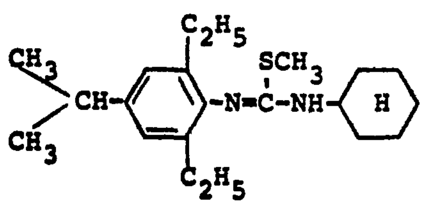 | 1000<br>100 | 100<br>0 |
| 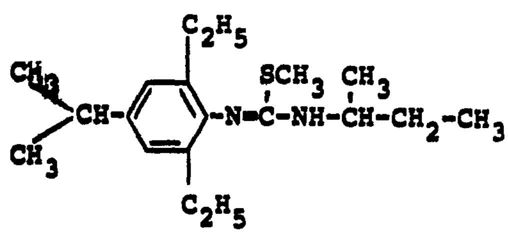 | 1000<br>100 | 100<br>50 |
| 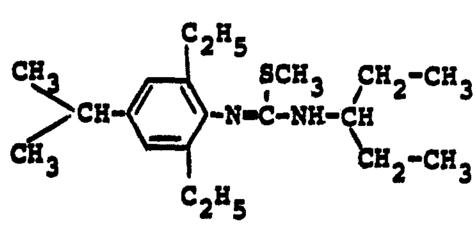 | 100<br>10 | 100<br>0 |

| Wirkstoff | Wirkstoffkonz. in ppm | Abtötende Wirkung in % Lucilia cuprina |
|---|---|---|
| 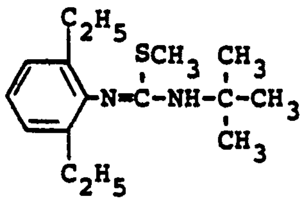 | 1000 | 100 |
| | 100 | 0 |
| 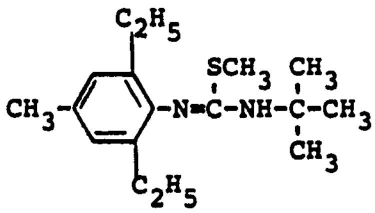 | 1000 | 100 |
| | 100 | 0 |

Beispiel G

Test mit parasitierenden Räudemilben (Psoroptes cuniculi)

Lösungsmittel: Cremophor

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

Etwa 10-25 Räudemilben (Psoroptes cuniculi) werden in 1 ml der zu testenden Wirkstoffzubereitung gebracht, die in Tablettennester einer Tiefziehverpackung pipettiert wurden. Nach 24 Stunden wird der Abtötungsgrad in Prozent bestimmt. Dabei bedeuten 100 %, daß alle und 0 %, daß keine Milben abgetötet worden sind.

| Wirkstoffe | Wirkstoffkonz. in ppm | Abtötende Wirkung in % Psorptes cuniculi |
|---|---|---|
| 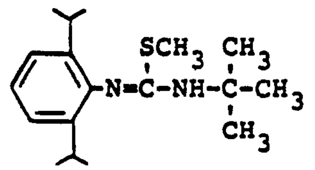 | 1<br>0,3 | 100<br>0 |
| 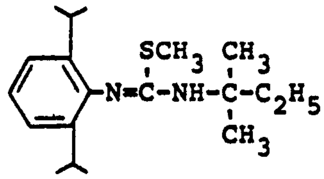 | 1<br>0,3 | 100<br>0 |
| 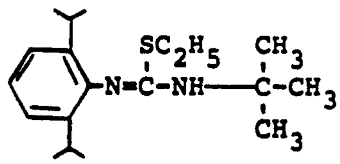 | 1 | 100 |
| 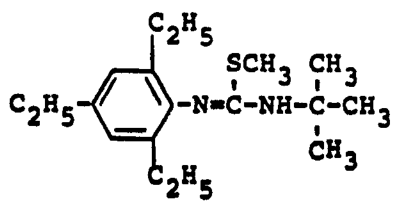 | 1<br>0,3 | 100<br>0 |
| 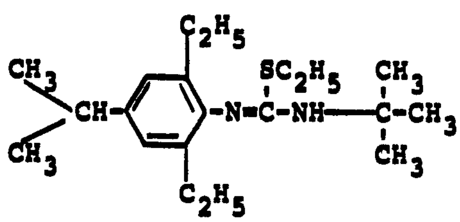 | 1 | 100 |
| 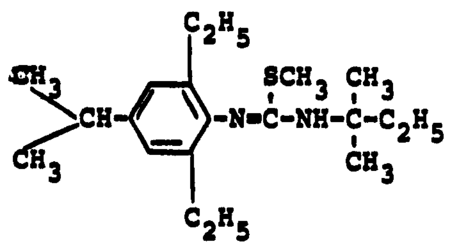 | 1 | 100 |

| Wirkstoffe | Wirkstoffkonz. in ppm | Abtötende Wirkung in % Psoroptes cuniculi |
|---|---|---|
| 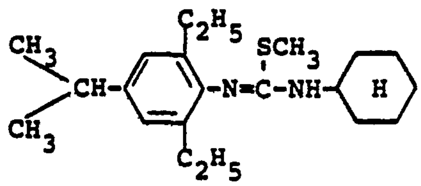 | 1 | 100 |
| 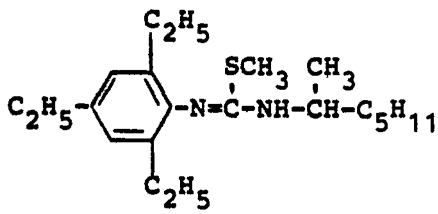 | 1 | 100 |
| 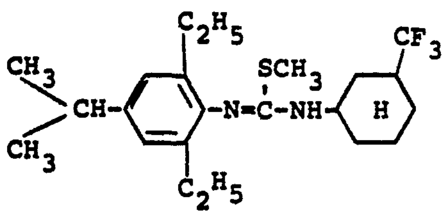 | 1 | 100 |
| 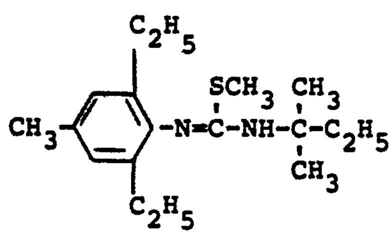 | 1 | 100 |

## Herstellungsbeispiele

### Beispiel 1

**N-(2,6-Diisopropyl-phenyl)-N'-tert.-butyl-S-methyl-isothioharnstoff:**

15,0 g N-(2,6-Diisopropyl-phenyl)-N'-tert.-butyl-thioharnstoff werden in 30 ml Dimethylformamid gelöst und bei 50°C 10 g Methyljodid zugegeben. Die Reaktion verläuft exotherm unter Temperaturanstieg auf ca. 70°C. Danach läßt man noch 16 Stunden stehen und verrührt mit 500 ml Wasser und 100 ml 15 %iger Sodalösung. Der ölig abgeschiedene Isothioharnstoff wird mit Methylenchlorid aufgenommen, der Extrakt mit Wasser gewaschen, über Kaliumcarbonat getrocknet, eingeengt und bei 60°C/1,0 Torr völlig vom Lösungsmittel befreit. Ausbeute 14 g Öl. Die Elementaranalyse, NMR- und IR-Spektrum stehen mit der angenommenen Konstitution in Übereinstimmung.

Verwendet man statt Methyljodid die äquivalente Menge Dimethylsulfat oder statt Dimethylformamid Aceton als Lösungsmittel, so erhält man die gleiche Verbindung.

Aus den entsprechenden Thioharnstoffen erhält man wie in vorstehendem Beispiel angegeben die folgenden Isothioharnstoffe:

N-(2,6-Diisopropyl-phenyl)-N'-tert.-pentyl-S-methyl-isothioharnstoff

N-(3-Isobutyl-2,6-diäthyl-phenyl)-N'-tert.-butyl-S-methyl-isothioharnstoff

N-(4-Isopropyl-2,6-diäthyl-phenyl)-N'-tert.-butyl-S-methyl-isothioharnstoff

N-(4-Isopropyl-2,6-diäthyl-phenyl)-N'-tert.-pentyl-S-methyl-isothioharnstoff

N-(4-Propyl-2,6-diäthyl-phenyl)-N'-tert.-butyl-S-methyl-isothioharnstoff

N-(2,6-Diisopropyl-phenyl)-N'-isopropyl-S-methyl-isothioharnstoff

N-(2,6-Diisopropyl-phenyl)-N'-propyl-S-methyl-isothioharnstoff

N-(2,4,6-Triäthyl-phenyl)-N'-isopropyl-S-methyl-isothioharnstoff

N-(4-Isopropyl-2,6-diäthyl-phenyl)-N'-isopropyl-S-methyl-isothioharnstoff

N-(4-Propyl-2,6-diäthyl-phenyl)-N'-isopropyl-S-methyl-isothioharnstoff

N-(4-Butyl-2,6-diäthyl-phenyl)-N'-isopropyl-S-methyl-isothioharnstoff

N-(4-Pentyl-(3)-2,6-diäthyl-phenyl)-N'-isopropyl-S-methyl-isothioharnstoff

Die aufgeführten Verbindungen stellen alle nichtdestillierbare bei 20°C viskose Flüssigkeiten dar, deren Konstitution durch Elementaranalyse, NMR- und IR-Spektrem gesichert wurde.

Beispiel 2

N-(2,6-Diäthyl-4-n-butyl-phenyl)-N'-(3,3-dimethyl-butyl)-S-methyl-isothioharnstoff

21,0 g N-(2,6-Diäthyl-4-n-butyl-phenyl)-N'-(3,3-dimethyl-butyl)-thioharnstoff werden bei 50°C in 40 ml Dimethylformamid gelöst und 10,0 g Methyljodid zugegeben. Nach dem Abklingen der exothermen Reaktion wird noch 24 Stunden bei 20°C aufbewahrt. Danach wird der Ansatz mit 500 ml Wasser und 100 ml 15 %iger Sodalösung verrührt, das ölige Reaktionsprodukt mit Methylenchlorid extrahiert, der Extrakt mit Wasser gewaschen, über Kaliumcarbonat getrocknet eingeengt und bei 60°C/1,0 Torr von Resten Lösungsmittel befreit. Ausbeute 18,0 g Öl.

Die Elementaranalyse, NMR- und IR-Spektrum stehen mit der angenommenen Konstitution in Übereinstimmung.

Aus den entsprechenden Thioharnstoffen können analog die folgenden Isothioharnstoffe hergestellt werden:

N-(4-Isopropyl-2,6-diäthyl-phenyl)-N'-isobutyl-S-methyl-isothioharnstoff
N-(4-Isopropyl-2,6-diäthyl-phenyl)-N'-sec.-butyl-S-methyl-isothioharnstoff
N-(4-Isopropyl-2,6-diäthyl-phenyl)-N'-pentyl-(3)-S-methyl-isothioharnstoff
N-(4-Isopropyl-2,6-diäthyl-phenyl)-N'-pentyl-(2)-S-methyl-isothioharnstoff

N-(4-Isopropyl-2,6-diäthyl-phenyl)-N'-heptyl-(2)-S-methyl-isothioharnstoff

N-(4-Propyl-2,6-diäthyl-phenyl)-N'-heptyl(2)-S-methyl-isothioharnstoff

N-(2,4,6-Triäthyl-phenyl)-N-heptyl-(2)-S-methyl-isothioharnstoff

N-(4-Butyl-2,6-diäthyl-phenyl)-N'-heptyl-(2)-S-methyl-isothioharnstoff

N-(4-Cyclohexyl-2,6-diäthyl-phenyl)-N'-sec.-butyl-S-methyl-isothioharnstoff

N-(4-Pentyl-(3)-2,6-diäthyl-phenyl)-N'-butyl-S-methyl-isothioharnstoff

N-(4-Pentyl-(3)-2,6-diäthyl-phenyl)-N'-isobutyl-S-methyl-isothioharnstoff

N-(4-Pentyl-(3)-2,6-diäthyl-phenyl)-N'-sec.-butyl-S-methyl-isothioharnstoff

N-(4-Pentyl-(3)-2,6-diäthyl-phenyl)-N'-pentyl-(2)-S-methyl-isothioharnstoff

N-(4-Pentyl-(3)-2,6-diäthyl-phenyl)-N'-tert.-pentyl-S-methyl-isothioharnstoff

Sämtliche vorstehenden Verbindungen stellen bei 20°C nichtdestillierbare, viskose Flüssigkeiten dar, deren Konstitution durch Elementaranalyse, NMR- und IR-Spektren gesichert wurde.

Beispiel 3

N-(2,6-Di-sec.-butyl-phenyl)-N'-cyclopentyl-S-methyl-isothioharnstoff

19,0 g N-(2,6-Di-sec.-butyl-phenyl)-N'-cyclopentyl-thioharnstoff werden bei 50°C in 40 ml Dimethylformamid gelöst und 10,0 g Methyljodid zugegeben. Nach dem Abklingen der exothermen Reaktion läßt man noch 24 Stunden bei 20°C stehen. Danach wird der Ansatz mit 500 ml Wasser und 100 ml 15 %iger Sodalösung verrührt und das abgeschiedene ölige Reaktionsprodukt mit Methylenchlorid aufgenommen. Der Extrakt wird mit Wasser gewaschen, über Kaliumcarbonat getrocknet, eingeengt und bei 60°C/1,0 Torr vom Lösungsmittel befreit. Ausbeute 16,0 g Öl. Die Elementaranalyse sowie NMR- und IR-Spektrum stehen mit der angenommenen Konstitution in Übereinstimmung.

Ersetzt man das Methyljodid durch die äquivalente Menge Methylbromid und arbeitet wie angegeben, so erhält man die gleiche Verbindung.

Analog können aus den entsprechenden Thioharnstoffen die folgenden Verbindungen hergestellt werden:

N-(2,6-Di-sec.-butyl-phenyl)-N'-tert.-butyl-S-methyl-isothioharnstoff
N-(4-Pentyl-(3)-2,6-diäthyl-phenyl)-N'-cyclopentyl-S-methyl-isothioharnstoff

Die aufgeführten Isothioharnstoffe stellen viskose Flüssigkeiten dar, deren Konstitution durch Elementaranalyse, NMR- und IR-Spektren gesichert wurde.

**Beispiel 4**

**N-(2,6-Diisopropyl-phenyl)-N'-tert.-butyl-S-äthyl-isothioharnstoff**

15.0 g N-(2,6-Diisopropyl-phenyl)-N'-tert.-butyl-thioharnstoff werden in 30 ml Dimethylformamid bei 50°C gelöst und 7,7 g Äthylbromid langsam zugesetzt. Danach wird der Ansatz noch 6 Stunden bei 50°C gehalten. Anschließend verrührt man mit 500 ml Wasser und 100 ml 15 %iger Sodalösung und nimmt das ölig abgeschiedene Reaktionsprodukt mit Methylenchlorid auf. Die erhaltene Lösung wird mit Wasser gewaschen, über Kaliumcarbonat getrocknet, eingeengt und bei 60°C/1,0 Torr von Resten Lösungsmittel befreit. Ausbeute 12,0 g Öl.

Verwendet man in vorstehendem Beispiel statt Äthylbromid die äquivalente Menge Propylbromid, so erhält man N-(2,6-Diisopropyl-phenyl)-N'-tert.-butyl-S-propyl-isothioharnstoff. Analog erhält man mit Allylbromid den N-(2,6-Diisopropyl-phenyl)-N'-tert.-butyl-S-allyl-isothioharnstoff; mit Crotylbromid die N-(2,6-Diisopropyl-phenyl)-N'-tert.-butyl-S-crotyl-isothioharnstoff; mit Methallylbromid den N-(2,6-Diisopropyl-phenyl)-N'-tert.-butyl-S-methallyl-isothioharnstoff. Die S-Alkyl-isothioharnstoffe bilden in allen Fällen viskose Öle. Die Konstitutionen wurden durch Elementaranalse, NMR- und IR-Spektren bestätigt.

Analog können aus den entsprechenden Thioharnstoffen die folgenden Isothioharnstoffe hergestellt werden:

N-(2,4,6-Triäthyl-phenyl)-N'-tert.-butyl-S-äthyl-isothioharnstoff

N-(4-Isopropyl-2,6-diäthyl-phenyl)-N'-tert.-butyl-S-äthyl-isothioharnstoff

N-(4-Isopropyl-2,6-diäthyl-phenyl)-N'-tert.-pentyl-S-äthyl-isothioharnstoff

N-(4-Propyl-2,6-diäthyl-phenyl)-N'-tert.-butyl-S-äthyl-isothioharnstoff

N-(4-Methyl-2,6-diäthyl-phenyl)-N'-tert.-butyl-S-allyl-isothioharnstoff

N-(4-Methyl-2,6-diäthyl-phenyl)-N'-tert.-pentyl-S-allyl-isothioharnstoff

N-(2,6-Di-sec.-butyl-phenyl)-N'-tert.-butyl-S-methyl-isothioharnstoff

N-(2,6-Diisopropyl-phenyl)-N'-propyl-S-äthyl-isothioharnstoff

## Beispiel 5

**N-(2,6-Diäthyl-phenyl)-N'-tert.-butyl-S-methyl-isothioharnstoff**

26,0 g N-(2,6-Diäthyl-phenyl)-N'-tert.-butyl-thioharnstoff werden bei 50°C in 25 ml Dimethylformamid gelöst und in Anteilen 17,0 g Methyljodid zugesetzt. Wenn die exotherme Reaktion beendet ist, läßt man noch 16 Stunden bei 20°C stehen. Danach wird der Ansatz mit 500 ml Wasser und 100 ml 15 %iger Sodalösung verrührt, das ölige Reaktionsprodukt mit Methylenchlorid aufgenommen und der Extrakt mit Wasser gewaschen. Man trocknet über Kaliumcarbonat, engt ein und entfernt das Lösungsmittel bei 60°C/1,0 Torr.

Ausbeute 26 g Öl. Elementaranalyse, NMR- und IR-Sprektrum stehen mit der angenommenen Konstitution in Einklang.

Analog erhält man aus den entsprechenden Thioharnstoffen die folgenden Isothiohharnstoffe:

N-(4-Methyl-2,6-diäthyl-phenyl)-N'-tert.-butyl-S-methyl-isothioharnstoff
N-(4-Methyl-2,6-diäthyl-phenyl)-N'-tert.-pentyl-S-methyl-isothioharnstoff

Die Verbindungen sind viskose Flüssigkeiten, deren Konstitution durch Elementaranalyse und NMR- sowie IR-Spektrum gesichert wurde.

Beispiel 6

N-(2,6-Diäthyl-4-pentyl-(3)-phenyl)-N'-cyclohexyl-S-methyl-isothioharnstoff

18,0 g N-(2,6-Diäthyl-4-pentyl-(3)-phenyl)-N'-cyclohexyl-thioharnstoff werden bei 50°C in 20 ml Dimethylformamid gelöst und 8,5 g Methyljodid zugegeben. Nach Abklingen der exothermen Reaktion wird noch 20 Stunden bei 20°C aufbewahrt. Danach wird der Ansatz mit 500 ml Wasser und 100 ml 15 %iger Sodalösung verrührt, das ölige Reaktionsprodukt mit Methylenchlorid aufgenommen und der Extrakt mit Wasser gewaschen. Man trocknet über Kaliumcarbonat, engt ein und entfernt das Lösungsmittel vollständig bei 60°C/1,0 Torr. Ausbeute 16 g Öl.

Die Elementaranalyse sowie NMR- und IR-Spektrum entsprechen der angenommenen Konstitution.

In analoger Weise können aus den entsprechenden Thioharnstoffen die folgenden Isothioharnstoffe hergestellt werden:

N-(4-Isopropyl-2,6-diäthyl-phenyl)-N'-cyclohexyl-S-methyl-isothioharnstoff
N-(4-Isopropyl-2,6-diäthyl-phenyl)-N'-(3-trifluormethyl-cyclohexyl)-S-methyl-isothioharnstoff
N-(4-Butyl-2,6-diäthyl-phenyl)-N'-cyclohexyl-S-methyl-isothioharnstoff
N-(4-Butyl-2,6-diäthyl-phenyl)-N'-cycloheptyl-S-methyl-isothioharnstoff

Die Verbindungen sind viskose Flüssigkeiten, deren Konstitution durch Elementaranalyse, NMR- und IR-Spektrum gesichert wurde.

<u>Beispiel 7</u>

<u>N-(2,4,6-Triäthyl-phenyl)-N'-norbornyl-S-methyl-isothioharnstoff</u>

21,0 g N-(2,4,6-Triäthyl-phenyl)-N'-norbornyl-thioharnstoff werden bei 60°C in 25 ml Dimethylformamid gelöst und 11 g Methyljodid zugegeben. Nach dem Abklingen der exothermen Reaktion wird noch 16 Stunden bei 20°C aufbewahrt. Danach

gießt man den Ansatz in 500 ml Wasser und 100 ml 15 %ige Sodalösung und nimmt das ölige Reaktionsprodukt in Methylenchlorid auf. Die Extraktphase wird mit Wasser gewaschen, über Kaliumcarbonat getrocknet, eingeengt und bei 60°C/1,0 Torr von Resten Lösungsmittel befreit. Ausbeute 20,0 g Öl.

Elementaranalyse, NMR- und IR-Spektrum stehen mit der angenommenen Konstitution in Übereinstimmung.

In analoger Weise können aus den enstprechenden Thioharnstoffen die folgenden Isothioharnstoffe hergestellt werden:

N-(4-Isopropyl-2,6-diäthyl-phenyl)-N'-norbornyl-S-methyl-isothioharnstoff
N-(4-Propyl-2,6-diäthyl-phenyl)-N'-norbornyl-S-methyl-isothioharnstoff
N-(4-Butyl-2,6-diäthyl-phenyl)-N'-norbornyl-S-methyl-isothioharnstoff
N-(4-Isobutyl-2,6-diäthyl-phenyl)-N'-norbornyl-S-methyl-isothioharnstoff
N-(4-tert.-Butyl-2,6-diäthyl-phenyl)-N'-norbornyl-S-methyl-isothioharnstoff
N-(4-Pentyl-(3)-2,6-diäthyl-phenyl)-N'-norbornyl-S-methyl-isothioharnstoff

Da das für die Synthese der Thioharnstoffe verwendete Norbornylamin ein Gemisch aus endo- und exo-Form darstellte, sind die als Endprodukte erhaltenen Isothioharnstoffe ebenfalls Gemische. Die Verbindungen stellen viskose Öle dar, deren Konstitution durch Elementaranalyse, NMR- und IR-Spektrum gesichert wurde.

Herstellung von Ausgangsverbindungen:

Beispiel 8

N-(2,6-Diäthyl-phenyl)-N'-isobutyl-thioharnstoff

15,0 g 2,6-Diäthyl-phenylisothiocyanat werden bei 20°C in 20 g Isobutylamin eingetragen und 12 Stunden stehen gelassen. Danach verrührt man mit überschüssiger verdünnter Salzsäure bis das zunächst ölig ausfallende Reaktionsprodukt kristallisiert. Man filtriert und wäscht mit Wasser neutral. Danach wird der Nutschkuchen mit Methanol gleichmäßig verrieben, Wasser zugesetzt, abgesaugt und getrocknet. Ausbeute 19,0 g; F: 68-70°C.

Elementaranalyse und NMR-Spektrum stehen mit der angenommenen Konstitution in Übereinstimmung.

Analog können aus dem entsprechenden 2,6-Diäthyl-phenylisothiocyanat und den entsprechenden aliphatischen Aminen die folgenden
N-Aryl-N'-alkyl-thioharnstoffe hergestellt werden:

N-(2,6-Diäthyl-phenyl)-N'-äthyl-thioharnstoff F: 72-73°C
N-(2,6-Diäthyl-phenyl)-N'-propyl-thioharnstoff F: 58-60°C
N-(2,6-Diäthyl-phenyl)-N'-butyl-thioharnstoff F: 41-43°C
N-(2,6-Diäthyl-phenyl)-N'-pentyl-thioharnstoff
N-(2,6-Diäthyl-phenyl)-N'-neopentyl-thioharnstoff F: 87-89°C
N-(2,6-Diäthyl-phenyl)-N'-hexyl-thioharnstoff
N-(2,6-Diäthyl-phenyl)-N'-heptyl-thioharnstoff
N-(2,6-Diäthyl-phenyl)-N'-dodecyl-thioharnstoff F: 120-123°C
N-(2,6-Diäthyl-phenyl)-N'-methallyl-thioharnstoff F: 65-68°C

Das Ausgangsprodukt 2,6-Diäthyl-phenylisothiocyanat kann folgendermaßen hergestellt werden: 100 g 2,6-Diäthylanilin in 200 ml Methylenchlorid werden bei 0 - 5°C unter Rühren zu einem Gemisch von 500 ml Methylenchlorid, 300 ml Wasser, 120 g Calciumcarbonat und 92 g Thiophosgen zugetropft. Danach erhitzt man zum Rückfluß, bis die $CO_2$-Entwicklung beendet ist. Der abgekühlte Ansatz wird filtriert, die Methylenchloridschicht abgetrennt, mit Calciumchlorid getrocknet und fraktioniert..
Ausbeute 112 g; Kp. 101 - 103°C/ 1,4 Torr.

Beispiel 2

N-(4-Methyl-2,6-diäthyl-phenyl)-N'-neopentyl-thioharnstoff

15,0 g 4-Methyl-2,6-diäthyl-phenylisothiocyanat werden bei 20°C in 9,0 g 2,2-Dimethyl-propylamin eingetragen, wobei exotherme Reaktion eintritt. Nach 12 Stunden verrührt man mit überschüssiger verdünnter Salzsäure, filtriert, wäscht mit Wasser und verdünntem Methanol und trocknet.
Ausbeute 21 g; F: 106-107°C.
Elementaranalyse und NMR-Spektrum stehen mit der angenommenen Konstitution in Übereinstimmung.

Analog können aus 4-Methyl-2,6-diäthyl-phenylisothiocyanat und den entsprechenden aliphatischen Aminen die folgenden Thioharnstoffe hergestellt werden:

N-(4-Methyl-2,6-diäthyl-phenyl)-N'-butyl-thioharnstoff
N-(4-Methyl-2,6-diäthyl-phenyl)-N'-isobutyl-thioharnstoff
F: 60-61°C
N-(4-Methyl-2,6-diäthyl-phenyl)-N'-methallyl-thioharnstoff
f: 86-88°C

<u>4-Methyl-2,6-diäthyl-phenylisothiocyanat</u> kann nach folgender Vorschrift hergestellt werden:
100 g 4-Methyl-2,6-diäthyl-anilin in 200 ml Methylenchlorid werden bei 0 - 5°C zu einem Gemisch von 500 ml Methylenchlorid, 300 ml Wasser, 120 g Calciumcarbonat und 92 g Thiophosgen unter Rühren zugetropft. Danach wird unter Rückfluß erwärmt, bis die $CO_2$-Entwicklung beendet ist. Nach dem Abkühlen wird der Ansatz von Feststoffen abfiltriert, die Methylenchloridschicht abgetrennt, über Calciumchlorid getrocknet und fraktioniert.
Ausbeute 110 g; Kp: 113-116°C/1,2 Torr.

<u>Beispiel 10</u>

<u>N-(2,6-Diisopropyl-phenyl)-N'-isobutyl-thioharnstoff</u>

15,0 g 2,6-Diisopropyl-phenylisothiocyanat werden bei 20°C in 20,0 g Isobutylamin eingetragen. Die Reaktion verläuft exotherm; man läßt 12 Stunden stehen und verrührt dann mit überschüssiger verdünnter Salzsäure. Das kristallin ausgefallene Reaktionsgemisch wird abgesaugt, mit Wasser und verdünntem Methanol gewaschen und getrocknet.
Ausbeute 19,0 g; F: 108-112°C.
Elementaranalyse und NMR-Spektrum entsprechen der angenommenen Konstitution.

Analog können aus 2,6-Diisopropyl-phenylisothiocyanat und den entsprechenden aliphatischen Aminen die folgenden Thioharnstoffe hergestellt werden:

N-(2,6-Diisopropyl-phenyl)-N'-propyl-thioharnstoff F: 106-107°C
N-(2,6-Diisopropyl-phenyl)-N'-butyl-thioharnstoff
N-(2,6-Diisopropyl-phenyl)-N'-(3-methyl-butyl)-thioharnstoff F: 64-68°C
N-(2,6-Diisopropyl-phenyl)-N'-pentyl-thioharnstoff F: 76-83°C

N-(2,6-Diisopropyl-phenyl)-N'-neopentyl-thioharnstoff
F: 150-155°C
N-(2,6-Diisopropyl-phenyl)-N'-hexyl-thioharnstoff
N-(2,6-Diisopropyl-phenyl)-N'-dodecyl-thioharnstoff
N-(2,6-Diisopropyl-phenyl)-N'-methallyl-thioharnstoff
F: 93-95°C

Das Ausgangsprodukt 2,6-Diisopropyl-phenylisothiocyanat kann folgendermaßen hergestellt werden:
100 g 2,6-Diisopropyl-anilin in 200 ml Methylenchlorid werden bei 0 - 5°C unter Rühren zu einem Gemisch aus 300 ml Wasser, 500 ml Methylenchlorid, 120 g Calciumcarbonat und 78 g Thiophosgen zugetropft. Danach erwärmt man unter Rückfluß, bis die $CO_2$-Entwicklung beendet ist. Nach dem Abkühlen wird filtriert, die Methylenchloridschicht abgetrennt, über Calciumchlorid getrocknet und fraktioniert. Ausbeute 110 g; Kp: 144-148°C/11 Torr.

Beispiel 11

N-(2,6-Di-sek-butyl-phenyl)-N'-methallyl-thioharnstoff

15,0 g 2,6-Di-sek-butyl-phenylisothiocyanat werden bei -10°C in 12,0 g Methallylamin eingetragen. Man läßt langsam auf +20°C kommen und bewahrt 12 Stunden bei 20°C auf. Danach wird der Ansatz mit überschüssiger verdünnter Salzsäure verrührt, das kristalline Reaktionsprodukt abgesaugt, mit Wasser und verdünntem Methanol gewaschen und getrocknet. Ausbeute 20,0 g; F: 69-72°C.

Analog können aus 2,6-Di-sek-butyl-phenylisothiocyanat und den entsprechenden aliphatischen Aminen die folgenden Thioharnstoffe hergestellt werden:

N-(2,6-Di-sek.-butyl-phenyl)-N'-propyl-thioharnstoff
F: 85-87°C

N-(2,6-Di-sek.-butyl-phenyl)-N'-allyl-thioharnstoff
F: 83-86°C

N-(2,6-Di-sek.-butyl-phenyl)-N'-butyl-thioharnstoff
F: Wachs

N-(2,6-Di-sek.-butyl-phenyl)-N'-isobutyl-thioharnstoff
F: 83-85°C

N-(2,6-Di-sek.-butyl-phenyl)-N'-pentyl-thioharnstoff (ölig)

N-(2,6-Di-sek.-butyl-phenyl)-N'-neopentyl-thioharnstoff
F: 88-90°C

N-(2,6-Di-sek.-butyl-phenyl)-N'-dodecyl-thioharnstoff (ölig)

Das Ausgangsprodukt 2,6-Di-sec.-butyl-phenylisothiocyanat kann folgendermaßen hergestellt werden:

100 g 2,6-Di-sek.-butyl-anilin in 200 ml Methylenchlorid werden bei 0 - 5°C unter Rühren zu einem Gemisch aus 300 ml Wasser, 500 ml Methylenchlorid, 100 g Calciumcarbonat und 68 g Thiophosgen zugetropft. Danach erwärmt man unter Rückfluß bis die $CO_2$-Entwicklung beendet ist. Nach dem Abkühlen wird von den Feststoffen abfiltriert, die Methylenchloridschicht abgetrennt, über Calciumchlorid getrocknet und fraktioniert: Ausbeute 112 g, Kp: 117-120°C/1,0 Torr.

Beispiel 12

N-(4-Methyl-2,6-diisopropyl-phenyl)-N'-isobutyl-thioharnstoff

15,0 g 4-Methyl-2,6-diisopropyl-phenylisothiocyanat werden bei 20°C in 20 g Isobutylamin eingetragen und die exotherme Reaktion durch Kühlung gemäßigt. Man läßt 12 Stunden stehen

und verrührt dann mit überschüssiger verdünnter Salzsäure. Das auskristallisierte Reaktionsprodukt wird abfiltriert, mit Wasser und verdünntem Methanol gewaschen und getrocknet. Ausbeute 18,0 g; F: 150-153°C.

Analog können aus 4-Methyl-2,6-diisopropyl-phenylisothiocyanat und den entsprechenden aliphatischen Aminen die folgenden Thioharnstoffe hergestellt werden:

N-(4-Methyl-2,6-diisopropyl-phenyl)-N'-butyl-thioharnstoff F: 126-129°C

N-(4-Methyl-2,6-diisopropyl-phenyl)-N'-propyl-thioharnstoff F: 125-127°C

Aus 4-Methyl-2,6-di-sek.-butyl-phenylisothiocyanat und Isobutylamin erhält man analog
N-(4-Methyl-2,6-di-sek.-butyl-phenyl-N'-isobutyl-thioharnstoff F: 98-102°C
mit n-Butylamin
N-(4-Methyl-2,6-di-sek.-butyl-phenyl)-N'-butyl-thioharnstoff F: 97-100°C

<u>4-Methyl-2,6-diisopropyl-phenylisothiocyanat</u> kann folgendermaßen hergestellt werden:

150 g 4-Methyl-2,6-diisopropyl-anilin in 200 ml Methylenchlorid werden bei 20°C zu einem gerührten Gemisch aus 500 ml Methylenchlorid, 300 ml Wasser, 120 g Calciumcarbonat und 110 g Thiophosgen zugetropft. Danach erwärmt man unter Rückfluß bis die $CO_2$-Entwicklung beendet ist. Der Ansatz wird von Feststoffen abfiltriert, die Methylenchloridschicht abgetrennt, über Calciumchlorid getrocknet und fraktioniert. Ausbeute 159 g erstarrendes Öl; Kp: 124-126°C/1,2 Torr.

Analog wird aus 4-Methyl-2,6-di-sek.-butyl-anilin und Thiophosgen das 4-Methyl-2,6-di-sek.-butyl-phenylisothiocyanat vom Kp: 130-132°C/1,2 Torr erhalten.

Beispiel 13

N-(3-Methyl-2,6-diäthyl-phenyl)-N'-neopentyl-thioharnstoff

15,0 g 3-Methyl-2,6-diäthyl-phenylisothiocyanat werden bei 20°C in 9,0 g 2,2-Dimethyl-propylamin eingetragen. Man läßt 12 Stunden stehen und verrührt dann mit überschüssiger verdünnter Salzsäure. Das auskristallisierte Reaktionsprodukt wird abgesaugt, mit Wasser und verdünntem Methanol gewaschen und getrocknet. Ausbeute 20,0 g; F: 102-104°C. Elementaranalyse und NMR-Spektrum stehen mit der angenommenen Konstitution in Übereinstimmung.

Analog kann aus 3-Methyl-2,6-diäthyl-phenylisothiocyanat und Methallylamin der
N-(3-Methyl-2,6-diäthyl-phenyl)-N'-methallyl-thioharnstoff
F: 105-106°C;
erhalten werden; aus 3,5-Dimethyl-2,6-diäthyl-phenylisothiocyanat und n-Butylamin der
N-(3,5-Dimethyl-2,6-diäthyl-phenyl)-N'-butyl-thioharnstoff
F: 108-110°C;
aus 3-Chlor-2,6-diäthyl-phenylisothiocyanat und n-Butylamin der N-(3-Chlor-2,6-diäthyl-phenyl)-N'-butyl-thioharnstoff
F: 108-110°C;
mit 2,2-Dimethyl-propylamin der
N-(3-Chlor-2,6-diäthyl-phenyl)-N'-neopentyl-thioharnstoff
F: 106-110°C;
aus 2-Äthyl-6-isopropyl-phenylisothiocyanat und Methallylamin der N-(2-Äthyl-6-isopropyl-phenyl)-N'-methallyl-thioharnstoff
F: 50-55°C

aus 2,4,6-Triisopropyl-phenylisothiocyanat und Methallylamin der N-(2,4,6-Triisopropyl-phenyl)-N'-methallyl-thioharnstoff F: 115-118°C.

Die Arylisothiocyanate können aus den Arylaminen und Thiophosgen wie in den vorhergehenden Beispielen angegeben hergestellt werden:

3-Methyl-2,6-diäthyl-phenylisothiocyanat; Kp: 110-113°/ 1,5 Torr

3,5-Dimethyl-2,6-diäthyl-phenylisothiocyanat: Kp: 119-127°C/ 1,2 Torr

3-Chlor-2,6-diäthyl-phenylisothiocyanat; Kp: 118-121°C/ 1,2 Torr

2-Äthyl-6-isopropyl-phenylisothiocyanat; Kp: 104-106°C/ 1,2-Torr

2,4,6-Triisopropyl-phenylisothiocyanat; Kp: 130-132°C/ 1,2 Torr.

## Beispiel 14

### N-(2,6-Diisopropyl-phenyl)-N'-methyl-thioharnstoff

120 g 2,6-Diisopropyl-anilin werden in 100 ml Triäthylamin gelöst und 53 g Methylisothiocyanat zugegeben. Die Thioharnstoffbildung verläuft schwach exotherm. Man rührt 12 Stunden, versetzt mit überschüssiger verdünnter Salzsäure, filtriert, wäscht mit Wasser und verdünntem Methanol und trocknet.
Ausbeute 142 g; F: 167-168°C.

Analog erhält man aus Methylisothiocyanat und den entsprechenden Anilin-derivaten die folgenden Thioharnstoffe:

N-(2,6-Diäthyl-phenyl)-N'-methyl-thioharnstoff F: 103 - 105°C

N-(4-Methyl-2,6-diäthyl-phenyl)-N'-methyl-thioharnstoff F: 97 - 100°C

N-(2-Methyl-6-äthyl-phenyl)-N'-methyl-thioharnstoff F: 65 - 67°C

N-(2,4-Dimethyl-6-äthyl-phenyl)-N'-methyl-thioharnstoff F: 125 - 126°C

Beispiel 15

N-(2,6-Di-sek.-butyl-phenyl)-N'-tert.-butyl-thioharnstoff

20,0 g 2,6-Di-sek.-butyl-phenylisothiocyanat werden in 20,0 g tert.-Butylamin gelöst und der Ansatz 12 Stdn. bei 20°C aufbewahrt. Dann gießt man in verdünnte Salzsäure, filtriert das Reaktionsprodukt ab, wäscht neutral und trocknet.

Ausbeute 25,0 g; 96 % d. Th. F: 115 - 117°C

Analog erhält man aus 2,6-Di-sek.-butyl-phenylisothiocyanat und tert.-Pentylamin den N-(2,6-Di-sek.-butyl-phenyl)-N'-tert.-pentyl-thioharnstoff vom F: 116 - 118°C.

Aus 2-Äthyl-6-sek.-butylphenylisothiocyanat und tert.-Butylamin die Verbindung
N-(2-Äthyl-6-sek.-butyl-phenyl)-N'-tert.-butyl-thioharnstoff vom Schmelzpunkt 96 - 99°C;

aus 2-Äthyl-6-sek.-butyl-phenylisothiocyanat und tert. Pentylamin die Verbindung
N-(2-Äthyl-6-sek.-butyl-phenyl)-N'-tert.-pentyl-thioharnstoff vom Schmelzpunkt 74 - 76°C;

aus 2-Isopropyl-6-sek.-butyl-phenylisothiocyanat und tert.-Butylamin die Verbindung
N-(2-Isopropyl-6-sek.-butyl-phenyl)-N'-tert.-butyl-thioharnstoff vom Schmelzpunkt 121 - 123oC;

aus 2-Isopropyl-6-sek.-butyl-phenyl)-isothiocyanat und tert. Pentylamin die Verbindung
N-(2-Isopropyl-6-sek.-butyl-phenyl)-N'-tert.-pentyl-thioharnstoff vom Schmelzpunkt 51 - 53°C.

Beispiel 16

N-(2,6-Diisopropyl-phenyl)-N'-tert.-butyl-thioharnstoff

15 g 2,6-Diisopropyl-phenylisothiocyanat werden in 25 g tert.-Butylamin eingetragen und 12 Stunden bei 20°C aufbewahrt. Danach gießt man in verd. Salzsäure, filtriert, wäscht und trocknet.
Ausbeute 17 g, F: 135 - 137°C.

Beispiel 17

N-(2,6-Diisopropyl-phenyl)-N'-tert.-pentyl-thioharnstoff

11,0 g 2,6-Diisopropyl-phenylisothiocyanat und 10 g tert.-Pentylamin werden mit 5 ml Triäthylamin 10 Minuten auf 100°C erwärmt. Nach 12-stündigem Stehen wird das teilweise auskristllisierte Gemisch mit Petroläther verrührt, auf -10°C gekühlt, abfiltriert und getrocknet.
Ausbeute 14,0 g; F: 134 - 135°C.

Durch Umsetzen von 15,0 g 2,6-Diisopropyl-phenylisothiocyanat mit 9,0 g 1,1,2,2-Tetramethyl-propylamin in 10 ml Triäthylamin, wie vorstehend beschrieben, wird der N-(2,6-Diisopropyl-phenyl)-N'-(1,1,2,2-tetramethyl-propyl)-thioharnstoff, F: 161 - 162°C, erhalten.

Beispiel 18

N-(2,6-Diäthyl-phenyl)-N'-tert.-butyl-thioharnstoff

20 g 2,6-Diäthyl-phenylisothiocyanat werden in 20 g tert.-Butylamin eingetragen. Man kühlt derart, daß 40°C nicht überschritten werden. Nach 3 Stunden wird mit verdünnter Salzsäure verrührt, das kristalline Reaktionsprodukt abgesaugt, neutral gewaschen und getrocknet.
Ausbeute 28,0 g; F: 98 - 100°C.

Ersetzt man in vorstehendem Beispiel das tert.-Butylamin durch die gleiche Menge tert.-Pentylamin und arbeitet wie angegeben, so erhält man den N-(2,6-Diäthyl-phenyl)-N'-tert.-pentyl-thioharnstoff; F: 85 - 87°C.

Beispiel 19

N-(2,6-Di-sec.-butyl-phenyl)-N'-isopropyl-thioharnstoff

15,0 g 2,6-Di-sec.-butyl-phenylisothiocyanat werden unter Kühlung in 20 g einer 65 %igen wäßrigen Lös-ng von Isopropylamin eingetragen. Nach 3-stündigem Rühren bei 20°C wird der Ansatz in verdünnte Salzsäure eingerührt, das kristallin ausgefallene Reaktionsprodukt abgesaugt, neutral gewaschen, die Paste mit 50 %igem Methanol verrieben, nochmals abgesaugt und getrocknet.
Ausbeute 17,0 g; F: 108 - 111°C.

Ersetzt man in obigem Beispiel das Isopropylamin durch 15,0 g sec.-Butylamin und arbeitet wie angegeben, so erhält man den N-(2,6-Di-sec.-butyl-phenyl)-N'-sec.-butyl-thioharnstoff;
F: 103 - 105°C

Durch Umsetzung von 2,6-Diäthyl-phenylisothiocyanat mit Isopropylamin nach dem vorstehend angegebenen Verfahren erhält man N-(2,6-Diäthyl-phenyl)-N'-isopropyl-thioharnstoff vom F: 108 bis 110°C; mit sec.-Butylamin den N-(2,6-Diäthyl-phenyl)-N'-sec.-butyl-thioharnstoff vom F: 98 - 80°C

Beispiel 20

N-(2-Methyl-6-äthyl-phenyl)-N'-tert.-pentyl-thioharnstoffe

15,0 g 2-Methyl-6-äthyl-phenylisothiocyanat werden unter Kühlung in 20,0 g tert.-Pentylamin eingetragen. Nach 2 Stunden wird der Ansatz in verd. Salzsäure eingerührt, das Reaktionsprodukt abgesaugt, mit 50 %igem Methanol verrieben, abgesaugt und getrocknet.
Ausbeute 19,0 g; F: 78 - 81°C.

Wird in vorstehendem Beispiel das tert.-Pentylamin durch die gleiche Menge tert.-Butylamin ersetzt, so erhält man den N-(2-Methyl-6-äthyl-phenyl)-N'-tert.-butyl-thioharnstoff; F: 94 - 96°C.

2-Methyl-6-äthyl-phenylisothiocyanat kann folgendermaßen hergestellt werden:

100 g 2-Methyl-6-äthyl-anilin in 200 ml Methylenchlorid werden bei 0 - 5° zu einem Gemisch von 500 ml Methylenchlorid, 300 ml Wasser, 120 g Calciumcarbonat und 103 g Thiophosgen zugetropft. Danach wird unter Rückfluß erhitzt, bis die $CO_2$-Entwicklung beendet ist. Danach filtriert man von Feststoffen, trennt die Methylenchloridschicht ab, trocknet über Calciumchlorid und fraktioniert.
Ausbeute 121 g; Kp: 88 - 91°C/1,0 Torr.

Beispiel 21

N-(4-Methyl-2,6-diäthyl-phenyl)-N'-tert.-butyl-thioharnstoff

15,0 g 4-Methyl-2,6-diäthyl-phenyl-isothiocyanat werden unter Kühlung in 20,0 g tert.-Butylamin eingetragen. Man rührt 2 Stunden, gießt in verdünnte Salzsäure, filtriert, wäscht und trocknet.
Ausbeute 18,0 g; F: 121 - 123°C.

Ersetzt man in obigem Beispiel das tert.-Butylamin durch die gleiche Menge tert.-Pentylamin und arbeitet wie angegeben, so erhält man den N-(4-Methyl-2,6-diäthyl-phenyl)-N'-tert.-pentyl-thioharnstoff, F: 98 - 101°C

Analog erhält man aus 2,4-Dimethyl-6-äthyl-phenylisothiocyanat und tert.-Butylamin den N-(2,4-Dimethyl-6-äthyl-phenyl)-N'-tert.-butyl-thioharnstoff, F: 130 - 132°C, und aus dem gleichen subst. Phenylisothiocyanat und tert.-Pentylamin den N-(2,4-Dimethyl-6-äthyl-phenyl)-N'-tert.-pentyl-thioharnstoff, F: 105 - 107°C.

Das vorstehend als Ausgangsverbindung verwendete 2,4-Dimethyl-6-äthyl-phenylisothiocyanat kann nach folgender Vorschrift hergestellt werden:

100 g 2,4-Dimethyl-6-äthylanilin in 200 ml Methylenchlorid werden bei 0 - 5°C zu einem Gemisch aus 500 ml Methylenchlorid, 300 ml Wasser, 120 g Calciumcarbonat und 92 g Thiophosgen unter Rühren zugetropft. Anschließend wird zum Rückfluß erhitzt, bis die $CO_2$-Entwicklung beendet ist. Nach dem Abkühlen wird der Ansatz von Feststoffen abfiltriert, die Methylenchloridschicht abgetrennt, über Calciumchlorid getrocknet und fraktioniert; Ausbeute 118 g, Kp 101 - 104°C/1,5 Torr.

Beispiel 22

4-Cyclohexyl-2,6-diäthyl-phenylisothiocyanat

231 g 4-Cyclohexyl-2,6-diäthyl-anilin in 300 ml Methylenchlorid werden bei 10 - 15°C zu einer Suspension aus 600 ml Methylenchlorid, 500 ml Wasser, 200 g Calciumcarbonat und 140 g Thiophosgen zugetropft. Danach heizt man zum Sieden, bis die Gasentwicklung beendet ist. Der erkaltete Ansatz wird von Feststoffen abfiltriert, die Methylenchloridschicht über Calciumchlorid getrocknet und fraktioniert; Kp 175 - 179°C/1,5 Torr; Ausbeute 251 g.

Analog können aus den entsprechenden Anilinderivaten die folgenden Arylisothiocyanate hergestellt werden:

2,4,6-Triäthyl-phenylisothiocyanat; Kp 128 - 130°C/1,3 Torr
4-n-Propyl-2,6-diäthyl-phenylisothiocyanat; Kp 132 - 137°C/1,5 Torr
4-Isopropyl-2,6-diäthyl-phenylisothiocyanat; Kp 130 - 132°C/1,3 Torr
4-n-Butyl-2,6-diäthyl-phenylisothiocyanat; Kp 150 - 155°C/2,0 Torr
4-Isobutyl-2,6-diäthyl-phenylisothiocyanat; Kp 133 - 136°C/1,4 Torr
4-tert.-Butyl-2,6-diäthyl-phenylisothiocyanat; Kp 130 - 133°C/1,8 Torr
2,6-Di-cyclopentyl-phenylisothiocyanat; Kp 165 - 158°C/1,3 Torr
4-Methyl-2,6-dicyclopentyl-phenylisothiocyanat; Kp 188 - 195°C/2,0 Torr

2,6-Di-pentyl-(2)-phenylisothiocyanat; Kp 148 - 152°C/1,4 Torr

4-Methyl-2,6-di-sec.-butyl-phenylisothiocyanat; Kp 130 - 132°C/1,2 Torr

2-Methyl-4,6-di-tert.-butyl-phenylisothiocyanat; Kp 135 - 139°C/1,5 Torr

3-Methyl-2,6-diäthyl-phenylisothiocyanat; Kp 110 - 113°C/1,0 Torr

3-Chlor-2,6-diäthyl-phenylisothiocyanat; Kp 118 - 121°C/1,2 Torr

3,4-Dimethyl-2,6-diäthyl-phenylisothiocyanat; Kp 125 - 130°C/1,2 Torr

3,5-Dimethyl-2,6-diäthyl-phenylisothiocyanat; Kp 119 - 127°C/1,0 Torr

4-Methyl-2-äthyl-6-sec.-butyl-phenylisothiocyanat; Kp 126 - 128°C/1,5 Torr

4-Methyl-2,6-diisopropyl-phenylisothiocyanat; Kp 124 - 126°C/1,2 Torr

2-Isopropyl-6-sec.-butyl-phenylisothiocyanat; Kp 115 - 117°C/1,0 Torr

2,4,6-Tri-isopropyl-phenylisothiocyanat; Kp 130 - 132°C/1,2 Torr

2-Äthyl-6-isopropyl-phenylisothiocyanat; Kp 104 - 106°C/1,2 Torr

4-[Pentyl-(3)]-2,6-diäthyl-phenylisothiocyanat; Kp 147 - 149°C/1,2 Torr

Die für die Synthese der Arylisothiocyanate als Ausgangsmaterial erforderlichen 2,6-disubstituierten Anilinderivate können nach den in Angew. Chemie Bd. 69, S. 124 ff (1957) beschriebenen Verfahren hergestellt werden.

Beispiel 23

4-Cyclohexyl-2,6-diäthyl-anilin

300 g 4-Amino-cyclohexylbenzol, 5,0 g Aluminiumgranulat und 17 g wasserfreies Aluminiumchlorid werden in einem Stahlautoklaven auf 250°C erhitzt und Äthylen bis zu einem Innendruck von 200 atü aufgepreßt. Nach Druckabfall wird weiter Äthylen zugepumpt, bis die Aufnahme beendet ist; Dauer ca. 7 Stunden. Nach dem Erkalten wird der Ansatz mit 500 ml Benzol, 300 ml 40 %iger Natronlauge und 500 ml Wasser 15 Minuten bei 40 - 50°C verrührt, die Benzolphase abgetrennt, mit Wasser gewaschen, über Kaliumcarbonat getrocknet und fraktioniert.
Kp 148 - 150°C/0,8 Torr; Ausbeute 318 g.

In analoger Weise können die folgenden Anilinderivate hergestellt werden:

2,4,6-Triäthyl-anilin; Kp 89 - 91°C/0,6 Torr
4-n-Propyl-2,6-diäthyl-anilin; Kp 102°C/1,4 Torr
4-Isopropyl-2,6-diäthyl-anilin; Kp 103 - 105°C/2,0 Torr
4-n-Butyl-2,6-diäthyl-anilin; Kp 117 - 118°C/2,0 Torr
4-Isobutyl-2,6-diäthyl-anilin; Kp 97 - 99°C/0,7 Torr
4-tert.-Butyl-2,6-diäthyl-anilin; Kp 89 - 91°C/0,6 Torr

2-Äthyl-6-isopropyl-anilin; Kp 127 - 128°C/16 Torr
2-Isopropyl-6-sec.-butyl-anilin; 136 - 143°C/13 Torr
3-Methyl-2,6-diäthyl-anilin; Kp 132 - 133°C/20 Torr
3-Chlor-2,6-diäthyl-anilin; Kp 145 - 148°C/15 Torr
3,4-Dimethyl-2,6-diäthyl-anilin; Kp 147 - 148°C/15 Torr

3,5-Dimethyl-2,6-diäthyl-anilin; Kp 146 - 154°C/15 Torr, F: 47 - 50°C

4-Methyl-2,6-diisopropyl-anilin; Kp 141 - 143°C/16 Torr

4-/Pentyl-(3)/-2,6-diäthyl-anilin; Kp 106 - 107°C/1,2 Torr

**Beispiel 24**

**2,6-Bis-(pentyl-(2))-anilin**

170 g Anilin, 5 g Aluminiumgranulat und 15 g wasserfreies Aluminiumchlorid werden in einem Stahlautoklaven auf 300°C erhitzt und 300 g Penten-(1) innerhalb von etwa 5 Stunden bis zu einem Innendruck von 300 atü eingepumpt. Anschließend wird der Ansatz noch 6 Stunden bei 300°C gehalten, wobei der Innendruck auf 107 atü fällt. Nach dem Erkalten wird der Autoklaveninhalt mit 500 g Benzol, 250 ml 40 %iger Natronlauge und 300 ml Wasser 15 Minuten bei 30 - 40°C verrührt, die Benzolschicht mit Wasser gewaschen, über Kaliumcarbonat getrocknet und fraktioniert. Man erhält 113 g 2-Mono-pentyl-(2)-anilin. Kp 78 - 82°C/0,6 Torr und 131 g 2,6-Bis-pentyl-(2)-anilin, Kp 168 - 174°C/1,5 Torr.

Analog erhält man aus Anilin und Cyclopenten
2-Cyclopentylanilin; Kp 102 - 109°C/1,7 Torr und
2,6-Di-cyclopentyl-anilin; Kp 159 - 165°C/1,5 Torr;
aus p-Toluidin und Cyclopenten
4-Methyl-2-cyclopentyl-anilin; Kp 104 - 106°C/0,7 Torr und
4-Methyl-2,6-dicyclopentyl-anilin; Kp 157 - 158°C/0,8 Torr;
aus p-Toluidin und Buten-(1)
4-Methyl-2-sec.-butyl-anilin; Kp 72°C/1,0 Torr und

4-Methyl-2,6-di-sec.-butyl-anilin; Kp 121 - 128°C/3,0 Torr. Aus Anilin und Buten-(1) erhält man analog 2-sec.-Butyl-anilin; Kp 109 - 111°C/13 Torr und 2,6-Di-sec.-butyl-anilin; Kp 145 - 147°C/13 Torr. Aus o-Toluidin und Isobuten erhält man mit Tonsil K 10 als Katalysator bei 200°C und 200 atü das 6-Methyl-2,4-di-tert.-butyl-anilin; Kp 101 - 103°C/1,1 Torr.

Die erfindungsgemäß dargestellten Verbindungen der allgemeinen Formel (I), welche sämtlich als ölige Substanzen anfallen,können durch die folgenden NMR- und IR-Daten charakterisiert werden:

Die einzelnen Abkürzungen in den Tabellen der NMR-Werte , bedeuten

S = Singulett
D = Dublett
T = Triplett
Q = Quaternett
M = Multiplett

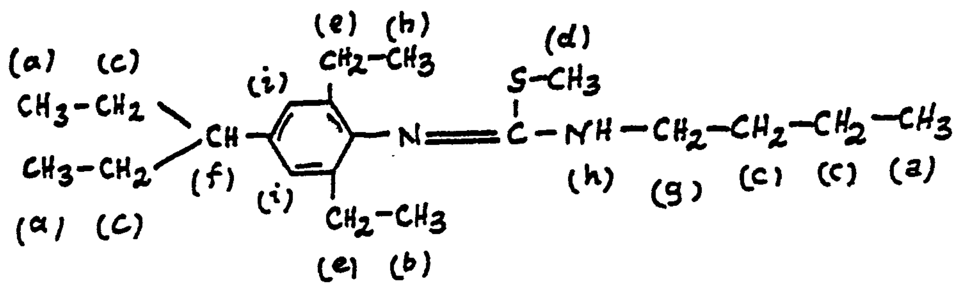

| Shift ppm NMR in $CDCl_3$ | Multiplizität | Zuordnung | Intensität |
|---|---|---|---|
| 0,75 | T | a | |
| 0,15 | T | b | überlagert 23 |
| 0,9 - 1,9 | M | c | |
| 2,3 | S | d | |
| 2,35 | Q | e | überlagert 8 |
| 1,9 - 2,7 | M | f | |
| 3,0 - 3,5 | M | g | 2 |
| 4,15 | S | h | 1 |
| 6,75 | S | i | 2 |

IR: 2955, 1615, 1456, 868 $cm^{-1}$

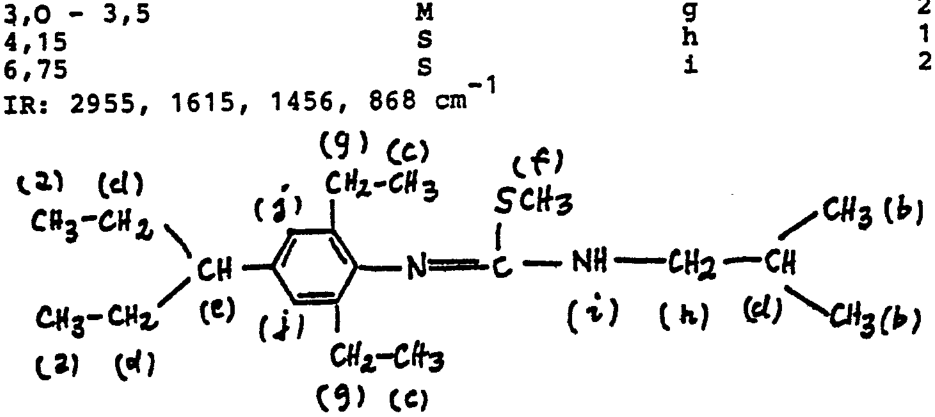

| Shift ppm NMR in $CDCl_3$ | Multiplizität | Zuordnung | Intensität |
|---|---|---|---|
| 0,8 | T | a | |
| 0,85 | D | b | überlagert 23 |
| 1,15 | T | c | |
| 0,9 - 1,9 | M | d | |
| 1,9 - 2,7 | M | e | |
| 2,4 | S | f | überlagert 8 |
| 2,45 | Q | g | |
| 3,08 | T | h | 2 |
| 4,25 | M | i | 1 |
| 6,78 | S | j | 2 |

IR: 2955, 1615, 1455, 868 $cm^{-1}$

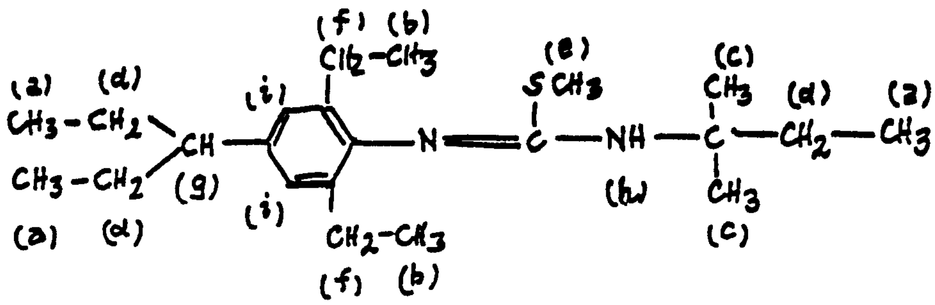

| Shift ppm NMR in $CDCl_3$ | Multiplizität | Zuordnung | Intensität |
|---|---|---|---|
| 0,75 | T's | a | |
| 1,15 | T | b überlagert | 27 |
| 1,4 | S | c | |
| 0,9 - 2,2 | M | d | |
| 2,15 | S | e | |
| 2,45 | M | f überlagert | 8 |
| 2,3 - 2,9 | M | g | |
| 4,0 | S | h | 1 |
| 6,73 | S | i | 2 |

IR: 2959, 1625, 1455, 1122, 868 $cm^{-1}$

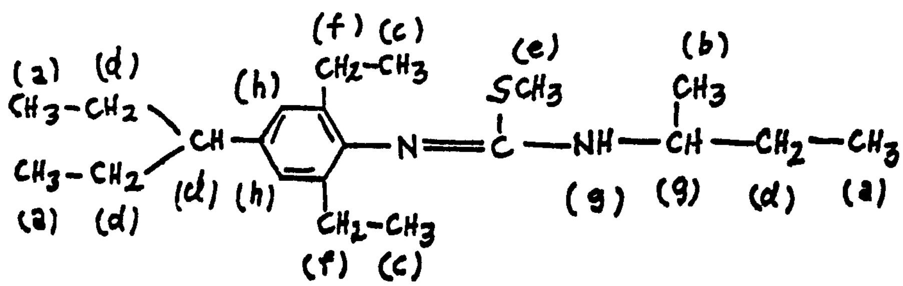

| Shift ppm NMR in $CDCl_3$ | Multiplizität | Zuordnung | Intensität |
|---|---|---|---|
| 0,75 | T | a | |
| 0,8 | D | b | ca. 25 |
| 1,15 | T | c | |
| 0,6 - 2,8 | M | d | |
| 2,38 | S | e | ca. 7 |
| 2,42 | Q | f | |
| 3,5 - 4,0 | M | g | 2 |
| 6,73 | S | h | 2 |

IR: 3370, 1616, 1595, 1456, 1279, 689 $cm^{-1}$.

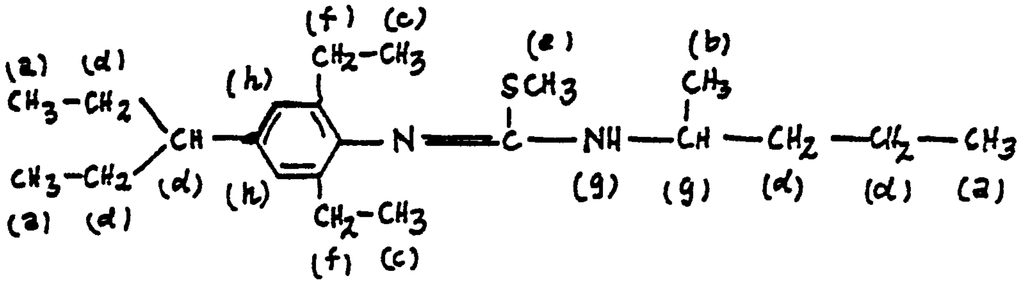

| Shift ppm NMR in $CDCl_3$ | Multiplizität | Zuordnung | Intensität |
|---|---|---|---|
| 0,77 | T | a | |
| 0,80 | D | b | ca. 27 |
| 1,15 | T | c | |
| 0,7 - 2,9 | M | d | |
| 2,38 | S | e | ca. 7 |
| 2,42 | Q | f | |
| 3,5 - 4,1 | S | g | 2 |
| 6,75 | S | h | 2 |

IR: 3370, 1610, 1590, 1452, 1280, 1150, 871 $cm^{-1}$

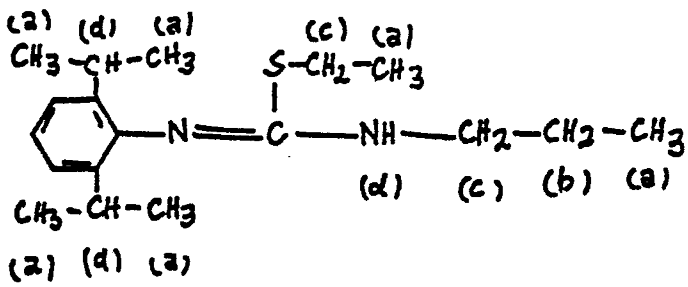

| Shift ppm NMR in $CDCl_3$ | Multiplizität | Zuordnung | Intensität |
|---|---|---|---|
| 0,9 - 1,2 | T+D | I a | ~18 |
| 2,6 | M | b | ~ 4 |
| 2,9 - 3,5 | M+DMF | c | ~ 6+DMF |
| 4,2 | breit | d | 1 |
| 7,0 + 7,2 | M | e | 3 |

IR: 3380 (NH, 780, 730 (Aromat. 1,2,3-Trisubst. möglich).

1670 ( 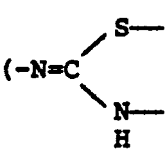 ).

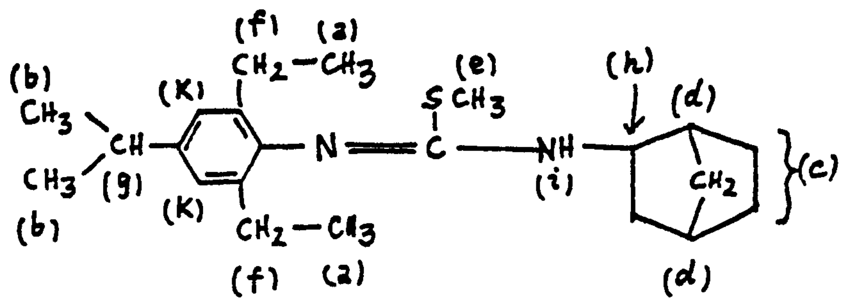

| Shift ppm | Multiplizität | Zuordnung | Intensität |
|---|---|---|---|
| NMR: (in $CDCl_3$) | | | |
| 1,17 | T | I a | 6 |
| 2,23 | D | b | 6 |
| 1,0 - 2,0 | M | c | 8 |
| 2,0 - 2,4 | M | d | 2 |
| 2,38 | S | e | 3 |
| 2,46 | Q | f | 4 |
| 2,6 - 3,1 | M | g | 1 |
| 3,3 - 3,8 | M | h | 1 |
| 4,0 | D | i | 1 |
| 6,88 | S | k | 2 |

IR: 1615 $cm^{-1}$ = C=N-Bande
876 $cm^{-1}$ = 1,2,3,5-Tetrasubst.

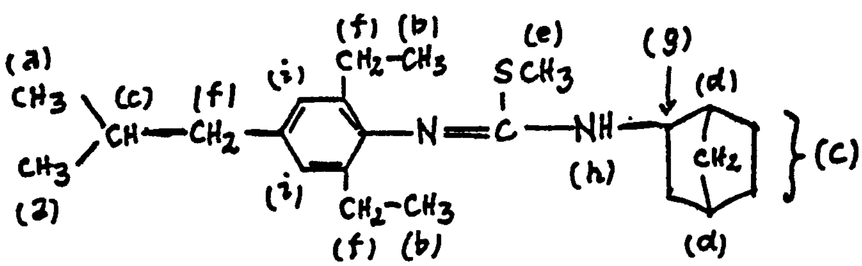

| Shift ppm | Multiplizität | Zuordnung | Intensität |
|---|---|---|---|
| NMR: (in $CDCl_3$) | | | |
| 0,89 | D | I a | 6 |
| 1,16 | T | b | 6 |
| 1,0 - 2,0 | M | c | 8+1 |
| 2,1 - 2,4 | M | d | 2 |
| 2,38 | S | e | 3 |
| 2,43 | Q | f | 6 |
| 3,3 - 3,9 | M | g | 1 |
| 4,0 | D | h | 1 |
| 6,81 | S | i | 2 |

IR: 879 $cm^{-1}$ = 1,2,3,5-Tetrasubst.
1615 $cm^{-1}$ = C=N-Bande

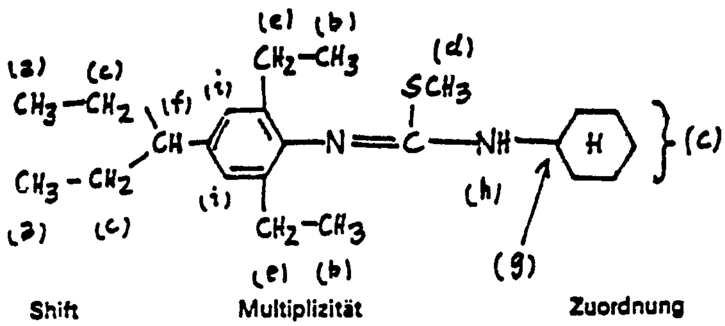

| Shift ppm | Multiplizität | Zuordnung | Intensität |
|---|---|---|---|
| NMR: ($CDCl_3$) | | | |
| 0,78 | T | I a | |
| 1,18 | T | b | |
| 1,0-2,2 | M | c | 34 |
| 2,38 | S | d | |
| 2,0-2,7 | M | e | |
| ca. 2,4-3,1 | M | f | |
| 3,1-4,0 | M | g | 1 |
| 4,1 | M | h | 1 |
| 6,78 | S | i | 2 |

IR:

871 $cm^{-1}$ = 1,2,3,5-Tetrasubst.
1610 $cm^{-1}$ = C=N-Bande

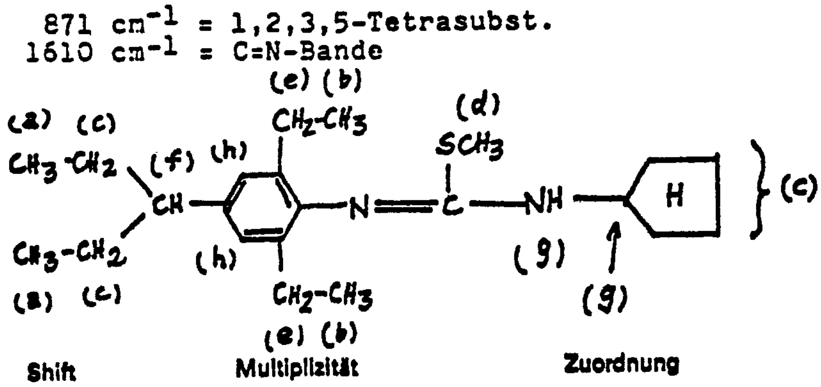

| Shift ppm | Multiplizität | Zuordnung | Intensität |
|---|---|---|---|
| NMR: (in $CDCl_3$) | | | |
| 0,78 | T | I a | 6 |
| 1,18 | T | b | 6 |
| 1,2-2,0 | M | c | 12 |
| 2,40 | S | d | 3 |
| 2,1-2,8 | Q | e | 4 |
| 2,4-3,0 | M | f | 1 |
| 3,7-4,3 | MS | g | 2 |
| 6,77 | S | h | 2 |

IR:

871 $cm^{-1}$ = 1,2,3,5-Tetrasubst.
1610 $cm^{-1}$ = C=N-Bande

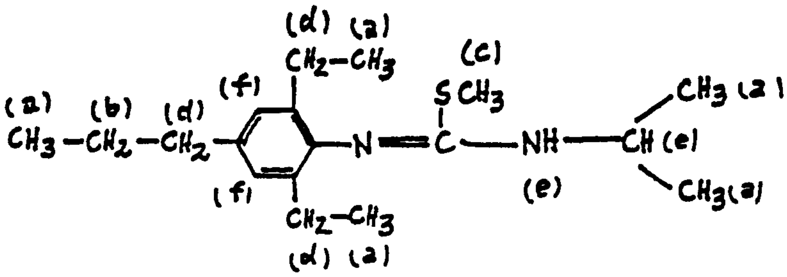

| Shift ppm | Multiplizität | Zuordnung | Intensität |
|---|---|---|---|
| NMR: (in $CDCl_3$) | | | |
| 0,7-1,3 | M | I a | 15 |
| 1,2-2,0 | M | b | 2 |
| 2,37 | S | c | 3 |
| 2,45 | Q | d | 6 |
| 3,6-4,2 | M | e | 2 |
| 6,86 | S | f | 2 |

IR: 1615 $cm^{-1}$ = C=N-Bande

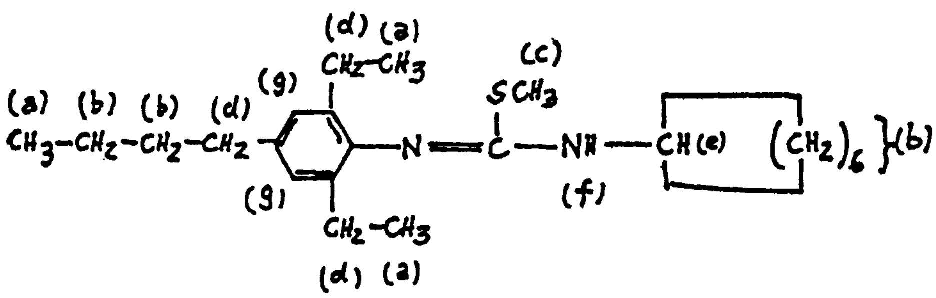

| Shift ppm | Multiplizität | Zuordnung | Intensität |
|---|---|---|---|
| NMR: (in $CDCl_3$) | | | |
| 0,8-1,3 | M | I a } | 25 |
| 1,3-2,0 | M | b } | |
| 2,37 | S | c } | 9 |
| 2,1-2,8 | M | d } | |
| 3,5-4,1 | M | e | 1 |
| 4,15 | D | f | 1 |
| 6,84 | S | g | 2 |

IR: 868 $cm^{-1}$ = 1,2,3,5-Tetrasubst.
1595 $cm^{-1}$ od. 1615 $cm^{-1}$ = C=N-Bande

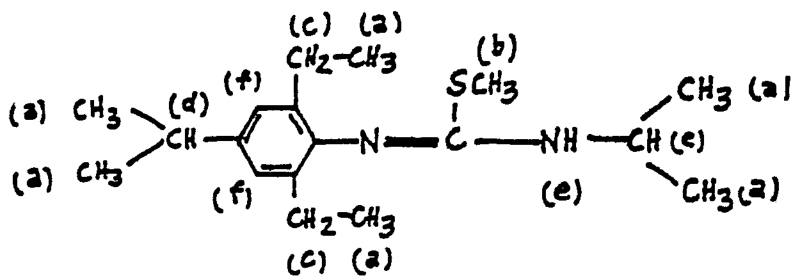

| Shift ppm NMR in $CDCl_3$ | Multiplizität | Zuordnung | Intensität |
|---|---|---|---|
| 0,95-1,4 | M | I a | 18 |
| 2,38 | S | b | 3 |
| 2,45 | Q | c | 4 |
| 2,5-3,1 | M | d | 1 |
| 3,8-4,2 | M | e | 1 |
| 6,89 | S | f | 2 |

IR: 874 $cm^{-1}$ = 1,2,3,5-Tetrasubst.
1610 $cm^{-1}$ = C=N-Bande

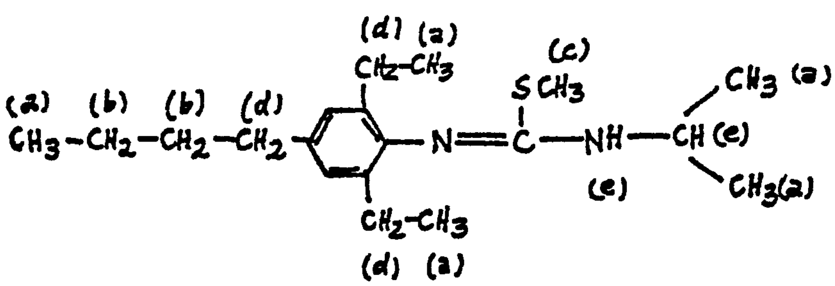

| Shift ppm NMR in $CDCl_3$ | Multiplizität | Zuordnung | Intensität |
|---|---|---|---|
| 0,7-1,4 | M | I a } | 19 |
| 1,0-2,0 | M | b } | |
| 2,37 | S | c | 3 |
| 2,44 | Q | d | 6 |
| 3,7-4,2 | M | e | 2 |
| 6,86 | S | f | 2 |

IR: 1615 $cm^{-1}$ = C=N-Bande
864 $cm^{-1}$ = 1,2,3,5-Tetrasubst.

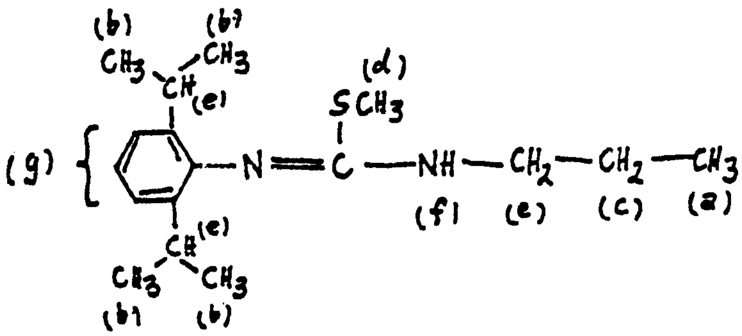

| Shift ppm | Multiplizität | Zuordnung | Intensität |
|---|---|---|---|
| NMR: (in $CDCl_3$) | | | |
| 0,88 | T | I a | 3 |
| 1,17/1,18 | D/D | b | 12 |
| 1,0-1,8 | M | c | 2 |
| 2,42 | S | d | 3 |
| 2,7-3,4 | M | e | 4 |
| 4,0-4,3 | M | f | 1 |
| 6,9-7,2 | M | g | 3 |

IR: 755 $cm^{-1}$ u. 772 $cm^{-1}$ = 1,2,3-Trisubst.
1612 $cm^{-1}$ = C=N-Bande

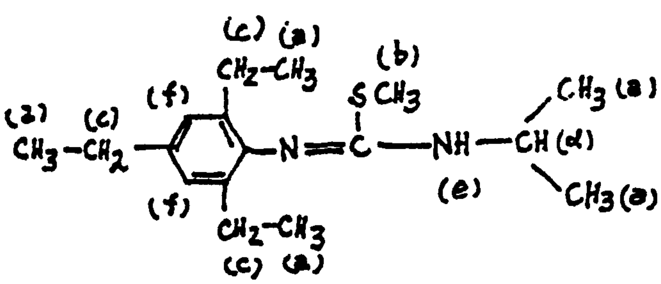

| Shift ppm | Multiplizität | Zuordnung | Intensität |
|---|---|---|---|
| NMR: (in $CDCl_3$) | | | |
| 0,9-1,4 | M | I a | 15 |
| 2,38 | S | b | 3 |
| 2,43 | Q | c | 6 |
| 3,7-4,3 | M | d | 1 |
| 3,9-4,5 | M | e | 1 |
| 6,88 | S | f | 2 |

IR: 874 $cm^{-1}$ = 1,2,3,5-Tetrasubst.
1615 $cm^{-1}$ = C=N-Bande

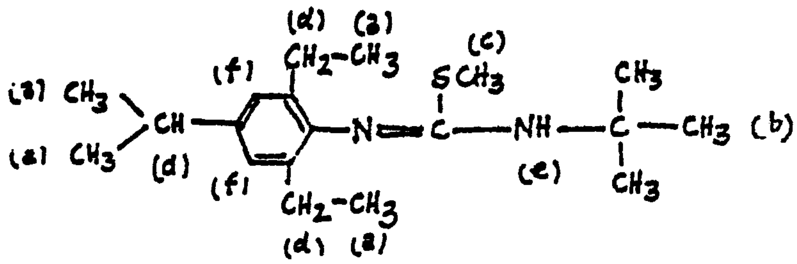

| Shift ppm | Multiplizität | Zuordnung | Intensität |
|---|---|---|---|
| NMR: (in $CDCl_3$) | | | |
| 1,2 | Q + D | I a | 12 |
| 1,4 | S | b | 9 |
| 2,2 | S | c | 3 |
| 2,2-2,9 | M | d | > 5 |
| 4,1 | S | e | 1 |
| 6,8 | S | f | 2 |

IR: 3450 (NH), 875, 895 (Aromat. 1,2,3,5-Tetrasubst.).

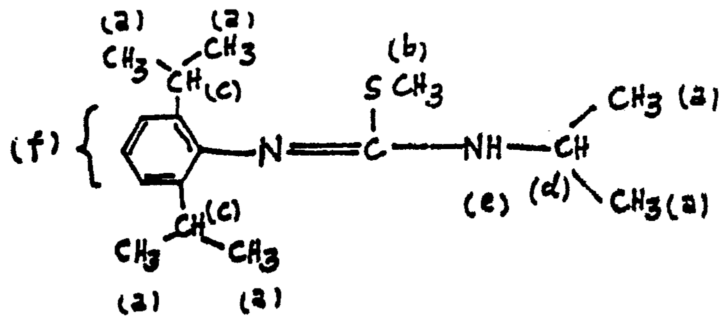

| Shift ppm | Multiplizität | Zuordnung | Intensität |
|---|---|---|---|
| NMR: (in $CDCl_3$) | | | |
| 1,17/1,18 | D/D | I a | 18 |
| 2,38 | S | b | 3 |
| 2,6-3,2 | M | c | 2 |
| 3,6-4,1 | M | d | 1 |
| 3,9 | D | e | 1 |
| 6,9-7,2 | M | f | 3 |

IR: 697 $cm^{-1}$ od. 755 $cm^{-1}$ u. 767 $cm^{-1}$ = 1,2,3-Trisubst.
1580 $cm^{-1}$ od.1610 $cm^{-1}$ = C=N-Bande

Die Verbindung enthält ca. 5 % Dimethylformamid.

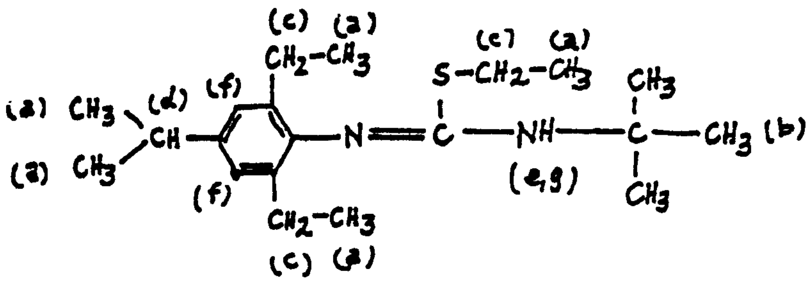

I

| Shift ppm | Multiplizität | Zuordnung | Intensität |
|---|---|---|---|
| NMR in $CDCl_3$ | | | |
| 1,2 | 2 x T + D | I a | 〉 24 |
| 1,5 | 2 x S | b | |
| 2,5 | 2 x Q | c | 〉 ~ 8 |
| 2,8 | (2x?)D | d | |
| 4,1 u. 5,2 | 2 x S | e | ~ 1 |
| 6,8 u. 7,0 | 2 x S | f | 2 |
| 7,5 u. 8,0 | 2 x S | g | ~ 1 |

IR: 3440, 3360, 3160 (NH), 880(Aromat. 1,2,3,5-Tetrasubst.).

Diese Daten lassen sich Struktur I zuordnen, die in zwei tautomeren Strukturen vorliegt.

$$R_1 - N = \overset{R2}{C} - NH - R_3$$

↓↑

$$R_1 - NH - \overset{R2}{C} = N - R_3$$

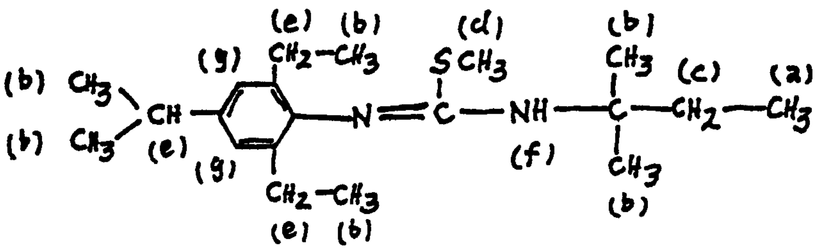

| Shift ppm | Multiplizität | Zuordnung | Intensität |
|---|---|---|---|
| NMR in $CDCl_3$ | | | |
| 0,9 | T | I a | ~ 3 |
| 1,1-1,4 | S+D+T | b | ~17 |
| 1,8 | Q | c | 2 |
| 2,2 | S | d | 3 |
| 2,2-2,7 | M | e | ~5 |
| 2,9 | DMF | | ~3 |
| 4,0 | S | f | 1 |
| 6,8 | S | g | 2 |
| 7,9 | S DMF | h | <1 |

IR: 3460, 3370 (NH), 875 (Aromat. 1,2,3,5-Tetrasubst.).

Die Verbindung enthält ca. 30 % Dimethylformamid.

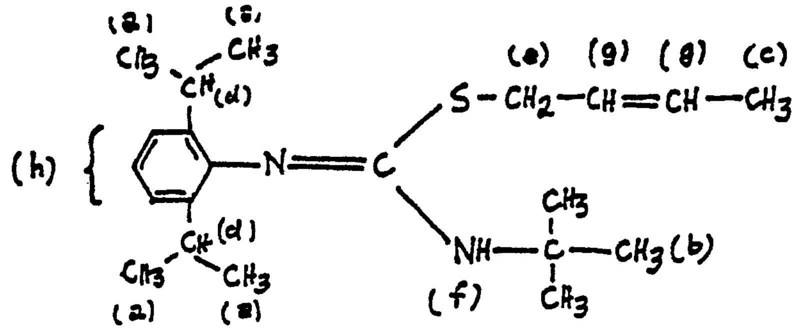

| Shift ppm | Multiplizität | Zuordnung | Intensität |
|---|---|---|---|
| NMR in $CDCl_3$ | | | |
| 1,2 | T | I a | 12 |
| 1,4 | S | b | 9 |
| 1,7 | D | c | ~ 4 |
| 3,0 | M | d | 2 |
| 3,3 | D | e | 2 |
| 4,4 | S | f | 1 |
| 5,6 | M | g | 2 |
| 7,0 | S | h | 3 |

IR: 3390 (NH), 755 (Aromat. 1,2,3-Trisubst.).

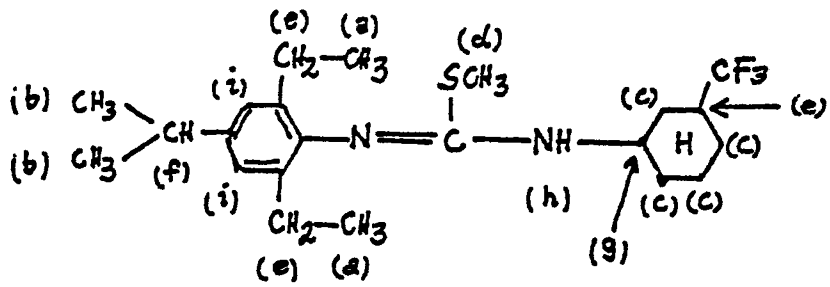

| Shift ppm | Multiplizität | Zuordnung | Intensität |
|---|---|---|---|
| NMR in $CDCl_3$ | | | |
| 0,8 - 1,2 | T | I a | 6 |
| 1,19 | D | b | 6 |
| 1,1-2,4 | M | c | 8 |
| 2,31 | S | d | 3 |
| 2,0-2,7 | M | e | 5 |
| 2,6-3,1 | M | f | 1 |
| 3,2-4,0 | M | g | 1 |
| 4,0 | D | h | 1 |
| 6,81 | S | i | 2 |

IR

1615 $cm^{-1}$ = C = N-Bande
871 $cm^{-1}$ = 1,2,3,5-Tetrasubst.
3370 $cm^{-1}$ o. 4450 $cm^{-1}$ = NH-Bande

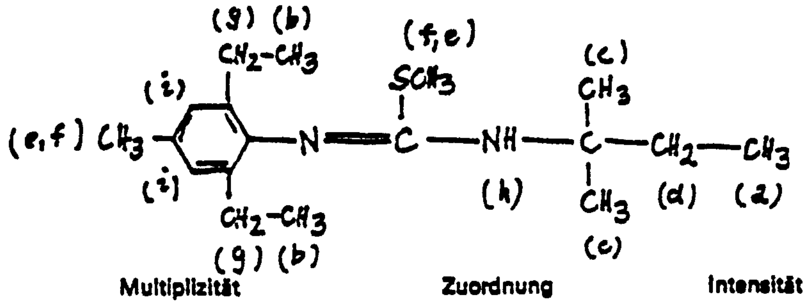

| Shift ppm | Multiplizität | Zuordnung | Intensität |
|---|---|---|---|
| NMR in $CDCl_3$ | | | |
| 0,90 | T | I a | 3 |
| 1,15 | T | b | 6 |
| 1,41 | S | c | 6 |
| 1,85 | Q | d | 2 |
| 2,19 | S | e | 3 |
| 2,23 | S | f | 3 |
| 2,43 | Q | g | 4 |
| 4,0 | S | h | 1 |
| 6,80 | S | i | 2 |

IR

855 $cm^{-1}$ o. 871 $cm^{-1}$ = 1,2,3,5-Tetrasubst.
1625 $cm^{-1}$ = C=N-Bande
3460 $cm^{-1}$ = NH-Bande

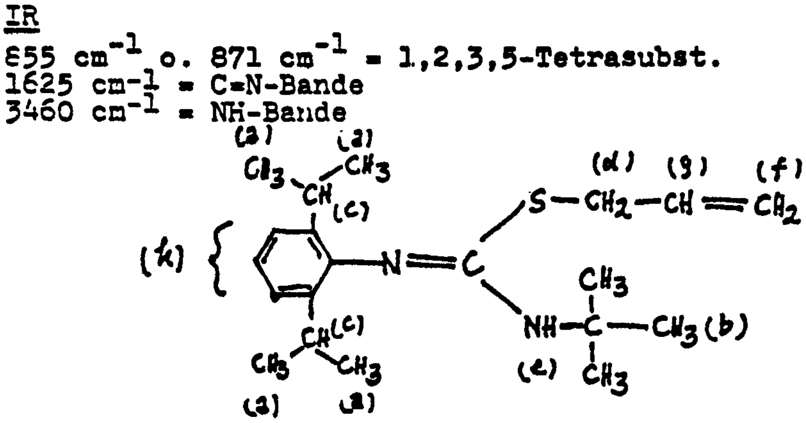

| Shift ppm | Multiplizität | Zuordnung | Intensität |
|---|---|---|---|
| NMR (in $CDCl_3$) | | | |
| 1,11/1,23 | D/D | I a | 12 |
| 1,46 | S | b | 9 |
| 3,02 | Septett | c | 2 |
| 3,38 | D | d | 2 |
| 4,4 | S | e | 1 |
| 5,1-5,5 | M | f | 2 |
| 5,70 | T (aufgesp.) | g | 1 |
| 7,04 | M | h | 1 |

IR

805 u. 761 $cm^{-1}$ = 1,2,3-Trisubst.
1625 $cm^{-1}$ = C = N-Bande
3420 $cm^{-1}$ = NH-Bande

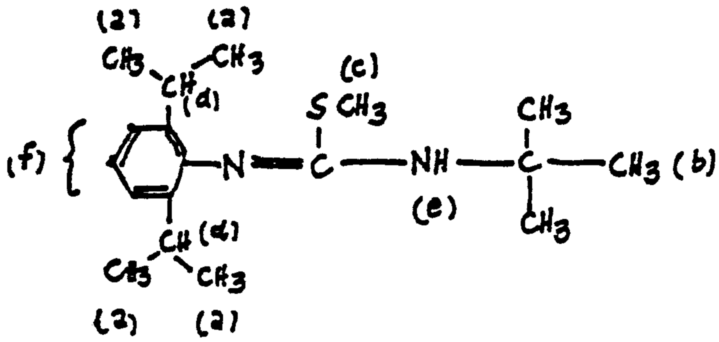

| Shift ppm | Multiplizität | Zuordnung | Intensität |
|---|---|---|---|
| NMR in $CDCl_3$ | | | |
| 1,2 | T | I a | ~ 12 |
| 1,5 | S | b | 9 |
| 2,2 | S | c | 3 |
| 3,0 | Sept. | d | 2 |
| 4,1 | S | e | 1 |
| 7,0 | S | f | 3 |

IR: 3460, 3370 (NH), 800, 760 (Aromat. 1,2,3-Trisubst.)

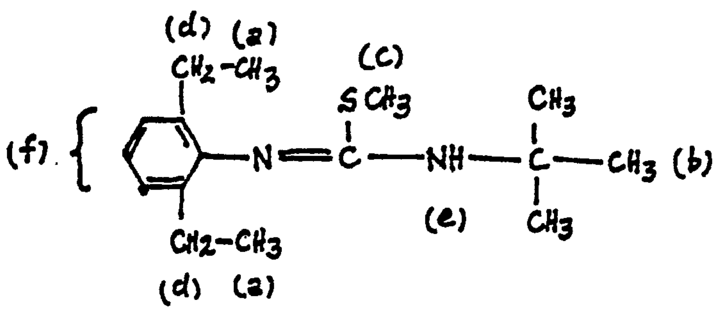

| Shift ppm | Multiplizität | Zuordnung | Intensität |
|---|---|---|---|
| <u>NMR</u> (in $CDCl_3$) | | | |
| 1,17 | T | I a | 6 |
| 1,47 | S | b | 9 |
| 2,18 | S | c | 3 |
| 2,50 | Q | d | 4 |
| 4,1 | S | e | 1 |
| 6,94 | S | f | 3 |

<u>IR:</u>

1620 $cm^{-1}$ = C=N-Bande

3450 $cm^{-1}$ = NH-Bande

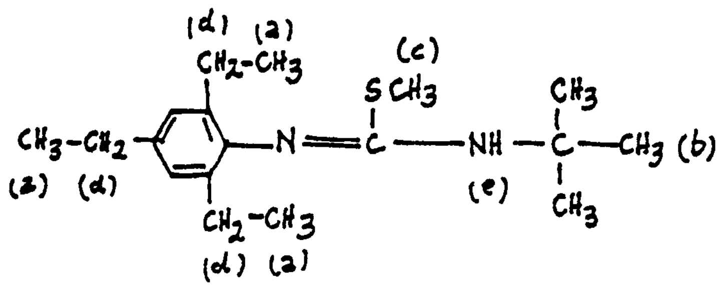

| Shift ppm | Multiplizität | Zuordnung | Intensität |
|---|---|---|---|
| NMR: (in $CDCl_3$) | | | |
| 1,16/1,21 | T | I a | 9 |
| 1,47 | S | b | 9 |
| 2,19 | S | c | 3 |
| 2,48 | Q | d | 6 |
| 4,1 | S | e | 1 |
| 6,87 | S | f | 2 |

IR:

870 $cm^{-1}$ = 1,2,3,5-Tetrasubst.
1620 $cm^{-1}$ = C=N-Bindung

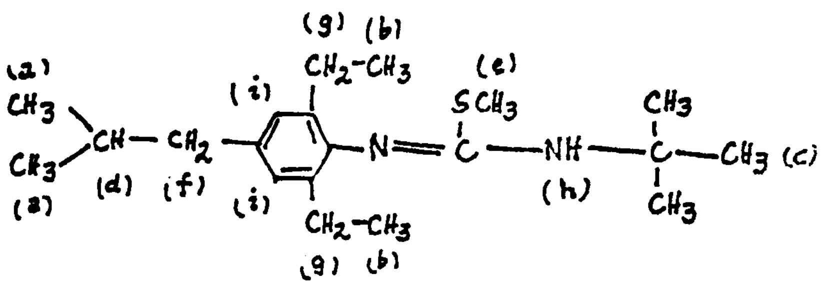

| Shift ppm | Multiplizität | Zuordnung | Intensität |
|---|---|---|---|
| NMR: (in $CDCl_3$) | | | |
| 0,90 | D | I a | 6 |
| 1,18 | T | b | 6 |
| 1,48 | S | c | 9 |
| 1,2-2,0 | M | d | 1 |
| 2,20 | S | e | 3 |
| 2,40 | D | f | 2 |
| 2,49 | Q | g | 4 |
| 4,1 | S | h | 1 |
| 6,80 | S | i | 2 |

IR:

ca. 880 $cm^{-1}$ = 1,2,3,5-Tetrasubst.
1620 $cm^{-1}$ = C=N-Bindung

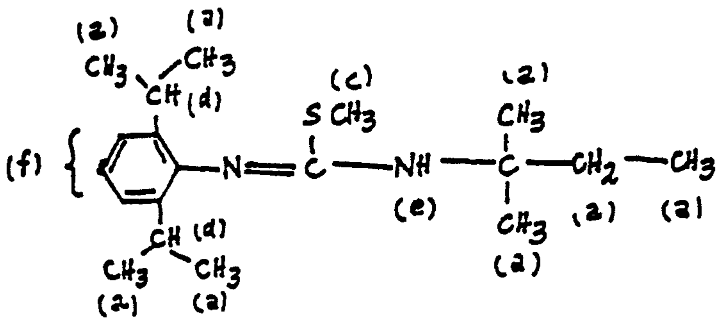

| Shift ppm | Multiplizität | Zuordnung | Intensität |
|---|---|---|---|
| NMR in $CDCl_3$ | | | |
| 0,6-1,5 | M | (a) | 21 |
| 1,6-2,3 | M | (b) überlagert | 5 |
| 2,2 | S | (c) | |
| ca. 2,95 | M | (d) | 2 |
| 4,0 | S | (e) | 1 |
| 7,0 | S | (f) | 3 |

IR: 3460 (NH), 2955, 1623 (C=N), 1145, 760 $cm^{-1}$

MS: $M^+$ 320, 273, 272 ($M-HSCH_3$), 187 (b.p.), 145.

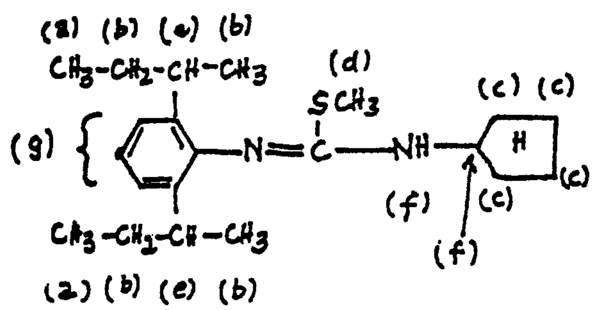

| Shift ppm | Multiplizität | Zuordnung | Intensität |
|---|---|---|---|
| NMR in $CDCl_3$ | | | |
| 0,81 | T | (a) | (6) |
| 1,15 | D | (b) überlagert | (18) |
| 1,0-2,3 | M | (c) | |
| 2,35-2,45 | S's | (d) überlagert | (5) |
| ca. 2,5 | M | (e) | |
| ca. 4,02 | M | (f) | (2) |
| 7,0 | M | (g) | (3) |

IR: 3380 (NH), 2958, 1614, 1582, 1275 $cm^{-1}$.

MS: $M^+$ 346, 300, 283, 270, 214, 201, 145, 69.

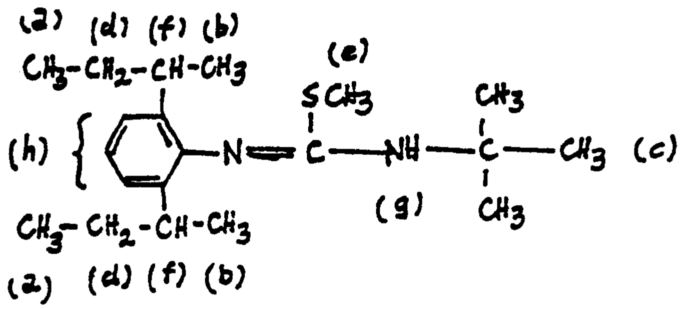

| Shift ppm | Multiplizität | Zuordnung | Intensität |
|---|---|---|---|
| NMR: (in $CDCl_3$) | | | |
| 0,88 | T | I a | 25 |
| 1,19 | D | I b | |
| 1,44 | S | I c | |
| 1,1-1,8 | M | I d | |
| 2,20 | S | I e | 3 |
| 2,4-2,9 | M | I f | 2 |
| 4,05 | S | I g | 1 |
| 6,92 | S | I h | 3 |

IR: 890 $cm^{-1}$ = 1,2,3,5-Tetrasubst.
3450 $cm^{-1}$ = NH-Bande

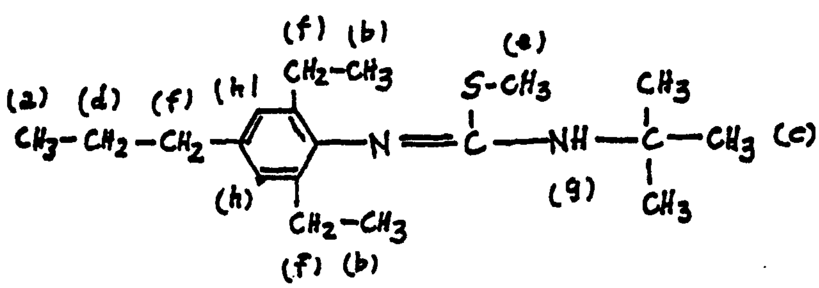

| Shift ppm | Multiplizität | Zuordnung | Intensität |
|---|---|---|---|
| NMR in $CDCl_3$ | | | |
| 1,05 | T | (a) | überlagert 9 |
| 1,17 | T | (b) | |
| 1,46 | S | (c) | 9 |
| 1,1-2,9 | M | (d) | ca. 2 |
| 2,19 | S | (e) | 3 |
| 2,47 | Q | (f) | 6 |
| 4,06 | S | (g) | 1 |
| 6,83 | S | (h) | 2 |

IR: 3455 (NH), 2958, 1521, 1457, 1125 $cm^{-1}$.

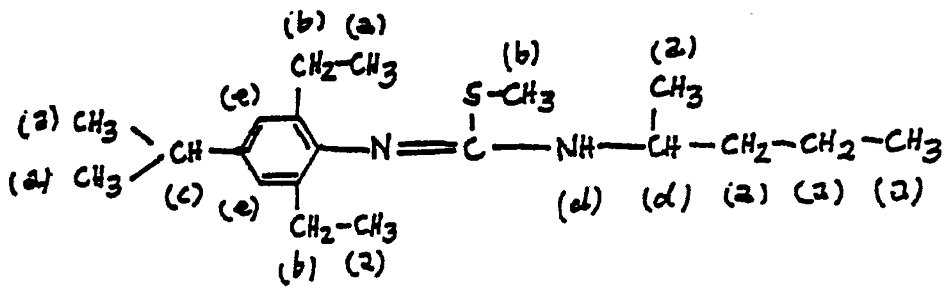

| Shift ppm NMR in $CDCl_3$ | Multiplizität | Zuordnung | Intensität |
|---|---|---|---|
| 0,9-1,4 | M | I a | 22 |
| 2,4 | Q + S | b | 7 |
| 2,8 | M | c | 1 |
| 3,9 | breit | d | 2 |
| 6,9 | S | e | 2 |

IR: 3450, 3370 (NH), 875 (Aromat. 1,2,3,5-Tetrasubst.).

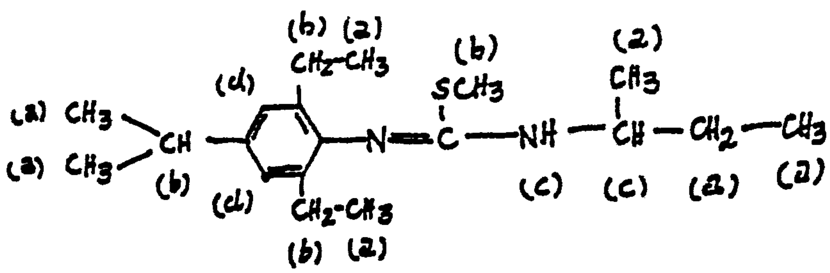

| Shift ppm | Multiplizität | Zuordnung | Intensität |
|---|---|---|---|
| NMR in $CDCl_3$ | | | |
| 0,9-1,2 | T + D | I a | ~ 20 |
| 2,4 | Q + S | b | ~ 9 |
| 3,8 | M | c | ~ 2 |
| 6,8 | S | d | 2 |

IR: 3460, 3370 (NH), 875 (Aromat. 1,2,3,5-Tetrasubst.).

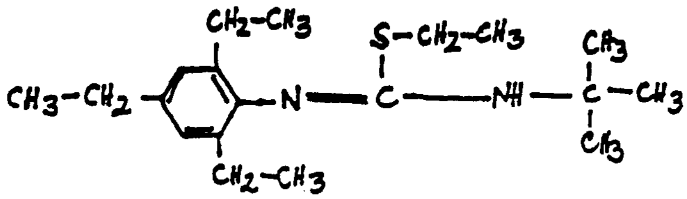

IR: 1620 $cm^{-1}$ = C = N-Bindung
870 $cm^{-1}$ = 1,2,3,5-Tetrasubst.

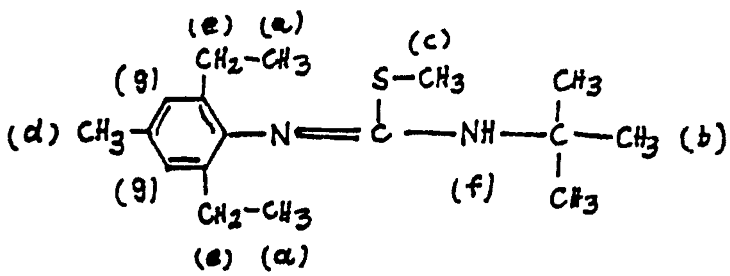

| Shift ppm | Multiplizität | Zuordnung | Intensität |
|---|---|---|---|
| NMR: (in $CDCl_3$) | | | |
| 1,16 | T | I a | > 15 |
| 1,48 | S | I b | |
| 2,19 | S | I c | |
| 2,25 | S | I d | > 10 |
| 2,45 | Q | I e | |
| 4,1 | S | I f | 1 |
| 6,8 | S | I g | 2 |

IR: 882 $cm^{-1}$ = 1,2,3,5-Tetrasubst.
3450 $cm^{-1}$ = NH-Bande

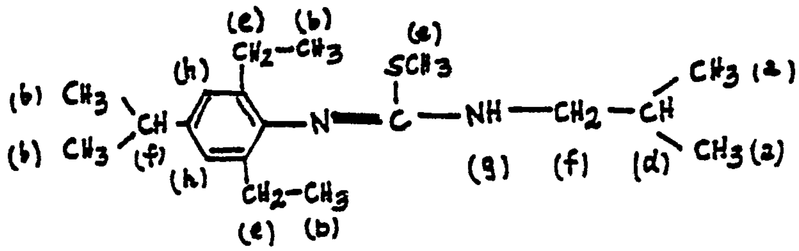

| Shift ppm | Multiplizität | Zuordnung | Intensität |
|---|---|---|---|
| NMR (in $CDCl_3$) | | | |
| 0,8 | D | I a | 6 |
| 1,2 | T | b | 〉12 |
| 1,3 | D | c | |
| 1,8 | M | d | 1 |
| 2,3 | Q + S | e | 8 |
| 2,6-3,1 | M | f | 4 |
| 4,2 | S | g | 1 |
| 6,8 | S | h | 2 |

IR: 3390 (NH), 880 (1,2,3,5- Aromat. Tetrasubst.).

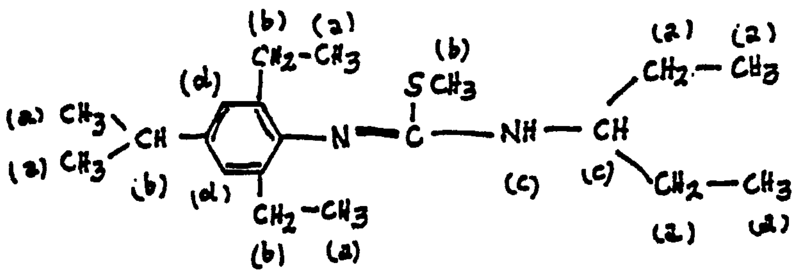

| Shift ppm | Multiplizität | Zuordnung | Intensität |
|---|---|---|---|
| NMR (in $CDCl_3$) | | | |
| 0,8 - 1,6 | T,T,D,M | I a | ~ 21 |
| 2,3 | S + Q | I b | ~ 9 |
| 2,9 | M | I c | ~ 2 |
| 6,8 | S | I d | 2 |

IR: 3460, 3370, (NH), 875 (Aromat. 1,2,3,5-Tetrasubst.)

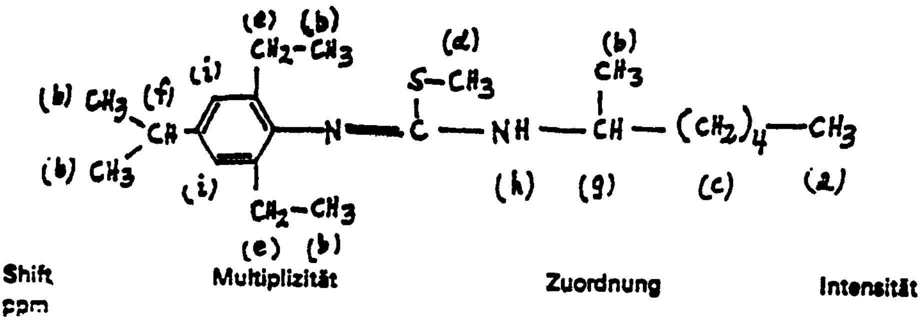

| Shift ppm | Multiplizität | Zuordnung | Intensität |
|---|---|---|---|
| **NMR** (in $CDCl_3$) | | | |
| 0,90 | T | I a | 26 |
| 0,90 - 1,50 | M | b | |
| 0,90 - 1,50 | M | c | |
| 2,37 | S | d | 3 |
| 2,46 | Q | e | 4 |
| 2,5-3,1 | M | f | 1 |
| 3,5-4,1 | M | g | 1 |
| 3,9 | S | h | 1 |
| 6,88 | S | i | 2 |

**IR**

872 $cm^{-1}$ = 1,2,3,5-Tetrasubst.

1612 $cm^{-1}$ = C=N-Bande

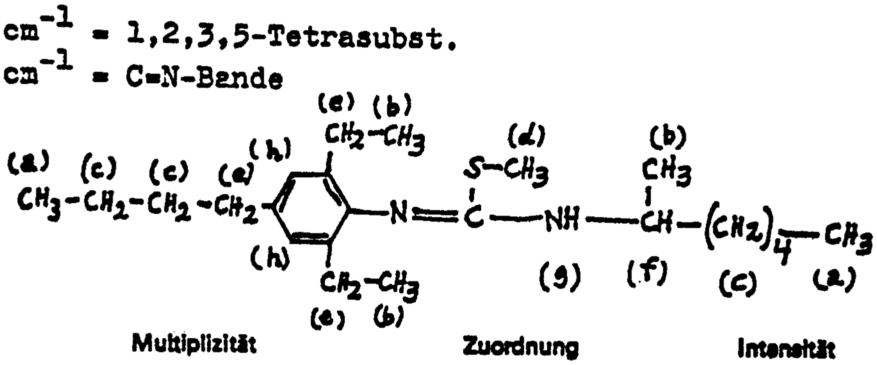

| Shift ppm | Multiplizität | Zuordnung | Intensität |
|---|---|---|---|
| **NMR** (in $CDCl_3$) | | | |
| 0,8-1,0 | M | I a | 27 |
| 1,0-1,4 | M | b | |
| 1,0-2,0 | M | c | |
| 2,37 | S | d | 3 |
| 2,47 | Q | e | 6 |
| 3,5-4,1 | M | f | 1 |
| 3,9 | S | g | 1 |
| 6,86 | S | h | 2 |

**IR**

864 $cm^{-1}$ = 1,2,3,5-Tetrasubst.

1615 $cm^{-1}$ = C=N-Bande

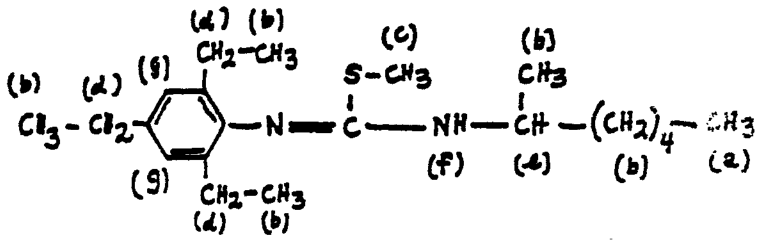

| Shift ppm | Multiplizität | Zuordnung | Intensität |
|---|---|---|---|
| **NMR** (in $CDCl_3$) | | | |
| 0,88 | T | I a | > 23 |
| 1,0-1,5 | M | b | |
| 2,37 | S | c | 3 |
| 2,46 | Q | d | 6 |
| 3,5-4,1 | M | e | 1 |
| 3,90 | S | f | 1 |
| 6,87 | S | g | 2 |

**IR**

1618 $cm^{-1}$ = C = N-Bande

871 $cm^{-1}$ = 1,2,3,5-Tetrasubst.

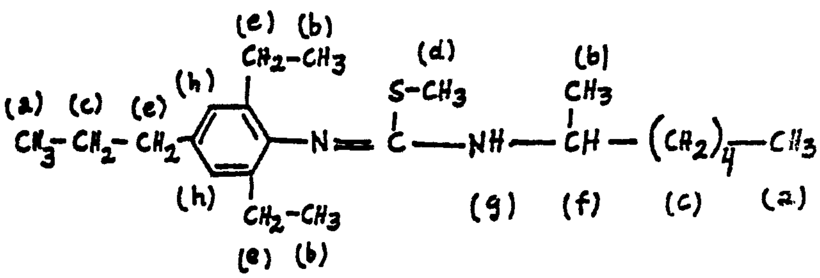

| Shift ppm | Multiplizität | Zuordnung | Intensität |
|---|---|---|---|
| **NMR** (in $CDCl_3$) | | | |
| 0,8-1,0 | M | I a | |
| 1,0-1,4 | M | b | } 25 |
| 1,0-2,0 | M | c | |
| 2,37 | S | d | 3 |
| 2,47 | Q | e | 6 |
| 3,5-4,1 | M | f | 1 |
| 3,9 | S | g | 1 |
| 6,86 | S | h | 2 |

**IR**

1615 $cm^{-1}$ = C=N-Bande

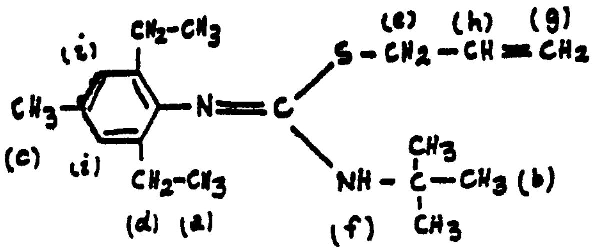

| Shift ppm | Multiplizität | Zuordnung | Intensität |
|---|---|---|---|
| NMR in $CDCl_3$ | | | |
| 1,2 | T | I a | 6 |
| 1,4 | S | b | 9 |
| 2,3 | S | c | 3 |
| 2,4 | Q | d | 4 |
| 3,3 | D | e | 2 |
| 4,3 | S | f | 1 |
| 5,2 | M | g | 2 |
| 5,8 | M | h | 1 |
| 6,8 | S | i | 2 |

IR: 3430 (NH), 865 (Aromat. 1,2,3,5-Tetrasubst.).

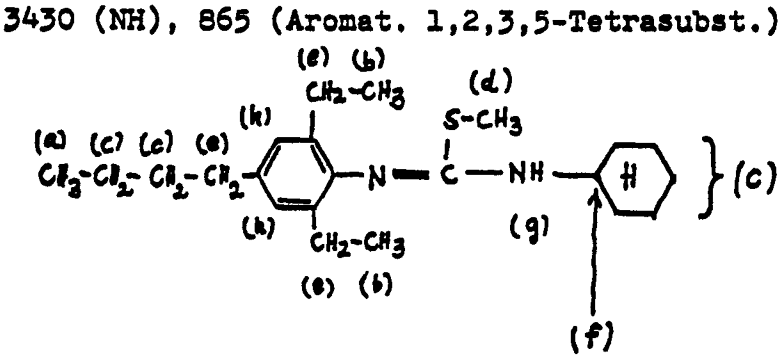

| Shift ppm | Multiplizität | Zuordnung | Intensität |
|---|---|---|---|
| NMR (in $CDCl_3$) | | | |
| 0,92 | T | I a | } 23 |
| 1,15 | T | b | |
| 1,0-2,2 | M | c | |
| 2,37 | S | d | 3 |
| 2,46 | Q | e | 6 |
| 3,3-3,9 | M | f | 1 |
| 4,1 | D | g | 1 |
| 6,85 | S | h | 2 |

IR

1615 $cm^{-1}$ = C=N-Bande

862 $cm^{-1}$ = 1,2,3,5-Tetrasubst.

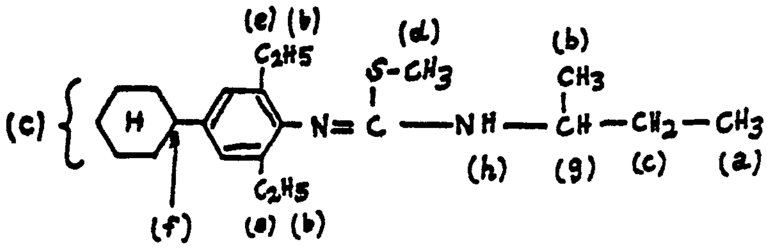

| Shift ppm | Multiplizität | Zuordnung | Intensität |
|---|---|---|---|
| NMR (in $CDCl_3$) | | | |
| 0,90 | T | I a | } 24 |
| 0,95-1,30 | M | b | |
| 1,0-2,2 | M | c | |
| 2,38 | S | d | 3 |
| 2,45 | Q | e | 4 |
| 2,7-3,1 | M | f | 1 |
| 3,4-4,0 | M | g | 1 |
| 3,9 | M | h | 1 |
| 6,87 | S | i | 2 |

IR:

867 $cm^{-1}$ = 1,2,3,5-Tetrasubst.

1620 $cm^{-1}$ = C = N-Bande

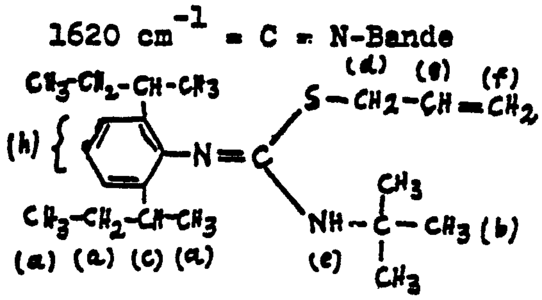

| Shift ppm | Multiplizität | Zuordnung | Intensität |
|---|---|---|---|
| NMR in $CDCl_3$ | | | |
| 0,7-1,7 | M | I a | > ~ 25 |
| 1,4 | S | b | |
| 2,7 | M | c | 2 |
| 3,4 | D | d | 2 |
| 4,3 | S | e | 1 |
| 5,0-5,4 | M | f | 2 |
| 5,8 | M | g | 1 |
| 7,0 | S | h | 3 |

IR: 3420 (NH), 760 (Aromat. 1,2,3-Trisubst. möglich).
1620 (C=N).

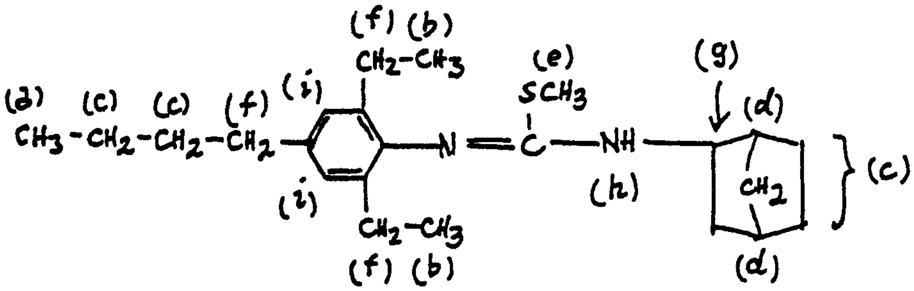

| Shift ppm | Multiplizität | Zuordnung | Intensität |
|---|---|---|---|
| NMR: (in $CDCl_3$) | | | |
| 0,90 | T | I a | 3 |
| 1,15 | T | b | 6 |
| 1,0-2,0 | M | c | 12 |
| 2,0-2,4 | M | d | 2 |
| 2,36 | S | e | 3 |
| 2,45 | Q | f | 6 |
| 3,3-3,8 | M | g | 1 |
| 4,0 | D | h | 1 |
| 6,85 | S | i | 2 |

IR: 1615 $cm^{-1}$ = C=N-Bande
867 od. 882 $cm^{-1}$ = 1,2,3,5-Tetrasubst.

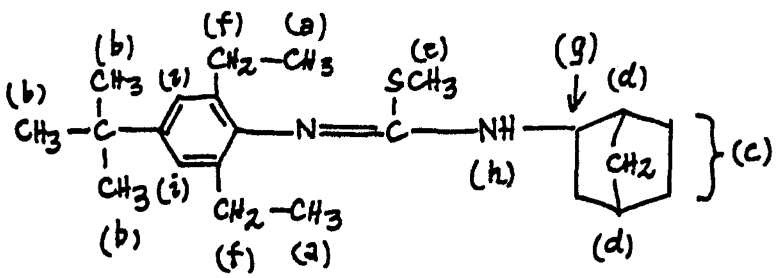

| Shift ppm | Multiplizität | Zuordnung | Intensität |
|---|---|---|---|
| NMR: (in $CDCl_3$) | | | |
| 1,18 | T | I a | 6 |
| 1,31 | S | b | 9 |
| 1,0-2,0 | M | c | 8 |
| 2,1-2,4 | M | d | 2 |
| 2,38 | S | e | 3 |
| 2,48 | Q | f | 4 |
| 3,3-3,8 | M | g | 1 |
| 4,05 | M | h | 1 |
| 7,05 | S | i | 2 |

IR: 1610 $cm^{-1}$= C=N-Bande
877 $cm^{-1}$= 1,2,3,5-Tetrasubst.

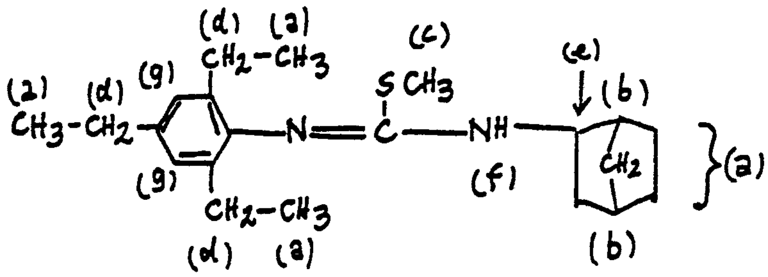

NMR: (in $CDCl_3$)

| Shift ppm | Multiplizität | Zuordnung | Intensität |
|---|---|---|---|
| 0,8-2,0 | M | I a | 17 |
| 2,0-2,4 | M | b | 2 |
| 2,37 | S | c | 3 |
| 2,45 | Q | d | 6 |
| 3,3-3,8 | M | e | 1 |
| 4,0 | D | f | 1 |
| 6,87 | S | g | 2 |

IR:

873 $cm^{-1}$ = 1,2,3,5-Tetrasubst.
1615 $cm^{-1}$ = C=N-Bande

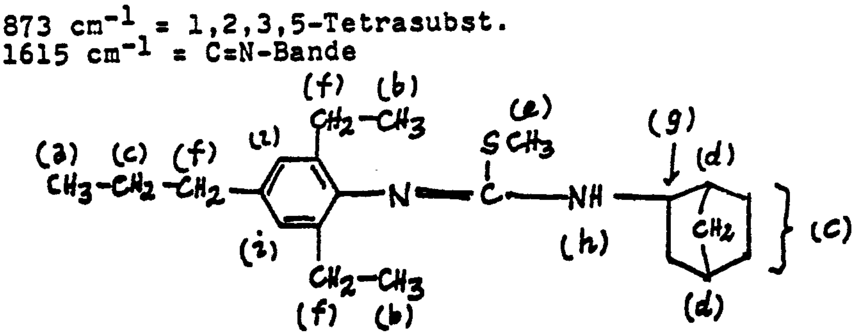

NMR: (in $CDCl_3$)

| Shift ppm | Multiplizität | Zuordnung | Intensität |
|---|---|---|---|
| 0,92 | T | I a | 3 |
| 1,15 | T | b | 6 |
| 1,0-2,0 | M | c | 8 + 2 |
| 2,0-2,4 | M | d | 2 |
| 2,37 | S | e | 3 |
| 2,45 | Q | f | 6 |
| 3,3-3,8 | M | g | 1 |
| 4,0 | D | h | 1 |
| 6,84 | S | i | 2 |

IR:
1610 $cm^{-1}$ = C=N-Bande

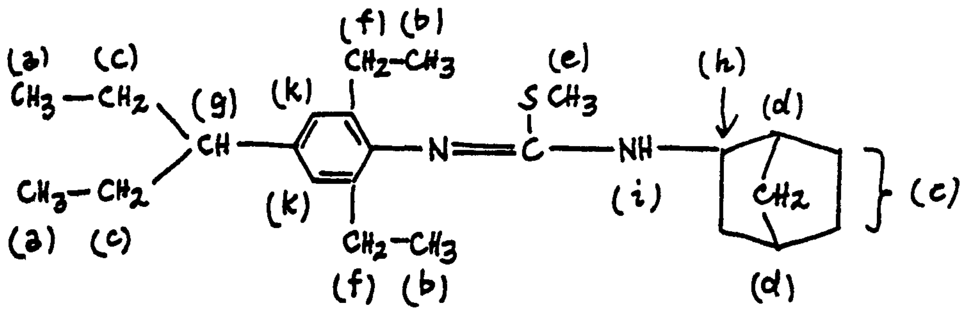

| Shift ppm | Multiplizität | Zuordnung | Intensität |
|---|---|---|---|
| NMR: (in $CDCl_3$) | | | |
| 0,73 | T | I a | 6 |
| 1,17 | T | b | 6 |
| 1,0-2,1 | M | c | 12 |
| 2,1-2,4 | M | d | 2 |
| 2,37. | S | e | 3 |
| 2,47 | Q | f | 4 |
| 2,5-3,0 | M | g | 1 |
| 3,3-3,8 | M | h | 1 |
| 4,05 | D | i | 1 |
| 6,80 | S | k | 2 |

IR: 1615 $cm^{-1}$ = C=N-Bande
873 $cm^{-1}$ = 1,2,3,5-Tetrasubst.

**Patentansprüche**

1. N-Aryl-N'-alkyl-S-alkyl-isothioharnstoffe der allgemeinen Formel

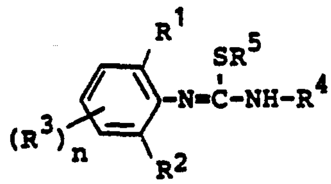

(I)

in welcher

$R^1$ für Alkyl oder Cycloalkyl,

$R^2$ für Alkyl mit mindestens zwei Kohlenstoffatomen oder für Cycloalkyl,

die Reste $R^3$ gleich oder verschieden sein können und

$R^3$ für Alkyl, Cycloalkyl oder Halogen,

n für eine ganze Zahl von 0 bis 2 steht

und $R^4$ für gegebenenfalls substituiertes Alkyl, gegebenenfalls substituiertes Alkenyl oder gegebenenfalls substituiertes Cycloalkyl steht,

und $R^5$ für Alkyl, Alkenyl oder Cycloalkyl steht,

und deren Salze.

2. Verbindungen gemäß der Formel (I) in Anspruch 1, in welcher

$R^1$ für Alkyl ($C_1$-$C_6$) oder Cycloalkyl ($C_4$-$C_7$),

$R^2$ für Alkyl ($C_2$-$C_6$) oder Cycloalkyl ($C_4$-$C_7$) steht,

die Reste $R^3$ gleich oder verschieden sein können und für Alkyl ($C_1$-$C_7$), Halogen, Cycloalkyl ($C_4$-$C_7$) stehen

und n für 0, 1 oder 2 steht,

$R^4$ für gegebenenfalls durch Halogen, Cycloalkyl ($C_4$-$C_7$), Cycloalkenyl ($C_5$-$C_7$) oder Trifluormethyl substituiertes Alkyl ($C_1$-$C_{14}$) oder für gegebenenfalls durch Halogen und/oder $CF_3$ ein- oder mehrfach substituiertes Alkenyl ($C_3$-$C_6$) steht oder für gegebenenfalls durch Alkyl ($C_1$-$C_4$), Halogen und/oder $CF_3$ ein- oder mehrfach substituiertes Cycloalkyl ($C_3$-$C_{10}$) steht,

und $R^5$ für Alkyl ($C_1$-$C_6$), Alkenyl ($C_3$-$C_6$) oder Cycloalkyl ($C_3$-$C_6$) steht,

und deren Salze.

3. Verfahren zur Herstellung von N-Aryl-N'-alkyl-S-alkyl-isothioharnstoffen der allgemeinen Formel

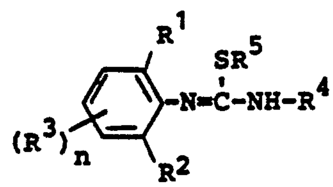

(I)

in welcher

$R^1$ für Alkyl oder Cycloalkyl,

$R^2$ für Alkyl mit mindestens zwei Kohlenstoffatomen oder für Cycloalkyl,

die Reste $R^3$ gleich oder verschieden sein können und

$R^3$ für Alkyl, Cycloalkyl oder Halogen,

n für eine ganze Zahl von 0 bis 2 steht

und $R^4$ für gegebenenfalls substituiertes Alkyl, gegebenenfalls substituiertes Alkenyl oder gegebenenfalls substituiertes Cycloalkyl steht

und $R^5$ für Alkyl, Alkenyl oder Cycloalkyl steht,

dadurch gekennzeichnet, daß man Thioharnstoffe der Formel

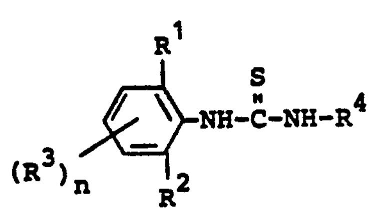

(II)

in welcher $R^1$, $R^2$, $R^3$, $R^4$ und n die oben angegebene Bedeutung besitzen, mit einer Verbindung der allgemeinen Formel

$$R^5\text{-}X \qquad \text{(III)}$$ umsetzt,

in welcher

$R^5$ die oben angegebene Bedeutung besitzt und

X für einen anionisch abspaltbaren Rest steht.

4. Ektoparasitizide Mittel, gekennzeichnet durch einen Gehalt an N-Aryl-N'-alkyl-S-alkyl-isothioharnstoffen gemäß Anspruch 1).

5. Insektizide und akarizide Mittel, gekennzeichnet durch einen Gehalt an N-Aryl-N'-alkyl-S-alkyl-isothioharnstoffen gemäß Anspruch 1).

6. Verfahren zur Herstellung von ektoparasitiziden Mitteln, dadurch gekennzeichnet, daß man N-Aryl-N'-alkyl-S-alkyl-isothioharnstoffe gemäß Anspruch 1) mit inerten, nichttoxischen Hilfs- und/oder Trägerstoffen vermischt.

7. Verfahren zur Herstellung von insektiziden und akariziden Mitteln, dadurch gekennzeichnet, daß man N-Aryl-N'-alkyl-S-alkylthioharnstoffe gemäß Anspruch 1) mit inerten, nichttoxischen Hilfs- und/oder Trägerstoffen vermischt.

8. Verfahren zur Bekämpfung von Ektoparasiten, dadurch gekennzeichnet, daß man von Ektoparasiten befallene Tiere mit N-Aryl-N'-alkyl-S-alkyl-isothioharnstoffen gemäß Anspruch 1) behandelt.

9. Verfahren zur Bekämpfung von Insekten und Acariden, dadurch gekennzeichnet, daß man von Insekten und/ oder Acariden befallene Pflanzen mit N-Aryl-N'-alkyl-S-alkylisothioharnstoffen gemäß Anspruch 1) behandelt.

Europäisches Patentamt

# EUROPÄISCHER RECHERCHENBERICHT

Nummer der Anmeldung

EP 78 10 0290

0000365

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | FR - A - 1 549 381 (CIBA)<br>* Zusammenfassung 1° * | 1 |

**KLASSIFIKATION DER ANMELDUNG (Int.Cl.²)**

C 07 C 157/14
A 01 N 9/12

**RECHERCHIERTE SACHGEBIETE (Int. Cl.²)**

C 07 C 157/14

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 06-09-1978 | GAUTIER |